# EUROPEAN PATENT SPECIFICATION

(11) **EP 2 310 413 B1**
(45) Date of publication and mention of the grant of the patent: **01.03.2017**
(21) Application number: 09772912.3
(22) Date of filing: 02.07.2009
(51) Int. Cl.: C07K 16/00, C07K 16/24

(54) **CAMELID DERIVED ANTIGEN BINDING POLYPEPTIDES**
VON CAMELIDEN STAMMENDE ANTIGEN BINDENDE POLYPEPTIDE
POLYPEPTIDES DE LIAISON À L'ANTIGÈNE DÉRIVÉE DU CAMÉLIDÉS

(30) Priority: 02.07.2008 GB 0812120; 02.07.2008 US 77730 P; 31.10.2008 US 110161 P
(43) Date of publication of application: 20.04.2011
(60) Divisional application: 15190638.5
(73) Proprietor: arGEN-X N.V., 4811 AH Breda (NL)
(72) Inventor: DREIER, Torsten, B-9830 Saint Martens Latem (BE); BLANCHETOT, Christophe, Frederic, Jerome, NL-2806 RG Gouda (NL); DE HAARD, Johannes, Joesph, Wilhelmus, NL-4436 NA Oudelande (NL)
(74) Representative: Boult Wade Tennant
(86) International application number: PCT/IB2009/006329
(87) International publication number: WO 2010/001251

(56) References cited:
- WO-A1-01/32714
- WO-A2-2005/037989
- WO-A2-2006/122825
- WO-A2-2007/136525
- WO-A2-2008/070344
- HAMERS-CASTERMAN C ET AL: "Naturally occurring antibodies devoid of light chains" NATURE, NATURE PUBLISHING GROUP, LONDON, UK, vol. 363, no. 6428, 1 January 1993 (1993-01-01), pages 446-448, XP002535892 ISSN: 0028-0836
- "Thesis; Promoters: Mulydermans-S & Hamers-R; Academic year 2001-2002" In: Ilham Legssyer: "The camel (camelus dromedarius) immunoglobulin light chains" June 2002 (2002-06), , Brussels , XP002562099 pages 49-103 page 54; table 1 page 62, paragraph 2 page 65, paragraph abstract page 69 page 74 page 79, paragraph 1; table 2a page 82, paragraph 3
- Dominique Scaviner: "Protein display: Llama (Lama glama) IGH J-REGIONs" 1 July 2002 (2002-07-01), XP002562100 Retrieved from the Internet: URL:http://imgt.cines.fr/textes/IMGTrepert oire/Proteins/protein/Lama/IGH/IGHJ/Lg_IGH Jallgenes.html> [retrieved on 2009-12-18]
- Dominique Scaviner: "Protein display: Llama (Lama glama) IGH V-REGIONs" 5 February 2002 (2002-02-05), XP002562101 Retrieved from the Internet: URL:http://www.imgt.org/textes/IMGTreperto ire/Proteins/protein/Lama/IGH/IGHV/Lg_IGHV allgenes.html> [retrieved on 2009-12-18]
- Dominique Scaviner: "Table of alleles: Llama (Lama glama ) IGHV" 5 February 2002 (2002-02-05), XP002562102 Retrieved from the Internet: URL:http://www.imgt.org/textes/IMGTreperto ire/Proteins/taballeles/lama/IGH/IGHV/Lg_I GHVall.html> [retrieved on 2009-12-18]
- Christèle Martinez-Jean and Joumana Jabado-Michaloud: "Protein display: Arabian camel (Camelus dromedarius) IGH C-REGIONs" 1 July 2002 (2002-07-01), XP002562103 Retrieved from the Internet: URL:http://www.imgt.org/textes/IMGTreperto ire/Proteins/protein/camel/IGH/IGHC/Cd_IGH Callgenes.html> [retrieved on 2009-12-18]
- GONZALES NOREEN R ET AL: "Minimizing the immunogenicity of antibodies for clinical application" TUMOR BIOLOGY, KARGER, BASEL, CH, vol. 26, no. 1, 28 February 2005 (2005-02-28), pages 31-43, XP008081934 ISSN: 1010-4283
- MOREA V ET AL: "Antibody Modeling: Implications for Engineering and Design" METHODS : A COMPANION TO METHODS IN ENZYMOLOGY, ACADEMIC PRESS INC., NEW YORK, NY, US, vol. 20, no. 3, 1 March 2000 (2000-03-01), pages 267-279, XP004466884 ISSN: 1046-2023
- DE GENST E ET AL: "Antibody repertoire development in camelids" DEVELOPMENTAL AND COMPARATIVE IMMUNOLOGY, PERGAMON PRESS, US, vol. 30, no. 1-2, 1 January 2006 (2006-01-01), pages 187-198, XP025088320 ISSN: 0145-305X [retrieved on 2006-01-01]
- KHEE HWANG W Y ET AL: "Use of human germline genes in a CDR homology-based approach to antibody humanization" METHODS : A COMPANION TO METHODS IN ENZYMOLOGY, ACADEMIC PRESS INC., NEW YORK, NY, US, vol. 36, no. 1, 1 May 2005 (2005-05-01), pages 35-42, XP004852551 ISSN: 1046-2023
- KIM ET AL: "Antibody engineering for the development of therapeutic antibodies" MOLECULES AND CELLS,, vol. 20, no. 1, 1 January 2005 (2005-01-01), pages 17-29, XP002540664
- SAERENS ET AL: "Identification of a Universal VHH Framework to Graft Non-canonical Antigen-binding Loops of Camel Single-domain Antibodies" JOURNAL OF MOLECULAR BIOLOGY, LONDON, GB, vol. 352, no. 3, 23 September 2005 (2005-09-23), pages 597-607, XP005052664 ISSN: 0022-2836
- SHEN ET AL: "Single variable domain antibody as a versatile building block for the construction of IgG-like bispecific antibodies" JOURNAL OF IMMUNOLOGICAL METHODS, ELSEVIER SCIENCE PUBLISHERS B.V.,AMSTERDAM, NL, vol. 318, no. 1-2, 3 January 2007 (2007-01-03), pages 65-74, XP005820137 ISSN: 0022-1759
- HARMSEN ET AL: "Prolonged in vivo residence times of llama single-domain antibody fragments in pigs by binding to porcine immunoglobulins" VACCINE, BUTTERWORTH SCIENTIFIC. GUILDFORD, GB, vol. 23, no. 41, 30 September 2005 (2005-09-30), pages 4926-4934, XP005063278 ISSN: 0264-410X
- ALMAGRO JUAN C ET AL: "Humanization of antibodies", FRONTIERS IN BIOSCIENCE, FRONTIERS IN BIOSCIENCE, ALBERTSON, NY, US, vol. 13, 1 January 2008 (2008-01-01), pages 1619-1633, XP009126790, ISSN: 1093-9946
- HARRISON: "The unique properties of camelid IgG have potential to improve the treatment of snake bite", JOURNAL OF CAMEL PRACTICE AND RESEARCH,, vol. 14, no. 1, 1 January 2007 (2007-01-01), pages 15-16, XP009186727,
- HERRERA M ET AL: "Factors associated with adverse reactions induced by caprylic acid-fractionated whole IgG preparations: comparison between horse, sheep and camel IgGs", TOXICON, ELMSFORD, NY, US, vol. 46, no. 7, 1 December 2005 (2005-12-01), pages 775-781, XP027611127, ISSN: 0041-0101 [retrieved on 2005-12-01]
- MIKHAIL POPKOV ET AL: "Chapter 15. Antibody Libraries from Immunized Repertoires", 1 January 2005 (2005-01-01), PHAGE DISPLAY IN BIOTECHNOLOGY AND DRUG DISCOVERY, CRC PRESS, PAGE(S) 529 - 657, XP009143046, ISBN: 978-0-8247-5466-2

## Description

### FIELD OF THE INVENTION

The invention relates to a novel platform for generation of antigen binding polypeptides, including monoclonal antibodies, which share a high degree of sequence and structural homology with the variable domains of human antibodies.

### BACKGROUND TO THE INVENTION

Monoclonal antibodies have many applications as research tools and, increasingly, as therapeutic or diagnostic agents. Currently more than 20 different monoclonal antibodies have received regulatory approval to treat a variety of different diseases, including cancer, inflammation, auto-immune disorders, infectious disease, asthma, cardiovascular diseases and transplant rejection and the number of monoclonal antibody drugs in the development pipeline is increasing year-on-year.

The utility of rodent (specifically murine) monoclonal antibodies in human therapy is limited because of problems associated with their non-human origin, in particular their immunogenicity in a human host. In order to minimize the human immune response against therapeutic antibody drugs, monoclonal antibody technology has evolved from full mouse antibodies to chimeric antibodies (mouse variable domains grafted on a human IgG backbone), to humanized antibodies (mouse CDRs grafted on a human IgG backbone), to "fully human" antibodies derived from synthetic libraries or immunized transgenic mice expressing part of the human IgG repertoire.

A number of technology platforms have been developed which allow production of fully human or "humanized" monoclonal antibodies against target antigens of therapeutic interest. Each of these platforms has its own particular characteristics and potential shortcomings.

Humanisation of mouse monoclonal antibodies was initially achieved by combining mouse variable domains with human constant domains, creating so called chimeric antibodies having about 70% of human content. A further degree of humanization was subsequently achieved by grafting the complementarity-determining regions (CDRs) of mouse monoclonal antibodies onto human framework regions of the variable antibody domains of human antibodies. In addition, several amino acid residues present in those framework regions were identified as interacting with the CDRs or antigen and were back mutated in the humanized antibody to improve binding. (Almagro et al. Frontiers in Bioscience. 13: 1619-1633 (2008)). Monoclonal antibodies engineered using this approach have a relatively high degree of primary sequence homology to human VH and VL domain sequences after humanisation, but a drawback is the possibility of ending up with hypervariable loops not having human-like structure, because not all mouse-encoded CDRs use canonical folds, and canonical fold combinations, which are not found in human antibodies (Almagro et al., Mol. Immunol. 34:1199-1214 (1997); Almagro et al., Immunogen. 47:355-63 (1998)). A further drawback is the large number of mutations typically required to humanise such antibodies (the procedure for which is complex and time-consuming), with the consequent risk of losing affinity and potency as a result of the number of changes needed for humanisation and, the fact that VKappa domains are mainly used in the murine repertoire, whereas approximately half of all human antibodies possess VLambda domains.

As a potential improvement on humanised mouse monoclonal antibodies, "fully human" monoclonal antibodies can be produced by two very different approaches. The first approach is selection from a fully synthetic human combinatorial antibody library (for example HuCAL®, MorphoSys). The potential drawback of this approach is that the synthetic library only approximates the functional diversity naturally present in the human germline, thus the diversity is somewhat limited. Also, antibodies generated using this approach are not derived from *in vivo* selection of CDRs via active immunisation, and typically affinity maturation has to be done in order to improve affinity for the target antigen. Affinity maturation is a lengthy process which may add considerable time to the antibody discovery process. Also, in the process of affinity maturation certain amino acid residues may be changed which may negatively affect the binding specificity or stability of the resulting antibody (Wu et al., J. Mol. Biol. 368: 652-65 (2007)).

Alternative "fully human" platforms are based on transgenic mice which have been engineered to replace the murine immunoglobulin encoding region with antibody-encoding sequences from the human germline (for example HuMab, Medarex). These systems have the advantage that antibodies are raised by active immunisation, with the target antigen, i.e. they have a high starting affinity for the antigen, and that no or only minimal antibody engineering of the original antibodies is required in order to make them more human-like. However, the transgenic mouse strains are by definition highly inbred and this has adverse consequences for the strength and diversity of the antibody response. Another drawback with this platform may be impaired B cell maturation due to human Fc/mouse Fc receptor interaction in some transgenic mouse systems.

A further platform is based on immunisation of non-human primates, specifically cynomologous monkeys. Due to the high degree of amino acid sequence identity between monkey and human immunoglobulins it is postulated that antibodies raised in monkeys will require little or no additional "humanisation" in the variable domains in order to render them useful as human therapeutics (see WO 93/02108).

WO2008/070344 is concerned with anitbodies that bind sphingosine-1-phosphate (S1P).

Hwang et al., Methods 36(2005),35-42 is concerned with a method of humanizing antibodies by CDR grafting. The application of this method to the humanization of mouse antibodies is disclosed.

Ilham Legssyer, Thesis submitted to the Vrije Universiteit Brussel (Academic Year 2001-2002) discloses light chain immunoglobulin sequences from *Camelus dromedarius.*

IMGT database entry XP-002562100 provides partial J-region amino acid sequences from llama.

IMGT database entry XP-002562102 (2002) provides a list of accession numbers for llama V-region alleles.

IMGT database entry XP-002562101 (2002) provides amino acid sequence information for a number of llama V-region clones.

IMGT database entry XP-002562103 provides amino acid sequence for the constant region of Arabian camel immunoglobulins.

WO2007/136525 discloses chimeric monoclonal antibodies recognising the antigen iNOS.

### SUMMARY OF THE INVENTION

The invention is defined by the claims.
present inventors have recognised the need for a "humanised" monoclonal antibody (antigen binding polypeptide) platform which avoids some or all of the shortcomings they have observed with prior art humanised or fully human antibody platforms and which enables the production of antibodies of high specificity and affinity against a broad range of target antigens of therapeutic importance whilst minimising immunogenicity in a human host.

The present inventors have observed that both the VH and the VL domains of conventional antibodies from the family *Camelidae* exhibit a high degree of amino acid sequence identity with the VH and VL domains of human antibodies over the framework regions. In fact, the degree of sequence identity between camelid conventional VH domains and human VH domains, and between camelid conventional VL domains and human VL domains can approach that observed between humans and other primate species, e.g. cynomologous monkeys, and is much higher than might be expected given the phylogenetic distance between humans and camelids. This finding is surprising given that the variable domains of heavy-chain camelid antibodies (VHH) do not show this high degree of sequence homology with human variable domains.

In addition, the inventors have observed that the hypervariable loops (H1, H2, L1, L2 and L3) of camelid VH and VL domains often exhibit a high degree of structural homology with the hypervariable loops of human VH and VL domains, which is again unexpected given the evolutionary distance between humans and camelids. The high degree of structural homology between camelid conventional antibodies (or rather the hypervariable loops of such antibodies) and human antibodies is also surprising since the hypervariable loops of heavy-chain camelid antibodies have been reported to vary substantially in conformation and length from the corresponding loops in human and mouse VH (see review De Genst et al., Develop Comp. Immunol. 30:187-98 (2006)).

The high degree of primary amino acid sequence homology with the framework regions of human antibodies, coupled with the high degree of structural homology of the antigen binding sites comprising the hypervariable loops with the binding sites of human antibodies, plus the fact that *Camelidae* conventional antibodies can be raised by active immunisation of an outbred animal population, which are phylogenetically quite distant from humans, has led the present inventors to surmise that conventional antibodies from the family *Camelidae* are an attractive starting point for engineering monoclonal antibodies having potential utility as human therapeutics.

Therefore, in accordance with a first aspect of the invention there is provided a monoclonal antigen binding polypeptide comprising a VH domain and a VL domain, wherein each of the hypervariable loops in both the VH domain and the VL domain is obtained from a VH or VL domain of a species in the family Camelidae, wherein hypervariable loop H1 and hypervariable loop H2 in the VH domain each exhibit a predicted or actual canonical fold structure which is identical to a canonical fold structure which occurs in human antibodies, and wherein hypervariable loop H1 and hypervariable loop H2 in the VH domain form a combination of predicted or actual canonical fold structures which is identical to a combination of canonical fold structures known to occur in a human germline VH domain.

Also described is a monoclonal antigen binding polypeptide comprising a VH domain and a VL domain, wherein each of the hypervariable loops or complementarity determining regions (CDRs) in the VH domain and the VL domain is obtained from a VH or VL domain of a species in the family *Camelidae.*

In one embodiment the antigen binding polypeptide of the invention may be immunoreactive with a target antigen. In another embodiment the antigen binding polypeptide may bind specifically to a target antigen.

In a non-limiting embodiment the antigen binding polypeptide of the invention may be a recombinant polypeptide.

In a non-limiting embodiment the antigen binding polypeptide of the invention may be a chimeric polypeptide.

In a non-limiting embodiment the antigen binding polypeptide of the invention may be a monoclonal antibody.

In a non-limiting embodiment the antigen binding polypeptide of the invention may be a recombinantly expressed chimeric monoclonal antibody.

In a non-limiting embodiment the antigen binding polypeptide according to the invention may comprise hypervariable loops or complementarity determining regions which have been obtained by active immunisation of a species in the family *Camelidae.*

Also described is a process for preparing an antigen binding polypeptide immunoreactive with a target antigen, said process comprising:
(a) determining the nucleotide sequence encoding at least one hypervariable loop or complementarity determining region (CDR) of the VH and/or the VL domain of a *Camelidae* conventional antibody immunoreactive with said target antigen; and
(b) expressing an antigen binding polypeptide immunoreactive with said target antigen, said antigen binding polypeptide comprising a VH and a VL domain, wherein at least one hypervariable loop or complementarity determining region (CDR) of the VH domain or the VL domain has an amino acid sequence encoded by the nucleotide sequence determined in part (a).

Also described is a process for preparing a recombinant antigen binding polypeptide that is immunoreactive with (or specifically binds to) a target antigen, said antigen binding polypeptide comprising a VH domain and a VL domain, wherein at least one hypervariable loop or complementarity determining region (CDR) in the VH domain or the VL domain is obtained from a species in the family *Camelidae,* said process comprising the steps of:
(a) isolating *Camelidae* nucleic acid encoding at least one hypervariable loop or complementarity determining region (CDR) of the VH and/or the VL domain of a *Camelidae* conventional antibody immunoreactive with said target antigen;
(b) preparing a polynucleotide comprising a nucleotide sequence encoding hypervariable loop(s) or complementarity determining region(s) having amino acid sequence identical to the hypervariable loop(s) or complementarity determining region(s) encoded by the nucleic acid isolated in step (a), which polynucleotide encodes an antigen binding polypeptide comprising a VH domain and a VL domain that is immunoreactive with (or specifically binds to) said target antigen; and
(c) expressing said antigen binding polypeptide from the recombinant polynucleotide of step (b).

The polynucleotide prepared in step (b) is preferably recombinant.

The invention further provides a polynucleotide comprising a nucleotide sequence which encodes an antigen binding polypeptide according to the first aspect of the invention. Also described is a polynucleotide which encodes a fragment of said antigen binding polypeptide, which fragment comprises at least one hypervariable loop or complementarity determining region (CDR) obtained from a VH or VL domain of a species in the family *Camelidae.*

The invention also provides an expression vector comprising the polynucleotide defined above operably linked to regulatory sequences which permit expression of the antigen binding polypeptide in a host cell or cell-free expression system, a host cell or cell-free expression system containing the expression vector, and a method of producing a recombinant antigen binding polypeptide which comprises culturing the host cell or cell free expression system under conditions which permit expression of the antigen binding polypeptide and recovering the expressed antigen binding polypeptide.

Also described is a test kit comprising an antigen binding polypeptide according to the first aspect of the invention and a pharmaceutical formulation comprising an antigen binding polypeptide according to the first aspect of the invention and at least one pharmaceutically acceptable diluent, excipient or carrier.

### BRIEF DESCRIPTION OF THE DRAWINGS

Figure 1 - shows the results of an ELISA in which sera from llamas immunised with IL1-Beta were tested for the presence of antibodies against IL1-Beta, on day 0 and day 28 following immunisation.
Figure 2 - illustrates the amino acid sequences of "humanized" variants of two Fabs immunoreactive with IL-1 Beta, coded 1 E2 and 1 F2. Based on the alignment against the closest human germlines, mutations in the VH and Vλ framework regions of 1 E2 and 1 F2 were proposed. Besides the fully humanized (hum) and the wild type (wt) V regions, also a "safe variant" with only three wild type residues remaining was proposed (safe).
Figure 3 - shows the results of an ELISA in which recombinantly expressed Fabs were tested for their ability to bind biot-IL-1 Beta. For this the Fabs were captured on an antimyc coated Maxisorp plate. Biotinylated human IL-1 Beta was added and bound cytokine was detected using HRP-conjugated streptavidin.
Figure 4 - shows the results of phage ELISA in which phage displaying humanized variants of Fabs 1 E2 and 1 F2 were tested for binding to IL-1 Beta.
Figure 5 - shows the results of ELISA in which chimeric 1 E2 was tested for binding to IL-1 Beta.

### DETAILED DESCRIPTION OF THE INVENTION

The invention is defined by the claims.

The invention relates to a new platform technology for production of antigen binding polypeptides having specificity for a desired target antigen which is based on the conventional antibody repertoire of species in the family *Camelidae,* and to antigen binding polypeptides obtained using this technology platform.

Thus, in a first aspect the invention provides a monoclonal antigen binding polypeptide comprising a VH domain and a VL domain, wherein each of the hypervariable loops or complementarity determining regions (CDRs) in the VH domain or the VL domain is obtained from a VH or VL domain of a species in the family *Camelidae,* wherein hypervariable loop H1 and hypervariable loop H2 in the VH domain each exhibit a predicted or actual canonical fold structure which is identical to a canonical fold structure which occurs in human antibodies, and wherein hypervariable loop H1 and hypervariable loop H2 in the VH domain form a combination of predicted or actual canonical fold structures which is identical to a combination of canonical fold structures known to occur in a human germline VH domain.

In the following passages different aspects of the invention are defined in more detail. Each aspect so-defined may be combined with any other aspect or aspects unless clearly indicated to the contrary. In particular, any feature indicated as being preferred or advantageous may be combined with any other feature or features indicated as being preferred or advantageous.

### Definitions

The term "antigen binding polypeptide" refers to any polypeptide comprising a VH domain and a VL domain which is immunoreactive with, exhibits specific binding to, a target antigen. Exemplary antigen binding polypeptides include antibodies and immunoglobulins, and also antibody fragments, as discussed elsewhere herein.

The term "antigen", when referring to the "target antigen" against which the antigen binding polypeptide is immunoreactive, takes its normal meaning to a person of ordinary skill in the art, and includes, *inter alia*, polypeptide, peptide, polysaccharide, glycoprotein, polynucleotide (e.g. DNA), or synthetic chemical antigens.

The term "antigen" can also be used to describe the material employed in the immunisation of animals (e.g. camelids) during the manufacture of antigen binding polypeptides of the invention. In this context the term "antigen" may take a wider meaning, and could encompass purified forms of the antigen, and also crude or semipurified preparations of the antigen, such as for example cells, cell lysates or supernatants, cell fractions, e.g. cell membranes, etc. , plus haptens conjugated with an appropriate carrier protein. The "antigen" used in an immunisation protocol is not necessarily structurally identical to the "target antigen" with which the resulting antigen binding polypeptide is to immunoreact. Typically the "antigen" used for immunisation may be a truncated form of the "target antigen", e.g. a fragment containing an immunogenic epitope. Further characteristics of "antigens" used for active immunisation are described elsewhere herein, and would be generally known to a person skilled in the art.

"Specific binding" between and antigen binding polypeptide and a target antigen refers to immunological specificity. An antigen binding polypeptide binds "specifically" to its target antigen if it binds an epitope on the target antigen in preference to other epitopes. "Specific binding" does not exclude cross-reactivity with other antigens bearing similar antigenic epitopes.

"Antibodies" (Abs) and "immunoglobulins" (Igs) are glycoproteins which exhibit binding specificity to a (target) antigen.

The camelid species are known to possess two different types of antibodies; the classical or "conventional" antibodies and also the heavy-chain antibodies.

As used herein, the term "conventional antibody" refers to antibodies of any isotype, including IgA, IgG, IgD, IgE or IgM. Native or naturally occurring "conventional" camelid antibodies are usually heterotetrameric glycoproteins, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end (N-terminal) a variable domain (VH) followed by a number of constant domains. Each light chain has a variable domain (VL) at one end (N-terminal) and a constant domain (CL) at its other end; the constant domain of the light chain is aligned with the first constant domain of the heavy chain, and the light-chain variable domain is aligned with the variable domain of the heavy chain. Particular amino acid residues are believed to form an interface between the light- and heavy-chain variable domains.

The term "heavy-chain antibody" refers to the second type of antibodies known to occur naturally in camelid species, such antibodies being naturally devoid of light chains (Hamers-Casterman, et al. Nature. 1993; 363; 446-8). The heavy-chain antibodies (abbreviated to HCAb) are composed of two heavy chains linked by a covalent disulphide bond. Each heavy chain in the HCAb has a variable domain at one end. The variable domains of HCAbs are referred to as "VHH" in order to distinguish them from the variable domains of the heavy chains of "conventional" camelid antibodies (VH). The VHH domains and VH domains are entirely distinct and are encoded by different gene segments in the camelid genome.

The VL domains in the polypeptide of the invention may be of the VLambda type or the Vkappa type. The term "VL domain" therefore refers to both VKappa and VLambda isotypes from *Camelidae*, and engineered variants thereof which contain one or more amino acid substitutions, insertions or deletions relative to a *Camelidae* VL domain.

The term "VH domain" refers to a VH domain of any known heavy chain isotype of *Camelidae*, including γ, ε, δ, α or µ isotypes, as well as engineered variants thereof which contain one or more amino acid substitutions, insertions or deletions relative to a *Camelidae* VH domain. The term "VH domain" refers only to VH domains of camelid conventional antibodies and does not encompass camelid VHH domains.

The term "variable" refers to the fact that certain portions of the variable domains VH and VL differ extensively in sequence among antibodies and are used in the binding and specificity of each particular antibody for its target antigen. However, the variability is not evenly distributed throughout the variable domains of antibodies. It is concentrated in three segments called "hypervariable loops" in each of the VL domain and the VH domain which form part of the antigen binding site. The first, second and third hypervariable loops of the VLambda light chain domain are referred to herein as L1(λ), L2(λ) and L3(λ) and may be defined as comprising residues 24-33 (L1(λ), consisting of 9, 10 or 11 amino acid residues), 49-53 (L2(λ), consisting of 3 residues) and 90-96 (L3(λ), consisting of 5 residues) in the VL domain (Morea et al., Methods 20:267-279 (2000)). The first, second and third hypervariable loops of the VKappa light chain domain are referred to herein as L1(κ), L2(κ) and L3(κ) and may be defined as comprising residues 25-33 (L1(κ), consisting of 6, 7, 8, 11, 12 or 13 residues), 49-53 (L2(κ), consisting of 3 residues) and 90-97 (L3(κ), consisting of 6 residues) in the VL domain (Morea et al., Methods 20:267-279 (2000)). The first, second and third hypervariable loops of the VH domain are referred to herein as H1, H2 and H3 and may be defined as comprising residues 25-33 (H1, consisting of 7, 8 or 9 residues), 52-56 (H2, consisting of 3 or 4 residues) and 91-105 (H3, highly variable in length) in the VH domain (Morea et al., Methods 20:267-279 (2000)).

Unless otherwise indicated, the terms L1, L2 and L3 respectively refer to the first, second and third hypervariable loops of a VL domain, and encompass hypervariable loops obtained from both Vkappa and Vlambda isotypes from *Camelidae.* The terms H1, H2 and H3 respectively refer to the first, second and third hypervariable loops of the VH domain, and encompass hypervariable loops obtained from any of the known heavy chain isotypes from *Camelidae,* including γ, ε, δ, α or µ.

The hypervariable loops L1, L2, L3, H 1, H2 and H3 may each comprise part of a "complementarity determining region" or "CDR". The terms "hypervariable loop" and "complementarity determining region" are not strictly synonymous, since the hypervariable loops (HVs) are defined on the basis of structure, whereas complementarity determining regions (CDRs) are defined based on sequence variability (Kabat et al., Sequences of Proteins of Immunological Interest, 5th Ed. Public Health Service, National Institutes of Health, Bethesda, MD., 1983) and the limits of the HVs and the CDRs may be different in some VH and VL domains.

The CDRs of the VL and VH domains can typically be defined as comprising the following amino acids: residues 24-34 (CDRL1), 50-56 (CDRL2) and 89-97 (CDRL3) in the light chain variable domain, and residues 31-35 or 31-35b (CDRH1), 50-65 (CDRH2) and 95-102 (CDRH3) in the heavy chain variable domain; Kabat et al., Sequences of Proteins of Immunological Interest, 5th Ed. Public Health Service, National Institutes of Health, Bethesda, MD. (1991)). Thus, the HVs may be comprised within the corresponding CDRs and references herein to the "hypervariable loops" of VH and VL domains should be interpreted as also encompassing the corresponding CDRs, and *vice versa*, unless otherwise indicated.

The more highly conserved portions of variable domains are called the framework region (FR). The variable domains of native heavy and light chains each comprise four FRs (FR1, FR2, FR3 and FR4, respectively), largely adopting a β-sheet configuration, connected by the three hypervariable loops. The hypervariable loops in each chain are held together in close proximity by the FRs and, with the hypervariable loops from the other chain, contribute to the formation of the antigen-binding site of antibodies. Structural analysis of antibodies revealed the relationship between the sequence and the shape of the binding site formed by the complementarity determining regions (Chothia et al., J. Mol. Biol. 227: 799-817 (1992)); Tramontano et al., J. Mol. Biol, 215:175-182 (1990)). Despite their high sequence variability, five of the six loops adopt just a small repertoire of main-chain conformations, called "canonical structures". These conformations are first of all determined by the length of the loops and secondly by the presence of key residues at certain positions in the loops and in the framework regions that determine the conformation through their packing, hydrogen bonding or the ability to assume unusual main-chain conformations.

The constant domains are not involved directly in binding of an antibody to an antigen, but exhibit various effector functions, such as participation of the antibody in antibody-dependent cellular toxicity (ADCC) or complement-dependent cytotoxicity (CDC).

In all aspects and embodiments of the invention, the *Camelidae* (or camelid) species (from which the hypervariable loops or CDRs of the antigen binding polypeptide of the invention are obtained) can be camel, llama, dromedary, vicunia, guanaco or alpaca and any crossings thereof. Llama (*Lama glama*) and alpaca (*Lama pacos*) are the preferred *Camelidae* species for all aspects of the invention.

The antigen binding polypeptides of the invention are characterised in that they contain hypervariable loops or complementarity determining regions which are obtained from a VH domain or a VL domain of a species in the family *Camelidae.* For the avoidance of doubt, the terms "VH domain" "VL domain" refer to domains derived from camelid *conventional* antibodies. This definition excludes the camelid heavy chain only VHH antibodies, and recombinant constructs containing solely HVs or CDRs of camelid VHH domains, which are not encompassed within the scope of the present invention.

By "hypervariable loop or complementarity determining region obtained from a VH domain or a VL domain of a species in the family *Camelidae*" is meant that that hypervariable loop (HV) or CDR has an amino acid sequence which is identical, or substantially identical, to the amino acid sequence of a hypervariable loop or CDR which is encoded by a *Camelidae* immunoglobulin gene. In this context "immunoglobulin gene" includes germline genes, immunoglobulin genes which have undergone rearrangement, and also somatically mutated genes. Thus, the amino acid sequence of the HV or CDR *obtained from* a VH or VL domain of a *Camelidae* species may be identical to the amino acid sequence of a HV or CDR present in a mature *Camelidae* conventional antibody. The term "obtained from" in this context implies a structural relationship, in the sense that the HVs or CDRs of the antigen binding polypeptide of the invention embody an amino acid sequence (or minor variants thereof) which was originally encoded by a *Camelidae* immunoglobulin gene. However, this does not necessarily imply a particular relationship in terms of the *production process* used to prepare the antigen binding polypeptide of the invention. As will be discussed below, there are several processes which may be used to prepare antigen binding polypeptides comprising HVs or CDRs with amino acid sequences identical to (or substantially identical to) sequences originally encoded by a *Camelidae* immunoglobulin gene.

For the avoidance of doubt, the terms "VH domain of a conventional antibody of a camelid" and "VH domain obtained from a species of *Camelidae*" are used synonymously and encompass VH domains which are the products of synthetic or engineered recombinant genes (including codon-optimised synthetic genes), which VH domains have an amino acid sequence identical to (or substantially identical to) the amino acid sequence of a VH domain encoded by a *Camelidae* immunoglobulin gene (germline, rearranged or somatically mutated). Similarly, the terms "VL domain of a conventional antibody of a camelid" and "VL domain obtained from a species of *Camelidae*" are used synonymously and encompass VL domains which are the products of synthetic or engineered recombinant genes (including codon-optimised synthetic genes), which VL domains have an amino acid sequence identical to (or substantially identical to) the amino acid sequence of a VL domain encoded by a *Camelidae* immunoglobulin gene (germline, rearranged or somatically mutated).

The antigen binding polypeptides of the invention are typically recombinantly expressed polypeptides, and may be chimeric polypeptides. The term "chimeric polypeptide" refers to an artificial (non-naturally occurring) polypeptide which is created by juxtaposition of two or more peptide fragments which do not otherwise occur contiguously. Included within this definition are "species" chimeric polypeptides created by juxtaposition of peptide fragments encoded by two or more species, e.g. camelid and human.

The antigen binding polypeptides of the invention are not naturally occurring human antibodies, specifically human autoantibodies, due to the requirement for at least one hypervariable loop (or CDR) from camelid. By "naturally occurring" human antibody is meant an antibody which is naturally expressed within a human subject. Antigen binding polypeptides having an amino acid sequence which is 100% identical to the amino acid sequence of a naturally occurring human antibody, or a fragment thereof, which natural antibody or fragment is not chimeric and has not been subject to any engineered changes in amino acid sequence (excluding somatic mutations) are excluded from the scope of the invention.

The antigen binding polypeptides according to the invention comprise both a heavy chain variable (VH) domain and a light chain variable (VL) domain, and are characterised in that each of the hypervariable loops or complementarity determining regions in the VH domain and the VL domain is obtained from a species in the family *Camelidae.*

In one specific embodiment each of the hypervariable loops H1, H2, H3, L1, L2 and L3 in both the VH domain and the VL domain may be obtained from a species in the family *Camelidae.*

In one embodiment the entire VH domain and/or the entire VL domain may be obtained from a species in the family *Camelidae.* The *Camelidae* VH domain and/or the *Camelidae* VL domain may then be subject to protein engineering, in which one or more amino acid substitutions, insertions or deletions are introduced into the *Camelidae* sequence. These engineered changes preferably include amino acid substitutions relative to the *Camelidae* sequence. Such changes include "humanisation" or "germlining" wherein one or more amino acid residues in a camelid-encoded VH or VL domain are replaced with equivalent residues from a homologous human-encoded VH or VL domain.

In certain embodiments, *Camelidae* hypervariable loops (or CDRs) may be obtained by active immunisation of a species in the family *Camelidae* with a desired target antigen. As discussed and exemplified in detail herein, following immunisation of *Camelidae* (either the native animal or a transgenic animal engineered to express the immunoglobulin repertoire of a camelid species) with the target antigen, B cells producing (conventional *Camelidae*) antibodies having specificity for the desired antigen can be identified and polynucleotide encoding the VH and VL domains of such antibodies can be isolated using known techniques.

Thus, in a specific embodiment, the invention provides a recombinant antigen binding polypeptide immunoreactive with a target antigen, the polypeptide comprising a VH domain and a VL domain, wherein at least one hypervariable loop or complementarity determining region in the VH domain or the VL domain is obtained from a VH or VL domain of a species in the family *Camelidae,* which antigen binding polypeptide is *obtainable by* a process comprising the steps of:
(a) immunising a species in the family *Camelidae* with a target antigen or with a polynucleotide encoding said target antigen and raising an antibody to said target antigen;
(b) determining the nucleotide sequence encoding at least one hypervariable loop or complementarity determining region (CDR) of the VH and/or the VL domain of a *Camelidae* conventional antibody immunoreactive with said target antigen; and
(c) expressing an antigen binding polypeptide immunoreactive with said target antigen, said antigen binding polypeptide comprising a VH and a VL domain, wherein at least one hypervariable loop or complementarity determining region (CDR) of the VH domain or the VL domain has an amino acid sequence encoded by the nucleotide sequence determined in part (a).

Isolated *Camelidae* VH and VL domains obtained by active immunisation can be used as a basis for engineering antigen binding polypeptides according to the invention. Starting from intact *Camelidae* VH and VL domains, it is possible to engineer one or more amino acid substitutions, insertions or deletions which depart from the starting *Camelidae* sequence. In certain embodiments, such substitutions, insertions or deletions may be present in the framework regions of the VH domain and/or the VL domain. The purpose of such changes in primary amino acid sequence may be to reduce presumably unfavourable properties (e.g. immunogenicity in a human host (so-called humanization), sites of potential product heterogeneity and or instability (glycosylation, deamidation, isomerisation, etc.) or to enhance some other favourable property of the molecule (e.g. solubility, stability, bioavailability, etc.). In other embodiments, changes in primary amino acid sequence can be engineered in one or more of the hypervariable loops (or CDRs) of a *Camelidae* VH and/or VL domain obtained by active immunisation. Such changes may be introduced in order to enhance antigen binding affinity and/or specificity, or to reduce presumably unfavourable properties, e.g. immunogenicity in a human host (so-called humanization), sites of potential product heterogeneity and or instability, glycosylation, deamidation, isomerisation, etc., or to enhance some other favourable property of the molecule, e.g. solubility, stability, bioavailability, etc.

Thus, in one embodiment, the invention provides a recombinant antigen binding polypeptide which contains at least one amino acid substitution in at least one framework or CDR region of either the VH domain or the VL domain in comparison to a *Camelidae* VH or VL domain obtained by active immunisation of a species in the family *Camelidae* with a target antigen. This particular embodiment excludes antigen binding polypeptides containing native *Camelidae* VH and VL domains produced by active immunisation.

As an alternative to "active immunisation" with a target antigen (or a composition comprising the target antigen or a polynucleotide encoding it) it is also possible to make use of immune responses in diseased *Camelidae* animals or naturally occurring immune responses within *Camelidae* species as a source of VH and/or VL domains which can be used as components of antigen binding polypeptides with the desired antigen-binding properties. Such VH/VL domains may also be used as the starting point for engineering antigen-binding polypeptides in an analogous manner to VH/VL domains obtained by active immunisation. Also described is the use of non-immune libraries, and the invention still further encompasses antigen-binding polypeptides obtained/derived therefrom.

In other embodiments, the invention encompasses "chimeric" antibody molecules comprising VH and VL domains from *Camelidae* (or engineered variants thereof) and one or more constant domains from a non-camelid antibody, for example human-encoded constant domains (or engineered variants thereof). Also described are chimeric antigen binding polypeptides (e.g. antibody molecules) wherein one of the VH or the VL domain is camelid-encoded, and the other variable domain is non-camelid (e.g. human). In such embodiments it is preferred that both the VH domain and the VL domain are obtained from the same species of camelid, for example both VH and VL may be from *Lama glama* or both VH and VL may be from *Lama pacos* (prior to introduction of engineered amino acid sequence variation). In such embodiments both the VH and the VL domain may be derived from a single animal, particularly a single animal which has been actively immunised.

As an alternative to engineering changes in the primary amino acid sequence of *Camelidae* VH and/or VL domains, individual *Camelidae* hypervariable loops or CDRs, or combinations thereof, can be isolated from *Camelidae* VH/VL domains and transferred to an alternative (i.e. *non-Camelidae*) framework, e.g. a human VH/VL framework, by CDR grafting.

### Sequence identity/homology with human variable domains

The present inventors have observed that *Camelidae* germline and somatically mutated DNA sequences encoding both the VH and the VL domains of conventional antibodies from species in the family *Camelidae* exhibit a high degree of sequence identity/sequence homology with the human germline DNA sequences which encode VH and VL domains of human antibodies, over the framework regions.

Thus, the antigen binding polypeptides of the invention are characterised in that they exhibit a high degree of amino acid sequence homology with VH and VL domains of human antibodies.

In one embodiment the VH domain of the antigen binding polypeptide according to the invention may exhibit an amino acid sequence identity or sequence homology of 80% or greater with one or more human VH domains across the framework regions FR1, FR2, FR3 and FR4. In other embodiments the amino acid sequence identity or sequence homology between the VH domain of the polypeptide of the invention and one or more human VH domains may be 85% or greater, 90% or greater, 95% or greater, 97% or greater, or up to 99% or even 100%, of course with the proviso that at least one hypervariable loop or CDR is obtained from *Camelidae,* i.e. has an amino acid sequence which is identical (or substantially identical) to the amino acid sequence of a hypervariable loop or CDR encoded by a *Camelidae* VH or VL gene.

In one embodiment the VH domain of the polypeptide of the invention may contain one or more amino acid sequence mis-matches across the framework regions FR1, FR2, FR3 and FR4, in comparison to the closest matched human VH sequence. This latter embodiment would expressly exclude polypeptides comprising a VH domain, or both VH and VL domains, in which the framework region has entirely human sequence.

In another embodiment the VL domain of the antigen binding polypeptide according to the invention may exhibit a sequence identity or sequence homology of 80% or greater with one or more human VL domains across the framework regions FR1, FR2, FR3 and FR4. In other embodiments the amino acid sequence identity or sequence homology between the VL domain of the polypeptide of the invention and one or more human VL domains may be 80% or greater 90% or greater, 95% or greater, 97% or greater, or up to 99% or even 100%.

In one embodiment the VL domain of the polypeptide of the invention may contain one or more amino acid sequence mis-matches across the framework regions FR1, FR2, FR3 and FR4, in comparison to the closest matched human VL sequence. This latter embodiment would expressly exclude polypeptides comprising VL domain, or both VL and VH domains in which the framework region has entirely human sequence.

Also described are antigen binding polypeptides comprising a "fully human" VH or VL domain, provided that only one fully human variable domain is present, and then in combination with a variable domain comprising hypervariable loop(s) or CDR(s) obtained from *Camelidae.*

Representative alignments of *Camelidae* and human germline sequences included in the accompanying examples reveal that the conventional camelid VH and VL domains exhibit a remarkably high sequence homology to their human counterparts. From these examples it can be concluded that typically less than 8, and often only as few as 5 amino acid residues present in the framework regions of a VH or VL domain differ in a given position from the closest human germline-encoded sequences. Given that there are no structural limitations associated with those positions, humanization by site directed mutagenesis is expected to be straightforward.

Therefore, in a particular embodiment, the antigen binding polypeptides of the invention may comprise VH and/or VL domains of conventional *Camelidae* antibodies, for example conventional *Camelidae* antibodies obtained (obtainable) by active immunisation of *camelidae* with a target antigen (or polynucleotide encoding the target antigen), wherein said VH and VL domains have been (independently) engineered to introduce a total of between 1 and 10, i.e. 1, 2, 3, 4, 5, 6, 7, 8, 9 or 10 amino acid substitutions across the framework regions FR1, FR2, FR3 and FR4 in either one or both of the VH domain and the VL domain. Such amino acid substitutions may include (but are not limited to) substitutions which result in "humanisation", by replacing mis-matched amino acid residues in a starting *Camelidae* VH or VL domain with the equivalent residue found in a human germline-encoded VH or VL domain. It is also possible to independently make amino acid substitutions in the hypervariable loops (CDRs) of said camelid-derived VH and VL domains, and such variants may form part of the present invention. References herein to "amino acid substitutions" include substitutions in which a naturally occurring amino acid is replaced with a non-natural amino acid, or an amino acid subjected to post-translational modification.

Before analyzing the percentage sequence identity between *Camelidae* and human germline VH and VL, the canonical folds may be determined, which allows the identification of the family of human germline segments with the identical combination of canonical folds for H1 and H2 or L1 and L2 (and L3). Subsequently the human germline family member that has the highest degree of sequence homology with the *Camelidae* variable region of interest is chosen for scoring the sequence homology. The determination of Chothia canonical classes of hypervariable loops L1, L2, L3, H1 and H2 was performed with the bioinformatics tools publicly available on webpage www.bioinf.org.uk/abs/chothia.html.page. The output of the program shows the key residue requirements in a datafile. In these datafiles, the key residue positions are shown with the allowed amino acids at each position. The sequence of the variable region of the antibody is given as input and is first aligned with a consensus antibody sequence to assign the Kabat numbering scheme. The analysis of the canonical folds uses a set of key residue templates derived by an automated method developed by Martin and Thornton (Martin et al., J. Mol. Biol. 263:800-815 (1996)).

With the particular human germline V segment known, which uses the same combination of canonical folds for H1 and H2 or L1 and L2 (and L3), the best matching family member in terms of sequence homology was determined. With bioinformatics tools the percentage sequence identity between *Camelidae* VH and VL domain framework amino acid sequences and corresponding sequences encoded by the human germline can be determined, but actually manual aligning of the sequences can be applied as well. Human immunoglobulin sequences can be identified from several protein data bases, such as VBase (http://vbase.mrc-cpe.cam.ac.uk/) or the Pluckthun/Honegger database (http://www.bioc.unizh.ch/antibody/Sequences/Germlines. To compare the human sequences to the V regions of *Camelidae* VH or VL domains a sequence alignment algorithm such as available via websites like www.expasy.ch/tools/#align can be used, but also manual alignment with the limited set of sequences can be performed. Human germline light and heavy chain sequences of the families with the same combinations of canonical folds and with the highest degree of homology with the framework regions 1, 2, and 3 of each chain are selected and compared with the *Camelidae* variable region of interest; also the FR4 is checked against the human germline JH and JK or JL regions.

Note that in the calculation of overall percent sequence homology the residues of FR1, FR2 and FR3 are evaluated using the closest match sequence from the human germline family with the identical combination of canonical folds. Only residues different from the closest match or other members of the same family with the same combination of canonical folds are scored (NB - excluding any primer-encoded differences). However, for the purposes of humanization, residues in framework regions identical to members of other human germline families, which do not have the same combination of canonical folds, can be considered "human", despite the fact that these are scored "negative" according to the stringent conditions described above. This assumption is based on the "mix and match" approach for humanization, in which each of FR1, FR2, FR3 and FR4 is separately compared to its closest matching human germline sequence and the humanized molecule therefore contains a combination of different FRs as was done by Qu and colleagues (Qu et la., Clin. Cancer Res. 5:3095-3100 (1999)) and Ono and colleagues (Ono et al., Mol. Immunol. 36:387-395 (1999)).

The boundaries of the individual framework regions may be assigned using the IMGT numbering scheme, which is an adaptation of the numbering scheme of Chothia (Lefranc et al., NAR 27: 209-212 (1999); http://imgt.cines.fr).

Despite the unexpectedly high sequence homology between *Camelidae* and human across the framework regions of the VH and VL domains, it is nevertheless possible to distinguish camelid-encoded hypervariable loops (CDRs) from human-encoded hypervariable loops (CDRs) by straightforward sequence comparison with camelid and human germline VH and VL sequences.

### Structural homology with human-encoded VH and VL domains

In a first aspect the invention provides a monoclonal antigen binding polypeptide comprising a VH domain and a VL domain, wherein each of the hypervariable loops in both the VH domain and the VL domain is obtained from a VH or VL domain of a species in the family *Camelidae*, wherein hypervariable loop H1 and hypervariable loop H2 in the VH domain each exhibit a predicted or actual canonical fold structure which is identical to a canonical fold structure which occurs in human antibodies, and wherein hypervariable loop H1 and hypervariable loop H2 in the VH domain form a combination of predicted or actual canonical fold structures which is identical to a combination of canonical fold structures known to occur in a human germline VH domain.

It is well established in the art that although the primary amino acid sequences of hypervariable loops present in both VH domains and VL domains encoded by the human germline are, by definition, highly variable, all hypervariable loops, except CDR H3 of the VH domain, adopt only a few distinct structural conformations, termed canonical folds (Chothia et al., J. Mol. Biol. 196:901-917 (1987); Tramontano et al. Proteins 6:382-94 (1989)), which depend on both the length of the hypervariable loop and presence of the so-called canonical amino acid residues (Chothia et al., J. Mol. Biol. 196:901-917 (1987)). Actual canonical structures of the hypervariable loops in intact VH or VL domains can be determined by structural analysis (e.g. X-ray crystallography), but it is also possible to predict canonical structure on the basis of key amino acid residues which are characteristic of a particular structure (discussed further below). In essence, the specific pattern of residues that determines each canonical structure forms a "signature" which enables the canonical structure to be recognised in hypervariable loops of a VH or VL domain of unknown structure; canonical structures can therefore be predicted on the basis of primary amino acid sequence alone.

Based on analysis of germline and somatically mutated VH and VL sequences, the present inventors predict that the hypervariable loops of *Camelidae* VH and VL domains (with the exception of H3 in the VH domain and sometimes also L3 in the VL domain) also adopt canonical fold structures which are substantially identical to canonical fold structures adopted by the hypervariable loops of human antibodies.

The predicted canonical fold structures for the hypervariable loops of any given VH or VL sequence in an antigen binding polypeptide can be analysed using algorithms which are publicly available from www.bioinf.org.uk/abs/chothia.html, www.biochem.ucl.ac.uk/∼martin/antibodies.html and www.bioc.unizh.ch/antibody/Sequences/Germlines/Vbase_hVk.html. These tools permit query VH or VL sequences to be aligned against human VH or VL domain sequences of known canonical structure, and a prediction of canonical structure made for the hypervariable loops of the query sequence.

In the case of the VH domain, H1 and H2 loops derived from *Camelidae* may be scored as having a canonical fold structure "substantially identical" to a canonical fold structure known to occur in human antibodies if at least the first, and preferable both, of the following criteria are fulfilled:
1. An identical length, determined by the number of residues, to the closest matching human canonical structural class.
2. At least 33% identity, preferably at least 50% identity with the key amino acid residues described for the corresponding human H1 and H2 canonical structural classes.
(note for the purposes of the foregoing analysis the H1 and H2 loops are treated separately and each compared against its closest matching human canonical structural class)

The foregoing analysis relies on prediction of the canonical structure of the *Camelidae* H1 and H2 loops. If the *actual* structure of the H1 and H2 loops is known, for example based on X-ray crystallography, then the H1 and H2 loops derived from *Camelidae* may also be scored as having a canonical fold structure "substantially identical" to a canonical fold structure known to occur in human antibodies if the length of the loop differs from that of the closest matching human canonical structural class (typically by ±1 or ±2 amino acids) but the *actual* structure of the *Camelidae* H1 and H2 loops matches the structure of a human canonical fold.

Key amino acid residues found in the human canonical structural classes for the first and second hypervariable loops of human VH domains (H1 and H2) are described by Chothia et al., J. Mol. Biol. 227:799-817 (1992). In particular, Table 3 on page 802 of Chothia *et al.,* lists preferred amino acid residues at key sites for H1 canonical structures found in the human germline, whereas Table 4 on page 803 lists preferred amino acid residues at key sites for CDR H2 canonical structures found in the human germline. The accompanying examples contain an analysis of germline VH sequences from *Camelidae* species (specifically llama and dromedary) comparing the actual amino acid residues found in *Camelidae* versus the amino acid residues in the closest human germline VH sequence, for each of the positions in H1 and H2, and underlying framework regions, considered to be key for the canonical fold structure according to the criteria of Chothia et al., J Mol Biol. 227:799-817 (1992). It is observed that the number of identical key residues between camelid and human is most often above 33%, and typically in the range of from 50 to 100%.

In one embodiment, both H1 and H2 in the VH domain of the antigen binding polypeptide of the invention are obtained from a VH domain of a *Camelidae* species, yet exhibit a predicted or actual canonical fold structure which is substantially identical to a canonical fold structure which occurs in human antibodies.

The inventors surmise that it is important not only for the hypervariable loops, specifically H1 and H2 in the VH domain, *individually* to have canonical structures of a type which occurs naturally in human antibodies, it is also important for H1 and H2 in any given VH domain to form a *combination* of canonical fold structures which is identical to a combination of canonical structures known to occur in at least one human germline VH domain. It has been observed that only certain combinations of canonical fold structures at H1 and H2 actually occur in VH domains encoded by the human germline. The present inventors were surprised to discover that every available *Camelidae* germline or somatically mutated VH sequence which could be analysed exhibited not only individual canonical fold structures at H1 and H2 substantially identical to those used in human antibodies, but also the correct *combinations* of structures at H1 and H2 to match combinations found in human antibodies. This represents a distinct advantage over other platforms for production of antibodies for potential therapeutic use in humans which may produce antibodies having "correct" human-like canonical fold structures at H1 and H2 but in a combination which does not occur in human antibodies. By way of example, the inventors' own analysis of the structure of antibodies derived from non-human primates (Biogen IDEC's galiximab (anti-CD80) an lumiliximab (anti-CD23) and the non-human primate mAb against Anthrax Toxin from Pelat et al., J. Mol. Biol. 384:1400-7 (2008)) indicates that structurally they are not consistently very close to the human antibody structure, particularly having regard to the combination of canonical folds. The absence of a correct combination of canonical folds at H1 and H2 could lead to a given antigen binding polypeptide (which is "humanised" in the framework regions) being immunogenic in a human host.

Thus, in a further embodiment H1 and H2 in the VH domain of the antigen binding polypeptide of the invention are obtained from a VH domain of a *Camelidae* species, yet form a combination of predicted or actual canonical fold structures which is identical to a combination of canonical fold structures known to occur in a human germline or somatically mutated VH domain.

In non-limiting embodiments H1 and H2 in the VH domain of the antigen binding polypeptide of the invention are obtained from a VH domain of a *Camelidae* species, and form one of the following canonical fold combinations: 1-1, 1-2, 1-3, 1-6, 1-4, 2-1, 3-1 and 3-5.

It is preferred that the VH domain of the antigen binding polypeptide of the invention exhibit both high sequence identity/sequence homology with human VH, and also that the hypervariable loops in the VH domain exhibit structural homology with human VH.

It may be advantageous for the canonical folds present at H1 and H2 in the VH domain of the antigen binding polypeptide according to the invention, and the combination thereof, to be "correct" for the human VH germline sequence which represents the closest match with the VH domain of the antigen binding polypeptide of the invention in terms of overall primary amino acid sequence identity. By way of example, if the closest sequence match is with a human germline VH3 domain, then it may be advantageous for H1 and H2 (obtained from *Camelidae*) to form a combination of canonical folds which also occurs naturally in a human VH3 domain.

Thus, in one embodiment the VH domain of the antigen binding polypeptide of the invention may exhibit a sequence identity or sequence homology of 80% or greater, 85% or greater, 90% or greater, 95% or greater, 97% or greater, or up to 99% or even 100% with a human VH domain across the framework regions FR1, FR2 , FR3 and FR4, and in addition H1 and H2 in the same antigen binding polypeptide are obtained from a VH domain of a *Camelidae* species, but form a combination of predicted or actual canonical fold structures which is the same as a canonical fold combination known to occur naturally in the same human VH domain.

In other embodiments, L1 and L2 in the VL domain of the antigen binding polypeptide of the invention are each obtained from a VL domain of a *Camelidae* species, and each exhibit a predicted or actual canonical fold structure which is substantially identical to a canonical fold structure which occurs in human antibodies.

As with the VH domains, the hypervariable loops of VL domains of both VLambda and VKappa types can adopt a limited number of conformations or canonical structures, determined in part by length and also by the presence of key amino acid residues at certain canonical positions.

L1, L2 and L3 loops obtained from a VL domain of a *Camelidae* species, yet may be scored as having a canonical fold structure "substantially identical" to a canonical fold structure known to occur in human antibodies if at least the first, and preferable both, of the following criteria are fulfilled:
1. An identical length, determined by the number of residues, to the closest matching human structural class.
2. At least 33% identity, preferably at least 50% identity with the key amino acid residues described for the corresponding human L1 or L2 canonical structural classes, from either the VLambda or the VKappa repertoire.
(note for the purposes of the foregoing analysis the L1 and L2 loops are treated separately and each compared against its closest matching human canonical structural class)

The foregoing analysis relies on prediction of the canonical structure of the *Camelidae* L1, L2 and L3 loops. If the *actual* structure of the L1, L2 and L3 loops is known, for example based on X-ray crystallography, then L1, L2 or L3 loops derived from *Camelidae* may also be scored as having a canonical fold structure "substantially identical" to a canonical fold structure known to occur in human antibodies if the length of the loop differs from that of the closest matching human canonical structural class (typically by ±1 or ±2 amino acids) but the *actual* structure of the *Camelidae* loops matches a human canonical fold.

Key amino acid residues found in the human canonical structural classes for the CDRs of human VLambda and VKappa domains are described by Morea et al. Methods, 20: 267-279 (2000) and Martin et al., J. Mol. Biol., 263:800-815 (1996). The structural repertoire of the human VKappa domain is also described by Tomlinson et al. EMBO J. 14:4628-4638 (1995), and that of the VLambda domain by Williams et al. J. Mol. Biol., 264:220-232 (1996).

The accompanying examples contain an analysis of germline VL sequences or both kappa and lambda type from *Camelidae* species (specifically llama and dromedary), comparing the actual amino acid residues found in *Camelidae* versus the amino acid residues in the closest human germline VLambda or VKappa sequence, for each of the positions in L1 and L2 considered to be key for the canonical fold structure. It is observed that the number of identical key residues between camelid and human is typically in the range of from 33 to 100%, more often between 50 to 100%, typically closer to 100%.

L1 and L2 in the VL domain may form a combination of predicted or actual canonical fold structures which is identical to a combination of canonical fold structures known to occur in a human germline VL domain.

In non-limiting embodiments L1 and L2 in the VLambda domain may form one of the following canonical fold combinations: 11-7, 13-7(A,B,C), 14-7(A,B), 12-11, 14-11 and 12-12 (as defined in Williams et al. J. Mol. Biol. 264:220 -32 (1996) and as shown on http://www.bioc.uzh.ch/antibody/Sequences/Germlines/VBase hVL.html). In non-limiting embodiments L1 and L2 in the Vkappa domain may form one of the following canonical fold combinations: 2-1, 3-1, 4-1 and 6-1 (as defined in Tomlinson et al. EMBO J. 14:4628-38 (1995) and as shown on http://www.bioc.uzh.ch/antibody/Sequences/Germlines/VBase_hVK.html).

In a further embodiment, all three of L1, L2 and L3 in the VL domain may exhibit a substantially human structure. Most human Vκ germline segments encode also a single conformation of the L3 loop (type 1), which is stabilized by the conserved cis-proline on position 95, but other conformations in rearranged sequences are possible due to the process of V-J joining and the potential loss of this proline residue. The publicly available somatically mutated dromedary Vκ sequences have a type 1 canonical fold for L3(κ) like is found in human kappa germline sequences, and Proline on position 95 occurs in six out of seven dromedary Vκ domains. Therefore, where the antigen binding polypeptide contains a Vκ domain, this domain may possess the conserved Proline residue on position 95.

The structural repertoire of the human VL germline sequences was analyzed by Williams and colleagues (Williams et al., J. Mol. Biol. 264:220-232 (1996)). The three families analyzed here encode identical conformations of the L2 loop. The L3 loop conformation is thought to be more highly variable, as there is some length variation and no cis-proline residue. Indeed the available somatically mutated dromedary Vλ sequences show a high variability in the length of L3. Most of these have a canonical fold for L3 (f.i. VLambda 3-1 family members Camvl19 (10A) and Camvl20 (1/9A), VLambda 2-18 family members Camvl5, 17, 30, 36 and 52 (all 10B) and VLambda 1-40 family member Camvl44 (5/11A)).

It is preferred that the VL domain of the antigen binding polypeptide of the invention exhibit both high sequence identity/sequence homology with human VL, and also that the hypervariable loops in the VL domain exhibit structural homology with human VL.

In one embodiment, the VL domain of the antigen binding polypeptide of the invention may exhibit a sequence identity of 80% or greater, 85% or greater, 90% or greater, 95% or greater, 97% or greater, or up to 99% or even 100% with a human VL domain across the framework regions FR1, FR2 , FR3 and FR4, and in addition hypervariable loop L1 and hypervariable loop L2 may form a combination of predicted or actual canonical fold structures which is the same as a canonical fold combination known to occur naturally in the same human VL domain.

It is, of course, envisaged that VH domains exhibiting high sequence identity/sequence homology with human VH, and also structural homology with hypervariable loops of human VH will be combined with VL domains exhibiting high sequence identity/sequence homology with human VL, and also structural homology with hypervariable loops of human VL to provide antigen binding polypeptides containing (camelid-derived) VH/VL pairings with maximal sequence and structural homology to human-encoded VH/VL pairings. A particular advantage of the camelid platform provided by the invention is that both the VH domain and the VL domain exhibit high sequence and structual homology with the variable domains of human antibodies.

### Structure of the antigen binding polypeptide

The antigen binding polypeptide of the invention can take various different embodiments, provided that both a VH domain and a VL domain are present. Thus, in non-limiting embodiments the antigen binding polypeptide may be an immunoglobulin, an antibody or antibody fragment. The term "antibody" herein is used in the broadest sense and encompasses, but is not limited to, monoclonal antibodies (including full length monoclonal antibodies), polyclonal antibodies, multispecific antibodies (e.g., bispecific antibodies), so long as they exhibit the appropriate specificity for a target antigen. The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes) on the antigen, each monoclonal antibody is directed against a single determinant or epitope on the antigen.

"Antibody fragments" comprise a portion of a full length antibody, generally the antigen binding or variable domain thereof. Examples of antibody fragments include Fab, Fab', F(ab')2, bi-specific Fab's, and Fv fragments, diabodies, linear antibodies, single-chain antibody molecules, a single chain variable fragment (scFv) and multispecific antibodies formed from antibody fragments (see Holliger and Hudson, Nature Biotechnol. 23:1126-36 (2005).

In non-limiting embodiments, antibodies and antibody fragments according to the invention may comprise CH1 domains and/or CL domains, the amino acid sequence of which is fully or substantially human. Where the antigen binding polypeptide of the invention is an antibody intended for human therapeutic use, it is typical for the entire constant region of the antibody, or at least a part thereof, to have fully or substantially human amino acid sequence. Therefore, an antibody of the invention must comprise VH and VL domains, at least one of which includes at least one hypervariable loop derived from *Camelidae*, but one or more or any combination of the CH1 domain, hinge region, CH2 domain, CH3 domain and CL domain (and CH4 domain if present) may be fully or substantially human with respect to it's amino acid sequence.

Advantageously, the CH1 domain, hinge region, CH2 domain, CH3 domain and CL domain (and CH4 domain if present) may *all* have fully or substantially human amino acid sequence. In the context of the constant region of a humanised or chimeric antibody, or an antibody fragment, the term "substantially human" refers to an amino acid sequence identity of at least 90%, or at least 95%, or at least 97%, or at least 99% with a human constant region. The term "human amino acid sequence" in this context refers to an amino acid sequence which is encoded by a human immunoglobulin gene, which includes germline, rearranged and somatically mutated genes. The invention also contemplates polypeptides comprising constant domains of "human" sequence which have been altered, by one or more amino acid additions, deletions or substitutions with respect to the human sequence.

As discussed elsewhere herein, it is contemplated that one or more amino acid substitutions, insertions or deletions may be made within the constant region of the heavy and/or the light chain, particularly within the Fc region. Amino acid substitutions may result in replacement of the substituted amino acid with a different naturally occurring amino acid, or with a non-natural or modified amino acid. Other structural modifications are also permitted, such as for example changes in glycosylation pattern (e.g. by addition or deletion of N- or O-linked glycosylation sites). Depending on the intended use of the antibody, it may be desirable to modify the antibody of the invention with respect to its binding properties to Fc receptors, for example to modulate effector function. For example cysteine residue(s) may be introduced in the Fc region, thereby allowing interchain disulfide bond formation in this region. The homodimeric antibody thus generated may have improved internalization capability and/or increased complement-mediated cell killing and antibody-dependent cellular cytotoxicity (ADCC).

See Caron et al., J. Exp. Med. 176:1191 -1195 (1992) and Shopes, B. J. Immunol. 148:2918-2922 (1992). Alternatively, an antibody can be engineered which has dual Fc regions and may thereby have enhanced complement lysis and ADCC capabilities. See Stevenson et al., Anti-Cancer Drug Design 3:219-230 (1989). The invention also contemplates immunoconjugates comprising an antibody as described herein conjugated to a cytotoxic agent such as a chemotherapeutic agent, toxin (e.g., an enzymatically active toxin of bacterial, fungal, plant or animal origin, or fragments thereof), or a radioactive isotope (i.e., a radioconjugate). Fc regions may also be engineered for half-life extension.

The invention can, in certain embodiments, encompass chimeric *Camelidae*/*human* antibodies, and in particular chimeric antibodies in which the VH and VL domains are of fully camelid sequence (e.g. Llama or alpaca) and the remainder of the antibody is of fully human sequence. In preferred embodiments the invention also encompasses "humanised" or "germlined" *Camelidae* antibodies, and *Camelidae*/human chimeric antibodies, in which the VH and VL domains contain one or more amino acid substitutions in the framework regions in comparison to *Camelidae* VH and VL domains obtained by active immunisation. Such "humanisation" increases the % sequence identity with human germline VH or VL domains by replacing mis-matched amino acid residues in a starting *Camelidae* VH or VL domain with the equivalent residue found in a human germline-encoded VH or VL domain.

The invention still further encompasses CDR-grafted antibodies in which CDRs (or hypervariable loops) derived from a *Camelidae* antibody, for example an *Camelidae* antibody raised by active immunisation with a target antigen, or otherwise encoded by a camelid gene, are grafted onto a human VH and VL framework, with the remainder of the antibody also being of fully human origin. However, given the high degree of amino acid sequence homology and structural homology they have observed between *Camelidae* and human immunoglobulins, the inventors anticipate that in the majority of cases it will be possible to achieve the levels of human homology required for *in vivo* therapeutic use via "humanisation" of the framework regions of camelid-derived VH and VL domains without the need for CDR grafting or via CDR grafting on to limited number of backbone sequences without the need for veneering (also see Almagro et al, Frontiers in Bioscience 13: 1619-1633 (2008).

Humanised, chimeric and CDR-grafted antibodies according to the invention, particularly antibodies comprising hypervariable loops derived from active immunisation of *Camelidae* with a target antigen, can be readily produced using conventional recombinant DNA manipulation and expression techniques, making use of prokaryotic and eukaryotic host cells engineered to produce the polypeptide of interest and including but not limited to bacterial cells, yeast cells, mammalian cells, insect cells, plant cells , some of them as described herein and illustrated in the accompanying examples.

The invention also encompasses antigen binding polypeptides wherein either one or other of the VH or VL domain is obtained from *Camelidae*, or contains at least one CDR or hypervariable region derived from *Camelidae*, and the "other" variable domain has non-camelid, e.g. human, amino acid sequence. Thus, it is contemplated to pair a camelid VH domain with a human VL domain, or to pair a human VH domain with a camelid VL domain. Such pairings may increase the available antigen-binding repertoire from which to select high affinity binders with the desired antigen binding properties.

The invention still further extends to antigen binding polypeptides wherein the hypervariable loop(s) or CDR(s) of the VH domain and/or the VL domain are obtained from Camelidae, but wherein at least one of said (camelid-derived) hypervariable loops or CDRS has been engineered to include one or more amino acid substitutions, additions or deletions relative to the camelid-encoded sequence. Such changes include "humanisation" of the hypervariable loops/CDRs. Camelid-derived HVs/CDRs which have been engineered in this manner may still exhibit an amino acid sequence which is "substantially identical" to the amino acid sequence of a camelid-encoded HV/CDR. In this context, "substantial identity" may permit no more than one, or no more than two amino acid sequence mis-matches with the camelid-encoded HV/CDR.

Antibodies according to the invention may be of any isotype. Antibodies intended for human therapeutic use will typically be of the IgA, IgD, IgE IgG, IgM type, often of the IgG type, in which case they can belong to any of the four sub-classes IgG1, IgG2a and b, IgG3 or IgG4. Within each of these sub-classes it is permitted to make one or more amino acid substitutions, insertions or deletions within the Fc portion, or to make other structural modifications, for example to enhance or reduce Fc-dependent functionalities.

Antigen binding polypeptides according to the invention may be useful in a wide range of applications, both in research and in the diagnosis and/or treatment of diseases. Because of the high degree of amino acid sequence identity with the VH and VL domains of natural human antibodies, and the high degree of structural homology (specifically the correct combinations of canonical folds as are found in human antibodies) the antigen binding polypeptides of the invention, particularly in the form of monoclonal antibodies, will find particular utility as human therapeutic agents.

The invention provides a platform for production of antigen binding polypeptides, and specifically monoclonal antibodies, against a wide range of antigens and in its broadest aspect the invention is not intended to be limited with respect to the exact identity of the target antigen, nor indeed the specificity or affinity of binding to the target antigen. However, in particular, non-limiting, embodiments the target antigen may be a non-camelid antigen, a bacterial antigen, a viral antigen or a human antigen. In a preferred embodiment the target antigen may be an antigen of particular therapeutic importance. The term "target of therapeutic importance" refers to a target involved in formation, onset, progression, mediation of human or animal diseases or of the effects related to the respective disease. Included within this definition are targets wherein the expression levels and/or activity of the target are modulated by antibody binding (e.g. receptors whose activity may be modulated by binding of agonist or antagonist antibodies), and targets wherein the activity and/or expression of the target has a direct or indirect impact on a disease.

By way of example, "human antigens" may include naturally occurring human polypeptides (proteins) which function as receptors, receptor ligands, cell-signalling molecules, hormones, cytokines or cytokine receptors, neurotransmitters, etc. By "naturally occurring" is meant that the polypeptide is expressed within the human body, at any stage if its development, including polypeptides expressed by the human body during the course of a disease.

Non-limiting embodiments of the antigen binding polypeptide of the invention include the following:
A chimeric antigen binding polypeptide comprising a VH domain and a VL domain, wherein each of the hypervariable loops or complementarity determining regions (CDRs) in the VH domain or the VL domain is obtained from a VH or VL domain of a species in the family *Camelidae.* In a particular embodiment both the VH domain and the VL domain are obtained from Llama (*Lama glama*).

A recombinantly expressed antigen binding polypeptide comprising a VH domain and a VL domain, wherein each of the hypervariable loops or complementarity determining regions (CDRs) in the VH domain or the VL domain is obtained from a VH or VL domain of a species in the family *Camelidae.* In a particular embodiment both the VH domain and the VL domain are obtained from Llama (*Lama glama*).

A monoclonal antibody comprising a VH domain and a VL domain, wherein each of the hypervariable loops or complementarity determining regions (CDRs) in the VH domain or the VL domain is obtained from a VH or VL domain of a species in the family *Camelidae.* In a particular embodiment both the VH domain and the VL domain are obtained from Llama (*Lama glama*).

An antigen binding polypeptide comprising a VH domain and a VL domain, wherein each of the hypervariable loops or complementarity determining regions (CDRs) in the VH domain or the VL domain is obtained from a VH or VL domain of a species in the family *Camelidae* and wherein said antigen binding polypeptide is immunoreactive with a target antigen of therapeutic or diagnostic importance. In a particular embodiment both the VH domain and the VL domain are obtained from Llama (*Lama glama*).

A chimeric antigen binding polypeptide comprising or consisting of a VH domain of a conventional antibody of a camelid (in particular Llama or alpaca), a VL domain of a conventional antibody of a camelid (in particular Llama or alpaca) and one or more constant domains of a human antibody. In a particular embodiment both the VH domain and the VL domain are obtained from Llama (*Lama glama*).

A chimeric antigen binding polypeptide immunoreactive with a target antigen of therapeutic or diagnostic importance, which antigen binding polypeptide comprises or consists of a VH domain of a conventional antibody of a camelid (in particular Llama or alpaca), a VL domain of a conventional antibody of a camelid (in particular Llama or alpaca) and one or more constant domains of a human antibody. In a particular embodiment both the VH domain and the VL domain are obtained from Llama (*Lama glama*).

A chimeric antibody comprising or consisting of a VH domain of a conventional antibody of a camelid (in particular Llama or alpaca), a VL domain of a conventional antibody of a camelid (in particular Llama or alpaca) and the constant domains of a human antibody of an isotype selected from the group consisting of: IgG, IgM, IgD, IgE and IgA. In a particular embodiment both the VH domain and the VL domain are obtained from Llama (*Lama glama*).

A chimeric antigen binding polypeptide immunoreactive with a target antigen of therapeutic or diagnostic importance, which antigen binding polypeptide comprises or consists of a VH domain of a conventional antibody of a camelid (in particular Llama or alpaca), a VL domain of a conventional antibody of a camelid (in particular Llama or alpaca) and the constant domains of a human antibody of an isotype selected from the group consisting of: IgG, IgM, IgD, IgE, IgA. In a particular embodiment both the VH domain and the VL domain are obtained from Llama (*Lama glama*).

In particular embodiments of the foregoing, both the VH and the VL domain may be from the same species of camelid (in particular Llama or alpaca), and may even be from the same animal within this species, for example a single animal which has been actively immunised. In particular, both the VH domain and the VL domain may be obtained from a single actively immunised Llama. However, it is not excluded that the VH and VL domain may be obtained from different animals, or non-immune libraries.

In the foregoing embodiments, the terms "VH domain of a conventional antibody of a camelid" and "VL domain of a conventional antibody of a camelid" are intended to encompass variants which have been engineered to introduce one or more changes in amino acid sequence, such as variants which have been "humanised" or "germlined" in one or more framework regions, as described elsewhere herein, and also encompass the products of synthetic (e.g. codon-optimised) genes, as described elsewhere herein.

### Polynucleotides, vectors and recombinant expression

The invention also provides a polynucleotide molecule encoding the antigen binding polypeptide of the invention, an expression vector containing a nucleotide sequence encoding the antigen binding polypeptide of the invention operably linked to regulatory sequences which permit expression of the antigen binding polypeptide in a host cell or cell-free expression system, and a host cell or cell-free expression system containing this expression vector.

Polynucleotide molecules encoding the antigen binding polypeptide of the invention include, for example, recombinant DNA molecules.

The terms "nucleic acid", "polynucleotide" or a "polynucleotide molecule" as used herein interchangeably and refer to any DNA or RNA molecule, either single- or doublestranded and, if single-stranded, the molecule of its complementary sequence. In discussing nucleic acid molecules, a sequence or structure of a particular nucleic acid molecule may be described herein according to the normal convention of providing the sequence in the 5' to 3' direction. In some embodiments of the invention, nucleic acids or polynucleotides are "isolated." This term, when applied to a nucleic acid molecule, refers to a nucleic acid molecule that is separated from sequences with which it is immediately contiguous in the naturally occurring genome of the organism in which it originated. For example, an "isolated nucleic acid" may comprise a DNA molecule inserted into a vector, such as a plasmid or virus vector, or integrated into the genomic DNA of a prokaryotic or eukaryotic cell or non-human host organism. When applied to RNA, the term "isolated polynucleotide" refers primarily to an RNA molecule encoded by an isolated DNA molecule as defined above. Alternatively, the term may refer to an RNA molecule that has been purified/separated from other nucleic acids with which it would be associated in its natural state (i.e., in cells or tissues). An isolated polynucleotide (either DNA or RNA) may further represent a molecule produced directly by biological or synthetic means and separated from other components present during its production.

For recombinant production of an antigen binding polypeptide according to the invention, a recombinant polynucleotide encoding it may be prepared (using standard molecular biology techniques) and inserted into a replicable vector for expression in a chosen host cell, or a cell-free expression system. Suitable host cells may be prokaryote, yeast, or higher eukaryote cells, specifically mammalian cells. Examples of useful mammalian host cell lines are monkey kidney CV1 line transformed by SV40 (COS-7, ATCC CRL 1651); human embryonic kidney line (293 or 293 cells subcloned for growth in suspension culture, Graham et al., J. Gen. Virol. 36:59 (1977)); baby hamster kidney cells (BHK, ATCC CCL 10); Chinese hamster ovary cells/-DHFR (CHO, Urlaub et al., Proc. Natl. Acad. Sci. USA 77:4216 (1980)); mouse sertoli cells (TM4, Mather, Biol. Reprod. 23:243-251 (1980)); mouse myeloma cells SP2/0-AG14 (ATCC CRL 1581; ATCC CRL 8287) or NS0 (HPA culture collections no. 85110503); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci. 383:44-68 (1982)); MRC 5 cells; FS4 cells; and a human hepatoma line (Hep G2), as well as DSM's PERC-6 cell line. Expression vectors suitable for use in each of these host cells are also generally known in the art.

It should be noted that the term "host cell" generally refers to a cultured cell line. Whole human beings into which an expression vector encoding an antigen binding polypeptide according to the invention has been introduced are explicitly excluded from the scope of the invention.

In an important aspect, the invention also provides a method of producing a recombinant antigen binding polypeptide which comprises culturing a host cell (or cell free expression system) containing polynucleotide (e.g. an expression vector) encoding the recombinant antigen binding polypeptide under conditions which permit expression of the antigen binding polypeptide, and recovering the expressed antigen binding polypeptide. This recombinant expression process can be used for large scale production of antigen binding polypeptides according to the invention, including monoclonal antibodies intended for human therapeutic use. Suitable vectors, cell lines and production processes for large scale manufacture of recombinant antibodies suitable for *in vivo* therapeutic use are generally available in the art and will be well known to the skilled person.

Further aspects of the invention relate to test kits, including diagnostic kits etc. comprising an antigen binding polypeptide according to the invention, and also pharmaceutical formulations comprising an antigen binding polypeptide according to the invention.

Where the antigen binding polypeptide is intended for diagnostic use, for example where the antigen binding polypeptide is specific for an antigen which is a biomarker of a disease state or a disease susceptibility, then it may be convenient to supply the antigen binding polypeptide as a component of a test kit. Diagnostic tests typically take the form of standard immunoassays, such as ELISA, radioimmunoassay, Elispot, etc. The components of such a test kit may vary depending on the nature of the test or assay it is intended to carry out using the antigen binding polypeptide of the invention, but will typically include additional reagents required to carry out an immunoassay using the antigen binding polypeptide of the invention. Antigen binding polypeptides for use as diagnostic reagents may carry a revealing label, such as for example a fluorescent moiety, enzymatic label, or radiolabel.

Antigen binding polypeptides intended for *in vivo* therapeutic use are typically formulated into pharmaceutical dosage forms, together with one or more pharmaceutically acceptable diluents, carriers or excipients (Remington's Pharmaceutical Sciences, 16th edition., Osol, A. Ed. 1980). Antigen binding polypeptides according to the invention are typically formulated as sterile aqueous solutions, to be administered intravenously, or by intramuscular, intraperitoneal, intra-cerebrospinal, intratumoral, oral, peritumoral, subcutaneous, intra-synovial, intrathecal, topical, sublingual or inhalation routes, to a mammalian subject, typically a human patient, in need thereof. For the prevention or treatment of disease, the appropriate dosage of antigen binding polypeptide will depend on the type of disease to be treated, the severity and clinical course of the disease, plus the patient's age, weight and clinical history, and will be determined by the judgement of the attending physician.

### Processes for the production of antigen binding polypeptides

Also described herein are processes for the production of high affinity antigen binding polypeptides, and specifically monoclonal antibodies, against a target antigen of interest.

Accordingly, described herein is a process for preparing an antigen binding polypeptide immunoreactive with a target antigen, said process comprising:
(a) determining the nucleotide sequence encoding at least one hypervariable loop or complementarity determining region (CDR) of the VH and/or the VL domain of a *Camelidae* conventional antibody immunoreactive with said target antigen; and
(b) expressing an antigen binding polypeptide immunoreactive with said target antigen, said antigen binding polypeptide comprising a VH and a VL domain, wherein at least one hypervariable loop or complementarity determining region (CDR) of the VH domain or the VL domain has an amino acid sequence encoded by the nucleotide sequence determined in part (a).

The antigen binding polypeptide expressed in part (b) may be not identical to the *Camelidae* conventional antibody of part (a).

For example, described herein is a process for preparing a recombinant antigen binding polypeptide that is immunoreactive with (or specifically binds to) a target antigen, said an antigen binding polypeptide comprising a VH domain and a VL domain, wherein at least one hypervariable loop or complementarity determining region (CDR) in the VH domain or the VL domain is obtained from a species in the family *Camelidae*, said process comprising the steps of:
(a) isolating *Camelidae* nucleic acid encoding at least one hypervariable loop or complementarity determining region (CDR) of the VH and/or the VL domain of a *Camelidae* conventional antibody immunoreactive with said target antigen;
(b) preparing a recombinant polynucleotide comprising a nucleotide sequence encoding hypervariable loop(s) or complementarity determining region(s) having amino acid sequence identical to the hypervariable loop(s) or complementarity determining region(s) encoded by the nucleic acid isolated in step (a), which recombinant polynucleotide encodes an antigen binding polypeptide comprising a VH domain and a VL domain that is immunoreactive with (or specifically binds to) said target antigen; and
(c) expressing said antigen binding polypeptide from the recombinant polynucleotide of step (b).

The antigen binding polypeptide expressed in part (c) may be not identical to the *Camelidae* conventional antibody of part (a).

The foregoing methods may be referred to herein as "general processes" for preparing antigen binding polypeptides.

The first step of either process may involve active immunisation of a species in the family *Camelidae* in order to elicit an immune response against the target antigen, thereby raising camelid *conventional* antibodies immunoreactive with the target antigen. Protocols for immunisation of camelids are described in the accompanying examples. The antigen preparation used for immunisation may be a purified form of the target antigen, for example recombinantly expressed polypeptide, or an immunogenic fragment thereof. However, it is also possible to immunise with crude preparations of the antigen, such as like isolated cells or tissue preparations expressing or encoding the target antigen, cell lysates, cell supernatants or fractions such as cell membranes, etc., or with a polynucleotide encoding said target antigen (a DNA immunisation).

The process will typically involve immunisation of animals of a *Camelidae* species (including, but limited to, llamas and alpacas), and advantageously these animals will belong to an outbred population. However, it is also contemplated to use transgenic animals (e.g. transgenic mice) containing the Camelid conventional Ig locus, or at least a portion thereof.

A topic of increasing interest seems to be the difference between the complementarity determining regions (CDRs) of *in vivo* and *in vitro* generated antibodies. The inventors surmise that the *in vivo* selection has a favourable impact on the immunogenicity, functionality, stability and therefore improved manufacturability of the resulting antibodies, whilst synthetic CDRs generated and selected *in vitro* may have a disadvantage from this point of view. This is important since a given therapeutic antibody risks to be neutralized by the so called anti-idiotypic antibody response from the patient (Lonberg, Nature Biotechnology, 23: 1117-1125, (2005)).

A key advantage of the described processes based on active immunisation of camelids stems from the fact that all species of *Camelidae* can be maintained in large outbred populations where the individual animals have a different genetic background. It is therefore possible to use active immunisation to elicit a strong and diverse immune response against the antigen of interest from which a diverse pool of potential antigen binding molecules can be obtained. As illustrated in the accompanying examples, the present inventors have observed that active immunisation of camelids can generate Fab fragments binding to a target antigen with a high degree of immunodiversity. Without wishing to be bound by theory, the inventors surmise that the phylogenetic distance between humans and camelids may be important for production of a diverse immune response against a given target antigen. In contrast, the non-human primates are phylogenetically close to humans, thus targets with high homology between non-human primates and humans may elicit only a limited immune response in terms of strength and diversity in non-human primates.

The ability to use active immunisation in an outbred population which is phylogenetically distant from human would not be particularly advantageous if the antibodies so-produced were to exhibit a low sequence and structural homology with human antibodies such that substantial "protein engineering" would be required to create a candidate antibody with therapeutic potential. It is therefore extremely important that the inventors have shown that the *Camelidae* germline (and somatically mutated sequences) encodes both VH and VL domains with a very high degree of sequence and structural homology with human VH and VL domains (as explained above). This high degree of homology in *combination* with the availability of large outbred populations results in a very powerful platform for development of monoclonal antibodies for use as human therapeutics.

Following active immunisation with the target antigen, peripheral blood lymphocytes or biopsies such as lymph nodes or spleen biopsies may be isolated from the immunised animal and screened for production of conventional camelid antibodies against the target antigen. Techniques such as enrichment using panning or FACS sorting may be used at this stage to reduce the complexity of the B cell repertoire to be screened, as illustrated in the examples. Antigen-specific B cells are then selected and used for total RNA extraction and subsequent cDNA synthesis. Nucleic acid encoding the native camelid VH and VL domains (specific for the target antigen) can be isolated by PCR.

It is not essential to use active immunisation in order to identify camelid convention antibodies immunoreactive with a target of interest. It may be possible to make use of the camelid's own immune response, either the immunodiversity naturally present in the animal, or for example a diseased animal or animal which has been naturally exposed to a particular pathogen, e.g. by normal infection routes. In this regard, also described is the use of non-immune libraries. If "natural" immune responses within the camelid already give rise to antibodies which bind the target antigen of interest, then it is possible to make use of the genetic engineering techniques described herein, and other standard techniques known in the art, in order to culture and isolate B cells producing such antibodies, or produce monoclonal cultures of such antibodies, and/or to determine the nucleotide sequence of the camelid gene segments encoding the VH and/or VL domains of such antibodies. Armed with this sequence information, it is then possible to engineer recombinant DNA constructs encoding antigen binding polypeptides which embody the camelid derived VH and/or VL, or the hypervariable loops (or CDRs) thereof.

Nucleic acid encoding camelid VH and VL domains (whether obtained by active immunisation or by other means) may be cloned directly into an expression vector for the production of an antigen binding polypeptide according to the invention. In particular, these sequences could be cloned into an expression vector which also encodes a human antibody constant region, or a portion thereof, in order to produce a chimeric antibody. However, it is typical to carry out further manipulations on the isolated camelid VH and VL sequences before cloning and expression with human constant region sequences.

As a first step, candidate camelid VH and VL sequences (including sequences isolated following the active immunisation) may be used to prepare a camelid libraries (e.g. Fab libraries, as described in the accompanying examples). The library may then be screened (e.g. using phage display) for binding to the target antigen. Promising lead candidates can be further tested for target antigen binding, for example using Biacore or a suitable bioassay. Finally, the sequences encoding the VH and VL domains of the most promising leads can be cloned as an in-frame fusion with sequences encoding a human antibody constant region.

It is not essential that the polynucleotide sequence used to *encode* the (camelid-derived) HVs/CDRs (e.g. for recombinant expression of the antigen binding polypeptide of the invention) is identical to the native polynucleotide sequence which naturally encodes the HVs/CDRs in the camelid. Therefore, the invention encompasses/permits codon optimisation, and other changes in polynucleotide sequence related to cloning and/or expression, which do not alter the encoded amino acid sequence.

"Chain shuffling" may be performed in which a particular variable domain known to bind the antigen of interest is paired with each of a set of variable domains of the opposite type (i.e. VH paired with VL library or *vice versa*), to create libraries, and the resulting "promiscuous" combinations of VH/VL tested for antigen binding affinity and/or specificity. Alternatively, a library of VH domains could be paired with a library of VL domains, either randomly or in a hierarchical manner, and the resulting combinations tested (see Clackson et al., Nature., Vol. 352. pp624-638, 1991). In this process, the libraries may be libraries of rearranged VH and VL (Vκ or Vλ) from camelids which display immunity to the antigen of interest (including animals which have been actively immunised). The chain shuffling process can increase immunodiversity and produce pairings with significantly enhanced affinity.

The described process also contemplates performing epitope imprinted selection (so-called "guided selection") starting from a camelid VH or VL domain, wherein the other variable domain is taken from a non-camelid species, e.g. human. Thus, a camelid VH domain may be "shuffled" with a library of human-encoded VL domains, to replace the native camelid-encoded VL domain, resulting in camelid VH/human VL pairings. One or more of these pairings may then be subjected a second chain shuffling step in which the human VL domain is shuffled against a library of VH domains, which may be human-encoded. This second step may produce human-encoded VH/VL combinations which have the epitope imprint of the original camelid-encoded VH/VL combination.

Also described is the reverse "chain shuffling" process, starting with non-camelid (preferably human)-encoded VH/VL domain combination which binds to an antigen of interest. This could be, for example, a fully human therapeutic antibody against a validated disease target. Starting from this VH/VL combination, it is possible to carry out a first round of selection in which the VH domain is "shuffled" with a library of camelid-encoded VL domains (or *vice versa*), and the pairings tested for antigen binding. Selected non-camelid (e.g. human) VH/camelid VL pairings may then be subjected to a second round of selection in which the camelid-encoded VL is shuffled against a library of camelid-encoded VH, and the resulting pairings tested for antigen binding. As a result, it may be possible to produce a camelid VH/camelid VL combination which carries the epitope imprint of the starting VH/VL combination. This camelid VH/VL combination could be further engineered/modified and combined with human-encoded constant domains as required, using any of the processes described herein.

In the described processes, "native" camelid-derived VH and VL domains may be subject to protein engineering in which one or more selective amino acid substitutions are introduced, typically in the framework regions. The reasons for introducing such substitutions into the "wild type" camelid sequence can be (i) humanisation of the framework region, (ii) improvement in stability, bioavailability, product uniformity, tissue penetration, etc., or (iii) optimisation of target antigen binding.

"Humanisation" of camelid-derived VH and VL domains by selective replacement of one or more amino acid residues in the framework regions may be carried out according to well-established principles (as illustrated in the accompanying examples, and reviewed by Almagro et al. Frontiers in Bioscience 13:1619-1633 (2008). It will be appreciated that the precise identity of the amino acid changes made to achieve acceptable "humanisation" of any given VH domain, VL domain or combination thereof will vary on a case-by-case basis, since this will depend upon the sequence of the framework regions derived from *Camelidae* and the starting homology between these framework regions and the closest aligning human germline (or somatically mutated) framework region, and possible also on the sequence and conformation of the hypervariable loops which form the antigen binding site.

The overall aim of humanisation is to produce a molecule in which the VH and VL domains exhibit minimal immunogenicity when introduced into a human subject, whilst retaining the specificity and affinity of the antigen binding site formed by the parental VH and VL domains encoded by *Camelidae* (e.g. camelid VH/VL obtained by active immunisation). There are a number of established approaches to humanisation which can be used to achieve this aim. Techniques can be generally classified as either rational approaches or empirical approaches. Rational approaches include CDR-grafting, resurfacing or veneering, superhumanization and human string content optimisation. Empirical approaches include the FR library approach, guided selection, FR shuffling and humaneering. All of these techniques are reviewed in Almagro, Frontiers in Bioscience 2008, *ibid.* and any of these techniques, or combinations or modifications thereof, can be used to prepare "humanised" antigen binding polypeptides according to the invention.

### Methods of library construction

Also described is a method of producing a library of expression vectors encoding VH and/or VL domains of camelid conventional antibodies, said method comprising the steps:
a) amplifying regions of nucleic acid molecules encoding VH and/or VL domains of camelid conventional antibodies to obtain amplified gene segments, each gene segment containing a sequence of nucleotides encoding a VH domain or a sequence of nucleotides encoding a VL domain of a camelid conventional antibody, and
b) cloning the gene segments obtained in a) into expression vectors, such that each expression vector contains at least a gene segment encoding a VH domain and/or a gene segment encoding a VL domain, whereby a library of expression vectors is obtained.

The above methods of "library construction" may also form part of the general process for production of antigen binding polypeptides of the invention, described above. Hence, any feature described as being preferred or advantageous in relation to this aspect of the invention may also be taken as preferred or advantageous in relation to the general process, and *vice versa*, unless otherwise stated.

For example, the nucleic acid amplified in step a) comprises cDNA or genomic DNA prepared from lymphoid tissue of a camelid, said lymphoid tissue comprising one or more B cells, lymph nodes, spleen cells, bone marrow cells, or a combination thereof. Circulating B cells are particularly preferred. The present inventors have surprisingly found that peripheral blood lymphocytes (PBLs) can be used as a source of nucleic acid encoding VH and VL domains of conventional camelid antibodies, i.e. there is sufficient quantity of plasma cells (expressing antibodies) present in a sample of PBLs to enable direct amplification. This is advantageous because PBLs can be prepared from a whole blood sample taken from the animal (camelid). This avoids the need to use invasive procedures to obtain tissue biopsies (e.g. from spleen or lymph node), and means that the sampling procedure can be repeated as often as necessary, with minimal impact on the animal. For example, it is possible to actively immunise the camelid, remove a first blood sample from the animal and prepare PBLs, then immunise the same animal a second time, either with a "boosting" dose of the same antigen or with a different antigen, then remove a second blood sample and prepare PBLs.

Accordingly, a particular example of this method may involve: preparing a sample containing PBLs from a camelid, preparing cDNA or genomic DNA from the PBLs and using this cDNA or genomic DNA as a template for amplification of gene segments encoding VH or VL domains of camelid conventional antibodies.

The lymphoid tissue (e.g. circulating B cells) may be obtained from a camelid which has been actively immunised, as described elsewhere herein. However, this is non-limiting and it is also contemplated to prepare non-immune libraries and libraries derived from lymphoid tissue of diseased camelids, also described elsewhere herein.

Conveniently, total RNA (or mRNA) can be prepared from the lymphoid tissue sample (e.g. peripheral blood cells or tissue biopsy) and converted to cDNA by standard techniques. It is also possible to use genomic DNA as a starting material.

The described processes encompass both a diverse library approach, and a B cell selection approach for construction of the library. In a diverse library approach, repertoires of VH and VL-encoding gene segments may be amplified from nucleic acid prepared from lymphoid tissue without any prior selection of B cells. In a B cell selection approach, B cells displaying antibodies with desired antigen-binding characteristics may be selected, prior to nucleic acid extraction and amplification of VH and VL-encoding gene segments.

Various conventional methods may be used to select camelid B cells expressing antibodies with desired antigen-binding characteristics. For example, B cells can be stained for cell surface display of conventional IgG with fluorescently labelled monoclonal antibody (mAb, specifically recognizing conventional antibodies from llama or other camelids) and with target antigen labelled with another fluorescent dye. Individual double positive B cells may then be isolated by FACS, and total RNA (or genomic DNA) extracted from individual cells. Alternatively cells can be subjected to *in vitro* proliferation and culture supernatants with secreted IgG can be screened, and total RNA (or genomic DNA) extracted from positive cells. In a still further approach, individual B cells may be transformed with specific genes or fused with tumor cell lines to generate cell lines, which can be grown "at will", and total RNA (or genomic DNA) subsequently prepared from these cells.

Instead of sorting by FACS, target specific B cells expressing conventional IgG can be "panned" on immobilized monoclonal antibodies (directed against camelid conventional antibodies) and subsequently on immobilized target antigen. RNA (or genomic DNA) can be extracted from pools of antigen specific B cells or these pools can be transformed and individual cells cloned out by limited dilution or FACS.

B cell selection methods may involve positive selection, or negative selection.

Whether using a diverse library approach without any B cell selection, or a B cell selection approach, nucleic acid (cDNA or genomic DNA) prepared from the lymphoid tissue is subject to an amplification step in order to amplify gene segments encoding individual VH domains or VL domains.

Total RNA extracted from the lymphoid tissue (e.g. peripheral B cells or tissue biopsy) may be converted into random primed cDNA or oligo dT primer can be used for cDNA synthesis, alternatively Ig specific oligonucleotide primers can be applied for cDNA synthesis, or mRNA (i.e. poly A RNA) can be purified from total RNA with oligo dT cellulose prior to cDNA synthesis. Genomic DNA isolated from B cells can be used for PCR.

PCR amplification of heavy chain and light chain (kappa and lambda) gene segments encoding at least VH or VL can be performed with FR1 primers annealing to the 5' end of the variable region in combination with primers annealing to the 3' end of CH1 or Ckappa/Clambda region with the advantage that for these constant region primers only one primer is needed for each type. This approach enables camelid Fabs to be cloned. Alternatively sets of FR4 primers annealing to the 3' end of the variable regions can be used, again for cloning as Fabs (fused to vector encoded constant regions) or as scFv (single chain Fv, in which the heavy and light chain variable regions are linked via a flexible linker sequence); alternatively the variable regions can be cloned in expression vectors allowing the production of full length IgG molecules displayed on mammalian cells.

In general the amplification is performed in two steps; in the first step with non-tagged primers using a large amount of cDNA (to maintain diversity) and in the second step the amplicons are re-amplified in only a few cycles with tagged primers, which are extended primers with restriction sites introduced at the 5' for cloning. Amplicons produced in the first amplification step (non-tagged primers) may be gel-purified to remove excess primers, prior to the second amplification step. Alternatively, promoter sequences may be introduced, which allow transcription into RNA for ribosome display. Instead of restriction sites recombination sites can be introduced, like the Cre-Lox or TOPO sites, that permit the site directed insertion into appropriate vectors.

Amplified gene segments encoding camelid conventional VH and VL domains may then be cloned into vectors suitable for expression of VH/VL combinations as functional antigen binding polypeptides. By way of example, amplified VHCH1 / VKCK / VLCL gene segments from pools of B cells (or other lymphoid tissue not subject to any B cell selection) may be first cloned separately as individual libraries (primary libraries), then in a second step Fab or scFV libraries may be assembled by cutting out the light chain fragments and ligating these into vectors encoding the heavy chain fragments. The two step procedure supports the generation of large libraries, because the cloning of PCR products is relatively inefficient (due to suboptimal digestion with restriction enzymes). scFv encoding DNA fragments can be generated by splicing-by-overlap extension PCR (SOE) based on a small overlap in sequence in amplicons; by mixing VH and VL encoding amplicons with a small DNA fragment encoding the linker in a PCR a single DNA fragment is formed due to the overlapping sequences.

Amplicons comprising VH and VL-encoding gene segments can be cloned in phage or phagemid vectors, allowing selection of target specific antibody fragments by using phage display based selection methods. Alternatively amplicons can be cloned into expression vectors which permit display on yeast cells (as Fab, scFv or full length IgG) or mammalian cells (as IgG).

Cloning can be avoided by using the amplicons for ribosome display, in which a T7 (or other) promoter sequence and ribosome binding site is included in the primers for amplification. After selection for binding to target antigen, pools are cloned and individual clones are analyzed. In theory, larger immune repertoires can be sampled using this approach as opposed to a phage display library approach, because cloning of libraries and selection with phage is limited to 10¹⁰ to 10¹² clones.

When applying B cell sorting, amplicons contain VH or VL-encoding gene segments of individual target specific B cells can be cloned directly into bacterial or mammalian expression vectors for the production of antibody fragments (scFVs or Fabs) or even full length IgG.

In a particular, non-limiting, example of the "library construction" process, a method of producing a library of expression vectors encoding VH and VL domains of camelid conventional antibodies is described, said method comprising the steps:
a) actively immunising a camelid, thereby raising conventional camelid antibodies against a target antigen;
b) preparing cDNA or genomic DNA from a sample comprising lymphoid tissue (e.g. circulating B cells) from said immunised camelid (including, but not limited to, Llama or alpaca);
c) amplifying regions of said cDNA or genomic DNA to obtain amplified gene segments, each gene segment comprising a sequence of nucleotides encoding a VH domain or a sequence of nucleotides encoding a VL domain of a camelid conventional antibody; and
d) cloning the gene segments obtained in c) into expression vectors, such that each expression vector contains a gene segment encoding a VH domain and a gene segment encoding a VL domain and directs expression of an antigen binding polypeptide comprising said VH domain and said VL domain, whereby a library of expression vectors is obtained.

The foregoing methods may be used to prepare libraries of camelid-encoded VH and VL domains (in particular Llama and alpaca VH and VL domains), suitable for expression of VH/VL combinations as functional antigen-binding polypeptides, e.g. in the form of scFVs, Fabs or full-length antibodies.

Libraries of expression vectors prepared according to the foregoing process, and encoding camelid (including but not limited to Llama or alpaca) VH and VL domains, are also described.

Also described is a library of phage vectors encoding Fab or scFV molecules, wherein each Fab or scFV encoded in the library comprises a VH domain of a camelid conventional antibody and a VL domain of a camelid conventional antibody.

The library may be a "diverse" library, in which the majority of clones in the library encode VH domains of unique amino acid sequence, and/or VL domains of unique amino acid sequence, including diverse libraries of camelid VH domains and camelid VL domains. Therefore, the majority (e.g. >90%) of clones in a diverse library encode a VH/VL pairing which differs from any other VH/VL pairing encoded in the same library with respect to amino acid sequence of the VH domain and/or the VL domain.

Also described are expression vectors containing VH and VL-encoding gene segments isolated from a single selected B cell of a camelid (e.g. Llama or alpaca).

Also described is a method of selecting an expression vector encoding an antigen binding polypeptide immunoreactive with a target antigen, the method comprising steps of:
i) providing a library of expression vectors, wherein each vector in said library comprises a gene segment encoding a VH domain and a gene segment encoding a VL domain, wherein at least one of said VH domain or said VL domain is from a camelid conventional antibody, and wherein each vector in said library directs expression of an antigen binding polypeptide comprising said VH domain and VL domain;
ii) screening antigen binding polypeptides encoded by said library for immunoreactivity with said target antigen, and thereby selecting an expression vector encoding an antigen binding polypeptide immunoreactive with said target antigen.

This method encompasses screening/selection of clones immunoreactive with target antigen, from a library of clones encoding VH/VL pairings. The method may also encompass library construction, which may be carried out using the library construction method described above. Optional downstream processing/optimisation steps may be carried out on selected clones, as described below. This method of selection and screening, may also form part of the general process for production of antigen binding polypeptides of the invention, described above. Hence, any feature described as being preferred or advantageous in relation to this aspect of the invention may also be taken as preferred or advantageous in relation to the general process, and *vice versa*, unless otherwise stated.

### Screening and selection of clones immunoreactive with target antigen

Screening/selection typically involves contacting expression products encoded by clones in the library (ie. VH/VL pairings in the form of antigen binding polypeptides, e.g. Fabs, scFVs or antibodies) with a target antigen, and selecting one or more clones which encode a VH/VL pairings exhibiting the desired antigen binding characteristics.

Phage display libraries may be selected on immobilized target antigen or on soluble (often biotinylated) target antigen. The Fab format allows affinity driven selection due to its monomeric appearance and its monovalent display on phage, which is not possible for scFv (as a consequence of aggregation and multivalent display on phage) and IgG (bivalent format). Two to three rounds of selections are typically needed to get sufficient enrichment of target specific binders.

Affinity driven selections can be performed by lowering the amount of target antigen in subsequent rounds of selection, whereas extended washes with non-biotinylated target enables the identification of binders with extremely good affinities.

The selection procedure allows the user to home in on certain epitopes; whereas the classical method for elution of phage clones from the immobilized target is based on a pH shock, which denatures the antibody fragment and/or target, competition with a reference mAb against the target antigen or soluble receptor or cytokine leads to the elution of phage displaying antibody fragments binding to the relevant epitope of the target (this is of course applicable to other display systems as well, including the B cells selection method).

Individual clones taken from the selection outputs may be used for small scale production of antigen-binding polypeptides (e.g. antibody fragments) using periplasmic fractions prepared from the cells or the culture supernatants, into which the fragments "leaked" from the cells. Expression may be driven by an inducible promoter (e.g. the lac promoter), meaning that upon addition of the inducer (IPTG) production of the fragment is initiated. A leader sequence ensures the transport of the fragment into the periplasm, where it is properly folded and the intramolecular disulphide bridges are formed.

The resulting crude protein fractions may be used in target binding assays, such as ELISA. For binding studies, phage prepared from individual clones can be used to circumvent the low expression yields of Fabs, which in general give very low binding signals. These protein fractions can also be screened using *in vitro* receptor - ligand binding assays to identify antagonistic antibodies; ELISA based receptor - ligand binding assays can be used, also high throughput assays like Alphascreen are possible. Screening may be performed in radiolabelled ligand binding assays, in which membrane fractions of receptor overexpressing cell lines are immobilized; the latter assay is extremely sensitive, since only picomolar amounts of radioactive cytokine are needed, meaning that minute amounts of antagonistic Fabs present in the crude protein fraction will give a positive read-out. Alternatively, FACS can be applied to screen for antibodies, which inhibit binding of a fluorescently labelled cytokine to its receptor as expressed on cells, while FMAT is the high throughput variant of this.

Fabs present in periplasmic fractions or partially purified by IMAC on its hexahistidine tag or by protein G (known to bind to the CH1 domain of Fabs) can be directly used in bioassays using cells, which are not sensitive to bacterial impurities; alternatively, Fabs from individual *E. coli cells* can be recloned in mammalian systems for the expression of Fabs or IgG and subsequently screened in bioassays.

Following identification of positive expression vector clones, i.e. clones encoding a functional VH/VL combination which binds to the desired target antigen, it is a matter of routine to determine the nucleotide sequences of the variable regions, and hence deduce the amino acid sequences of the encoded VH and VL domains.

If desired, the Fab (or scFV) encoding region may be recloned into an alternative expression platform, e.g. a bacterial expression vector (identical to the phagemid vector, but without the gene 3 necessary for display on phage), which allows larger amounts of the encoded fragment to be produced and purified.

The affinity of target binding may be determined for the purified Fab (or scFV) by surface plasmon resonance (e.g. Biacore) or via other methods, and the neutralizing potency tested using *in vitro* receptor - ligand binding assays and cell based assays.

Families of antigen-binding, and especially antagonistic Fabs (or scFVs) may be identified on the basis of sequence analysis (mainly of VH, in particular the length and amino acid sequence of CDR3 of the VH domain).

### Potency optimisation

Clones identified by screening/selection as encoding a VH/VL combination with affinity for the desired target antigen may, if desired, be subject to downstream steps in which the affinity and/or neutralising potency is optimised.

Potency optimization of the best performing member of each VH family can be achieved via light chain shuffling, heavy chain shuffling or a combination thereof, thereby selecting the affinity variants naturally occurring in the animal. This is particularly advantageous where the original camelid VH/VL domains were selected from an actively immunised camelid, since it is possible to perform chain shuffling using the original library prepared from the same immunised animal, thereby screening affinity variants arising in the same immunised animal.

For light chain shuffling the gene segment encoding the VH region (or VHCH1) of VH/VL pairing with desirable antigen binding characteristics (e.g. an antagonistic Fab) may be used to construct a library in which this single VH-encoding gene segment is combined with the light chain repertoire of the library from which the clone was originally selected. For example, if the VH-encoding segment was selected from a library (e.g. Fab library) prepared from a camelid animal actively immunised to elicit an immune response against a target antigen, then the "chain shuffling" library may be constructed by combining this VH-encoding segment with the light chain (VL) repertoire of the same immunised camelid. The resulting library may then be subject to selection of the target antigen, but under stringent conditions (low concentrations of target, extensive washing with non-biotinylated target in solution) to ensure the isolation of the best affinity variant. Off-rate screening of periplasmic fractions may also assist in the identification of improved clones. After sequence analysis and recloning into a bacterial production vector, purified selected Fabs may be tested for affinity (e.g. by surface plasmon resonance) and potency (e.g. by bioassay).

Heavy chain shuffling can be performed by cloning back the gene segment encoding the light chain (VL) of a clone selected after light-chain shuffling into the original heavy chain library from the same animal (from which the original VH/VL-encoding clone was selected). Alternatively a CDR3 specific oligonucleotide primer can be used for the amplification of the family of VH regions, which can be cloned as a repertoire in combination with the light chain of the antagonistic Fab. Affinity driven selections and off-rate screening then allow the identification of the best performing VH within the family.

It will be appreciated that the light chain shuffling and heavy chain shuffling steps may, in practice, be performed in either order, i.e. light chain shuffling may be performed first and followed by heavy chain shuffling, or heavy chain shuffling may be performed first and followed by light chain shuffling. Both possibilities are contemplated.

From light chains or heavy chains of VH/VL pairings (e.g. Fabs) with improved affinity and potency the sequences of, in particular, the CDRs can be used to generate engineered variants in which mutations of the individual Fabs are combined. It is known that often mutations can be additive, meaning that combining these mutations may lead to an even more increased affinity.

### Germlining and formatting for human therapeutic use

The VH and VL-encoding gene segments of selected expression clones encoding VH/VL pairings exhibiting desirable antigen-binding characteristics (e.g. phage clones encoding scFVs or Fabs) may be subjected to downstream processing steps and recloned into alternative expression platforms, such as vectors encoding antigen binding polypeptide formats suitable for human therapeutic use (e.g. full length antibodies with fully human constant domains).

Promising "lead" selected clones may be engineered to introduce one or more changes in the nucleotide sequence encoding the VH domain and/or the VL domain, which changes may or may not alter the encoded amino acid sequence of the VH domain and/or the VL domain. Such changes in sequence of the VH or VL domain may be engineered for any of the purposes described elsewhere herein, including germlining or humanisation, codon optimisation, enhanced stability, optimal affinity etc.

The general principles germlining or humanisation described herein apply equally in this process. By way of example, lead selected clones containing camelid-encoded VH and VL domains may be germlined/humanised in their framework regions (FRs) by applying a library approach. After alignment against the closest human germline (for VH and VL) and other human germlines with the identical canonical folds of CDR1 and CDR2, the residues to be changed in the FRs are identified and the preferred human residue selected, as described elsewhere herein in detail. Whilst germlining may involve replacement of camelid-encoded residues with an equivalent residue from the closest matching human germline this is not essential, and residues from other human germlines could also be used.

The germlining of a VH domain having an amino acid sequence homologous to a member of the human VH3 family will often involve replacement/substitution of a number of residues, which already deviate in publically known *Lama glama, Lama pacos* or *Camelus dromedarius* derived germline sequences. Permitted amino acid substitutions for germlining/humanisation of a VH3 domain of *Lama glama, Lama pacos* or *Camelus dromedarius*, and in particular *Lama glama* include, but are not limited to, amino acid replacements at any one or any combination of positions 71, 83 and 84 in the framework region (using Kabat numbering). Such replacement(s) will involve substitution of the camelid-encoded residue(s) at these positions with a different amino acid, which may be a natural or non-natural amino acid, and is preferably an amino acid known to occur at the equivalent position in a human-encoded VH3 domain. For example, Alanine at position 71 might be replaced with serine or alanine, Lysine at position 83 might be replaced with Arginine and Proline at position 84 might be replaced with Alanine. Accordingly, particular non-limiting embodiments of the antigen binding polypeptide of the invention include variants comprising a camelid (and more specifically llama, alpaca or dromedary) VH domain exhibiting sequence homology to a human VH3 domain, which VH domain includes amino acid substitutions (versus the camelid-encoded sequence) at one or more or all of positions 71, 83 and 84 (using Kabat numbering). In particular, variants with one or more or any combination of the following substitutions are permitted: A changed to S at position 71, K changed to R at position 83 or P changed to A at position 84.

Once the amino acid sequences of the lead VH and VL domains (following potency optimisation, as appropriate) are known, synthetic genes of VH and VL can be designed, in which residues deviating from the human germline are replaced with the preferred human residue (from the closest matching human germline, or with residues occurring in other human germlines, or even the camelid wild type residue). At this stage the gene segments encoding the variable domains may be re-cloned into expression vectors in which they are fused to human constant regions of the Fab, either during gene synthesis or by cloning in an appropriate display vector.

The resulting VH and VL synthetic genes can be recombined into a Fab library or the germlined VH can be recombined with the wild type VL (and vice versa, referred to as "hybrid" libraries). Affinity-driven selections will allow the isolation of the best performing germlined version, in case of the "hybrid" libraries, the best performing germlined VH can be recombined with the best performing germlined VL.

Amino acid and nucleotide sequence information for the germlined Fabs can be used to generate codon-optimized synthetic genes for the production of full length human IgG of the preferred isotype (IgG1 for ADCC and CDC, IgG2 for limited effector functions, IgG4 as for IgG2, but when monovalent binding is required). For non-chronic applications and acute indications bacterially or mammalian cell produced human Fab can produced as well.

Combining steps of the above-described processes, in a particular non-limiting example is described a method of producing an expression vector encoding a chimeric antigen binding polypeptide immunoreactive with a target antigen, said method comprising the steps of:
a) actively immunising a camelid (including but not limited to Llama or alpaca), thereby raising conventional camelid antibodies against a target antigen;
b) preparing cDNA or genomic DNA from a sample comprising lymphoid tissue (e.g. circulating B cells) from said immunised camelid;
c) amplifying regions of said cDNA or genomic DNA to obtain amplified gene segments, each gene segment comprising a sequence of nucleotides encoding a VH domain or a sequence of nucleotides encoding a VL domain of a camelid conventional antibody;
d) cloning the gene segments obtained in c) into expression vectors, such that each expression vector contains a gene segment encoding a VH domain and a gene segment encoding a VL domain and directs expression of an antigen binding polypeptide comprising said VH domain and said VL domain, thereby producing a library of expression vectors;
e) screening antigen binding polypeptides encoded by the library obtained in step d) for immunoreactivity with said target antigen, and thereby selecting an expression vector encoding an antigen binding polypeptide immunoreactive with said target antigen;
f) optionally performing a light chain shuffling step and/or a heavy chain shuffling step to select an expression vector encoding a potency-optimised antigen binding polypeptide immunoreactive with said target antigen;
g) optionally subjecting the gene segment encoding the VH domain of the vector selected in step e) or step f) and/or the gene segment encoding the VL domain of the vector selected in step e) or step f) to germlining and/or codon optimisation; and
h) cloning the gene segment encoding the VH domain of the vector selected in part e) or f) or the germlined and/or codon optimised VH gene segment produced in step g) and the gene segment encoding the VL domain of the vector selected in part e) or f) or the germlined and/or codon optimised VL gene segment produced in step g) into a further expression vector, in operable linkage with a sequence of nucleotides encoding one or more constant domains of a human antibody, thereby producing an expression vector encoding a chimeric antigen binding polypeptide comprising the VH and VL domains fused to one or more constant domains of a human antibody.

The invention also extends to expression vectors prepared according to the above-described processes, and to a method of producing an antigen binding polypeptide immunoreactive with a target antigen, the method comprising steps of:
a) preparing expression vector encoding an antigen binding polypeptide immunoreactive with a target antigen using the method described above;
b) introducing said expression vector into host cell or cell-free expression system under conditions which permit expression of the encoded antigen binding polypeptide; and
c) recovering the expressed antigen binding polypeptide.

In one embodiment, the latter process encompasses bulk production-scale manufacture of the antigen-binding polypeptide of the invention, particularly bulk-scale manufacture of therapeutic antibodies intended for use as pharmaceutically active agents, by recombinant expression. In such embodiments, the expression vector prepared in step a) and the host cell/expression system used in step b) are selected to be suitable for large-scale production of recombinant antibodies intended for administration to human patients. The general characteristics of suitable vectors and expression systems for this purpose are well known in the art.

The invention will be further understood with reference to the following non-limiting experimental examples.

Examples 1 to 9 illustrate the process of raising an antibody against an example antigen denoted "cytokine x", starting from immunization of Ilamas. The same general protocol can be adapted for any target antigen in any camelid species, hence the precise identity of "cytokine x" is not material. The process is also illustrated for preparation of Fabs binding IL-1 Beta (Example 15 onwards).

### General protocol

### Example 1

### Immunization of llamas.

Immunizations of llamas *(Lama glama*) and harvesting of peripheral blood lymphocytes as well as the subsequent extraction of RNA and amplification of antibody gene fragments were performed as described by De Haard and colleagues (De Haard et al., J. Bact. 187: 4531-4541 (2005)). One llama was immunized intramuscularly with recombinant human Cytokine x using Freund's complete adjuvant or an appropriate animal-friendly adjuvant Stimune (Cedi Diagnostics BV, The Netherlands). Cytokine x (recombinantly expressed in engineered human cell line) was purchased . Prior to immunization the lyophilized cytokine x was reconstituted in PBS (Dulbecco) at a concentration of 250 µg/ml. The llama received 6 injections at weekly intervals, the first two injections with 100 µg of cytokine per injection, the four last injections with 50 µg for each boost. Four days after the last immunization a blood sample (PBL1) of 150 ml was collected from the animal and serum was prepared. Ten days after the last immunization a second blood sample (PBL2) of 150 ml was taken and serum was prepared. Peripheral blood lymphocytes (PBLs), as the genetic source of the llama immunoglobulins, were isolated from the blood sample using a Ficoll-Paque gradient (Amersham Biosciences) yielding between 1 and 5×10⁸ PBLs. The maximal diversity of antibodies is expected to be equal to the number of sampled B-lymphocytes, which is about 10% (between 9.2 - 23.2% (De Genst et al., Dev. Comp. Immunol. 30:187-98 (2006)) of the number of PBLs (1 - 5×10⁷). The fraction of conventional antibodies in Ilama serum is up to 80% of the amount of total immunoglobulin, which might be extrapolated to a similar fraction of B-lymphocytes that produce the conventional antibodies. Therefore, the maximal diversity of conventional antibodies in the 150 ml blood sample is calculated as 0.8 - 4x10⁷ different molecules. Total RNA was isolated from PBLs according to the method of Chomczynski et al. Anal. Biochem.162:156-159 (1987)).

### Example 2

### Enrichment of antigen reactive B cells by panning or FACS sorting (optional).

In order to reduce the complexity of the sampled B cell repertoire enabling the efficient cloning of the recombinatorial Fab phage display library, antigen reactive B cells were enriched by a FACS sorting (Weitkamp et al., J. Immunol. Meth. (2003) 275: 223-237) using fluorescently labelled antigen and a mAb recognizing camelid conventional antibody specifically (as B cell marker) or by a panning procedure on immobilized antigen (Lightwood et al., J. Immunol. Meth. 316:133-143 (2006)).

PBLs from immunized animals were isolated via a density centrifugation on Ficoll-Paque as described above. Optionally co-purified red blood cells were lysed by resuspending the PBL pellet in 20 ml of lysis buffer (8.29 g/L NH4Cl, 1.09 g/L KHCO3 and 37 mg/L EDTA) at room temperature followed by centrifugation for 10 minutes at 200xg. Optional was also the depletion of monocytes by adhering these to the plastic surface of TI50 culture flask. To achieve this cells were resuspended in 70 ml RPMI (Invitrogen) supplemented with 10% foetal calf serum, Glutamax, 25 mM Hepes, penicillin-streptomycin (Invitrogen) and 0.38% sodium citrate and incubated for 2 hours at 37 °C and 5% CO2 in the flasks. The supernatant fraction containing the B selected was recovered and cells were counted.

Bulk sorting in FACS of (living) B cells displaying target specific conventional antibodies was performed by simultaneous staining with the fluorescently labeled mAb specifically recognizing camelid conventional antibodies and target antigen, labeled with yet another fluorescent dye. Between 1,000 and 100,000 antigen specific cells were sorted and used for RNA extraction by applying the protocol of Gough and colleagues (Gough, Anal. Biochem. 173:93-95 (1988)) or by using the TRIzol kit (Invitrogen). Total RNA was converted into random primed cDNA as template for the amplification of the antibody heavy and light chain variable genes (see Example 3 and further).

### Example 3

### Amplification and cloning of Variable Region Genes.

Random primed cDNA was prepared from 80 µg of PBL RNA using the superscript III First-Strand Synthesis System for RT-PCR (Invitrogen). RNA was heat-denatured for 5 min at 65 °C in the presence of 2.5 µM of random hexanucleotide primer and 500µM dNTPs in 8 independent reaction of 20ul reaction. Subsequently, buffer and dithiothreitol were added according to the supplier's instructions, as well as 640 units of RNasOUT (40 units/µl, Invitrogen), and 3200 units of Superscriptlll reverse transcriptase (200 units/µl; Invitrogen) in a total final volume of 8x40µl. After 50min at 50°C, 5min at 85°C and 1 min at 1°C RNAse H was added (∼4U) and incubated for 20min at 37°C. The pooled cDNA was cleanup using QIAquick PCR Purification Kit according to supplier's recommandation and used for PCR.

Primers annealing to the 3' end of CH1 and 5' and 3' end of VH were designed on the basis of germline sequences from llama and dromedary, for which the deposited sequences could be retrieved from IMGT and other databases following the citations from De Genst and colleagues (De Genst et al, Dev. Comp. Immunol. 30:187-198 (2006)). For design of oligonucleotides for amplification of the light chain the rearranged and somatically mutated dromedary sequences were used that were published in a thesis study (I. Legssyer, Free University Brussels).

All primary PCRs were carried out with separate BACK primers annealing to the 5' end of the variable region and combined with FOR primers annealing to the 3' end of CH1, on relatively large amounts of random primed cDNA as template (up to 2.5 µl corresponding to 6 µg of total RNA) to maintain maximal diversity. The heavy chain derived amplicons can be reamplified with a combination of JHFOR primers, annealing to the 3' end of VH and containing a naturally occurring BstEII site, and Sfil-tagged VHBACK primers annealing to the 5' end of the VH gene, and subsequently cloned as VH fragments. The light chain V-genes were obtained by PCR with a set of CKFOR or CLFOR primer annealing to the 3' end of the constant domain and BACK primers priming at the 5' end of the V-regions. The amplicons from the first PCR reactions were reamplified with extended CH1 FOR (containing a Notl site) or CKFOR and CLFOR primers (containing an Ascl site) and subsequently cloned as Ilama Fab fragments. Alternatively, the DNA segments can be reamplified with primers tagged with restriction sites (FOR primers with Ascl site and FR4 based BACK primers with Xhol site) and cloned as VL fragments thus creating chimeric Fab's containing Ilama derived V regions combined with human C regions.

PCR was performed in a volume of 50 µl reactions using Phusion polymerase (Finnzymes) and 500 pM of each primer for 28 cycles (1 min at 96 °C, 1 min at 60 °C, and1 min at 72 °C. All products were purified from agarose gel with the QIAex-II extraction kit (Qiagen). As input for reamplification to introduce restriction sites, 100-200 ng of purified DNA fragment was used as template in a 100-µl reaction volume. The large amount of input, ensuring the maintenance of variability, was checked by analysis of 4 µl of the "unamplified" PCR mixture on agarose gel.

### Example 4

### Construction of the Primary and Secondary camelid Fab Repertoires.

For the construction of the primary heavy chain and the two primary light chain repertoires, the PCR products, appended with restriction sites, were gel-purified prior to digestion and the different VH, VK, and VL families combined into three groups. The VHCH1 fragments were digested with Sfil and Notl and the VKCK and VLCL fragments were digested with ApaLI and Ascl, and cloned into the phagemid vector pCB3 (similar to vector pCES1 with adapted multiple cloning site). The digested fragment (1 to 2 µg) were ligated to digested and purified PCB3 (2 to 4 µg) using T4-DNA ligase (Fermentas) at room temperature for several hours and then at 37°C for 1-2 hours. The desalted ligation mixture for light or heavy chain pools was used for electroporation of the E. coli strain TG1, to create the one-chain libraries.

Altenatively, the VH fragments, 1.5 µg in total, may be digested with Sfil and BstEII (present in the VH) and ligated in a 100-200-µl reaction mixture with 9 units of T4-DNA ligase at room temperature to 4 µg, gel-purified vector PCB4 (similar to vector PCB3, but with the pIII gene deleted). In addition, the VH gene segments may be cloned via Sfil and BstEII and the VK / VL gene segments via ApaLI and Xhol, yielding the chimeric Fd and VKCK and VLCL.

The Fab library was obtained by cloning of light chain fragments, digested from plasmid DNA prepared from the light chain repertoires, into the plasmid collection containing the heavy chain repertoires. Plasmid DNA, isolated from at least 3×10⁹ bacteria of the VL library (the donor vector), was digested with ApaLI and Ascl for cloning of the gel purified DNA fragments in the acceptor vector that already contained the heavy chain libraries, thus creating a separate Fab library with kappa light chains and another library consisting of Fabs with a lambda light chain with a size of 1 - 10x10⁹ clones. Similarly, the VLCL or VKCK from the single chain library can be extracted from agarose gel using ApaLI/AscI and cloned into the VHCH library vector using the same restriction site.

### Example 5

### Selection of the Library.

The rescue of phagemid particles with helper phage M13-KO7 or VCSM-13 was performed on 2-liter scale, using representative numbers of bacteria from the library for inoculation, to ensure the presence of at least 10 bacteria from each clone in the start inoculum. For selections, 10¹³ colony-forming units were used with antigens immobilized in immunotubes (Maxisorp tubes, Nunc) or in 96 wells microtiterplates (Maxisorp, Nunc) or with soluble biotinylated antigens. The amount of the immobilized antigens was reduced 10- 100 fold during subsequent selection rounds, starting at 10 µg/ml at round 1. Antigens were biotinylated at a ratio of 3 to 10 molecules of NHS-Biotin (Pierce) per molecule of antigen according to the supplier's recommendations and tested for their bioactivity in a bioassay. Unless stated otherwise, the antigens were used for selection at concentrations of 1 to 10 nM during round1 and 10 pM to 1 nM during subsequent rounds.

### Example 6

### Screening for antagonistic cytokine x specific Fab's.

Soluble Fab was produced from individual clones as described in Marks *et al.* (Marks et al., J. Mol. Biol. 222:581-597 (1991)), but preferably as monoclonal phage (Lee et al., Blood 108:3103-3111 (2006)) to boost the sensitivity. Culture supernatants containing soluble Fab or Fab displaying phage were tested in ELISA with directly coated antigen or captured via immobilized streptavidin. Recombinant human cytokine x and streptavidin were coated at 10 µg/ml in 0.1 M NaHCO₃ pH 9.6, for 16 h at 4 °C. Following 3 washes with PBS, 0.1% (v/v) Tween 20, biotinylated antigen was added for 30 to 60 minutes at room temperature at a concentration of 0.5 µg/ml. The plates were blocked during 30 min at room temperature with 2% (w/v) semi-skim milk powder (Marvel) in PBS or with 1% casein solution (in PBS). The culture supernatant was diluted 1- or 5-fold in 2% (w/v) Marvel/PBS and incubated 2 h; bound Fab was detected with anti-myc antibody 9E10 (5 µg/ml) recognizing the myc-peptide tag at the carboxyl terminus of the heavy Fd chain, and rabbit anti-mouse-HRP conjugate (Dako). Following the last incubation, staining was performed with tetramethylbenzidine and H₂O₂ as substrate and stopped by adding 0.5 volume of 1 M H₂SO₄; the optical density was measured at 450 nm. Clones giving a positive signal in ELISA (over 2 times background), were analyzed by BstNI or Hinfl fingerprinting of the PCR products obtained by amplification with the oligonucleotide primers M13-reverse and genelll-forward (4) or of the separate Fd and VKCK or VLCL amplicons.

Screening for the Fab's capacity to interfere with binding of cytokine x to its receptor was performed in an appropriate receptor - ligand binding ELISA. For this, low amounts of biotinylated cytokine x were incubated with Fab in culture supernatant on cytokine x - Receptor coated plates and bound cytokine x was subsequently detected with streptavidin-HRP conjugate. Positive hits were sequenced and Fab purified for determining their potency (IC50) in the in vitro receptor - ligand assay and to assess their affinity in BIAcore on immobilized cytokine x.

Large scale induction of soluble Fab fragments from individual clones was performed on a 50-ml or 250-ml scale in 2x TY containing 100 µg/ml carbenicillin and 2% glucose. After growth at 37 °C to an OD600 of 0.9, the cells were pelleted (10 min at 2,934 x g) and resuspended in 2x TY with carbenicillin and 1 mM isopropyl-1-thio-D-galactopyranoside (IPTG). Alternative the De Bellis procedure (De Bellis and Schwartz, NAR (1990) 18(5): 1311) was followed using 0.2% in stead of 2% glucose, thus permitting the direct addition of IPTG to the medium of the late log phase cells. Bacteria were harvested after 3.5 h of growth at 30 °C by centrifugation (as before); periplasmic fractions were prepared by resuspending the cell pellet in 1 ml of ice-cold PBS. After 2-16 h of rotating head-over-head at 4 °C, the spheroplasts were removed by two centrifugation steps; after spinning during 10 min at 3,400 x g, the supernatant was clarified by an additional centrifugation step during 10 min at 13,000 x g in an Eppendorf centrifuge. The periplasmic fraction obtained was directly used in the different functional assays (target binding ELISA, in vitro receptor - ligand binding assays and Biacore).

For sequencing, plasmid DNA was prepared from 5-ml cultures grown at 30 °C in LB-medium, containing 100 µg/ml carbenicillin and 2% glucose, using the Qiagen Mini-kit (Qiagen) or on amplicons with vector primers M13-reverse and geneIII-forward, which anneal at the borders of the Fab insert.

### Example 7

### Large scale production and purification of lead Fab's.

Fab inserts from 3 to 6 different leads were recloned via ApaLI - Notl in an expression vector (coded pCB5) identical to pCB3 including the hexahistidine and C-MYC tags fused to the carboxyterminus of the Fd, but lacking the bacteriophage M13 gene3. In parallel the V regions were recloned with the appropriate combination of restriction enzymes and sequentially cloned in gene3 deleted vectors containing human CH1 and CK or CL for the expression of chimeric Fabs. After fingerprint analysis, individual clones obtained after recloning were grown on 50-ml or 250-ml scale and periplasmic fractions were prepared as described above. Fab fragment was IMAC purified and the correctly formed Fab was further purified via Size Exclusion Chromatography using a Superdex 75HR column (Amersham Pharmacia Biotech). Depending on the cell based assay endotoxin was removed by passage over an anion exchange column (Source30Q, GE Healthcare), which was sensitized overnight in 1 M NaOH and subsequently equilibrated in D-PBS. The yield was determined by measuring the optical density at 280 nm using a molar extinction coefficient of 13 for Fabs.

The purified Fab's were tested in the *in vitro* receptor - ligand binding assay and Biacore to confirm the observations made with the Fab fragment as produced by the gene3 containing pCB3 vector. Finally the potency of the Fab was determined in the bioassay.

### Example 8

In this example the Ilama derived lead Fabs against cytokine x were humanized by using a soft randomization procedure targeting a small set of framework residues and by monovalent display of the resulting Fab library on the surface of filamentous phage in order to identify high affinity framework sequences via affinity based selection (US2003/0190317 A1). For instance, for dromedary derived germline VH (IGHV1S20) a small library was generated for positions 5 (Val, Leu), 55 (Gly, Ala), 83 (Ala, Ser), 95 (Lys, Arg), 96 (Ser, Ala) and 101 (Met, Val) maintaining 70% of the wild type residues (IMGT numbering). Amino acids in the hypervariable loops could be addressed in a similar way.

Site-directed mutagenesis to correct PCR errors or to introduce additional specific single codon changes was performed essentially as described by Kunkel et al. Curr. Protoc. Mol. Biol. CH8U8.1 (2001) or alternatively Synthetic genes encoding the variants ordered from GeneArt. The template for site-directed experiments was single-stranded DNA from a humanized Fab clone.

Site directed mutagenesis was also used to directly change a limited number of amino acid codons for humanization or modification purposes in the DNA encoding the wild type or humanized Fabs.

After selection the individual leads were tested on affinity and potency and the best leads were chosen for reformatting into human Fab/IgGs.

### Example 9

### Expression of human Fab fragments and human monoclonal antibodies.

Starting from the humanized VH and VL regions expression of humanized Fabs was performed as described in Rauchenberger et al. J. Biol. Chem. 278:38194-204 (2003). For the expression of human monoclonal antibodies two separate expressions vectors, one for the light chain and one for the heavy chain construct were constructed based on the pcDNA 3.1 vector. The expression vector for the light chain contained either the human C kappa or human C lambda sequence downstream of a CMV promoter as well as restriction sites allowing the cloning of the light chain construct as KPN1 BsmB1 fragments downstream of the CMV promoter and in frame with the light chain constant domain. The expression vector for the heavy chain contained then human CH1-hinge-CH2-CH3 sequence downstream of a CMV promoter as well as restriction sites allowing the cloning of the VH construct as KPN1 BsmB1 fragments downstream of the CMV promoter and in frame with the heavy chain constant domains.

The VL and VH fragments were cloned into the appropriate expression vectors as KPN1 BsmB1 fragments containing the Kozak sequence followed by the mouse IgG kappa leader sequences in frame with the respective VL or VH sequence. These sequences were obtained by gene synthesis and optimized for expression in mammalian cells (Geneart).

For full length IgG production VH and VL expression vectors constructs were co-transfected into mammalian cells (HEK-293 (transient) or CHO(stable)). Supernatant from transiently transfected cells or from stably transfected cells was purified via protein A chromatography.

Monoclonal antibodies or Fabs were tested in receptor binding assays and in bioassays and the best leads were selected for further development.

### Example 10 - camelid vs human homology analysis

### Methodology

Sequences of germline Ilama and dromedary J regions, which encode for FR4 were compared with human sequences and were completely identical (10 out of 10 residues match); the only exception is IGHJ4, which contains Glutamine (instead of Leucine or Methionine) on position 6 in FR4 (alignments shown below). For somatically mutated VLambda FR4 from dromedary 9 out of 10 residues match (90% homology), since most of the available sequences have Histidine in stead of Lysine or Glutamate or Glutamine on position 6 (alignment shown below). Finally there is again 90% identity (9 out of 10 residues) in FR4 of the set of six available somatically mutated dromedary VKappa sequences, because position 3 residue Serine deviates from what is found in the human germline JK segments, i.e. Glutamine, Proline and Glycine (alignment shown below).

For VH, VLambda and VKappa the analysis is supported by alignments (see below). Sequence alignment was done with closest human germline sequence, having identical H1 and H2 canonical folds.

Residues which don't align, but which appear in another family member (or subclass) of the same germline are considered as homologous, based on the assumption that it is feasible to mutate them back to said closest human germline sequence.

Canonical structures are compared using the following programs:
http://www.bioinf.org.uk/abs/chothia/html
and http://www.bioc.unizh.ch/antibody/Sequences/Germlines/VBase hVK.html. Residues of analyzed camelid antibodies (or from other species) which do not fit exactly the canonical fold algorithm are checked for their appearance in the sequence of members of the matching human germline family with the same combination of canonical folds, or mentioned residues allowing the folds are checked for their appearance within the family of camelid antibodies to which the analyzed antibody belongs.

Results are presented in the following sections:
10.1 - Dromedary VH
10.2 - Lama glama VH
10.3 - VL 1-40
10.4 - VL 2-18
10.5 - VL 3-1
10.6 - VL 3-12
10.7 - VKappa 2-40
10.8 - J(H) region comparisons of Ilama and human
10.9 - Comparison of light chain J regions
10.10 - Lama pacos VH germline homology
10.11 - Lama glama derived VH homology analysis
10.12 -Lama glama derived VL analysis

### 10.1 Dromedary VH

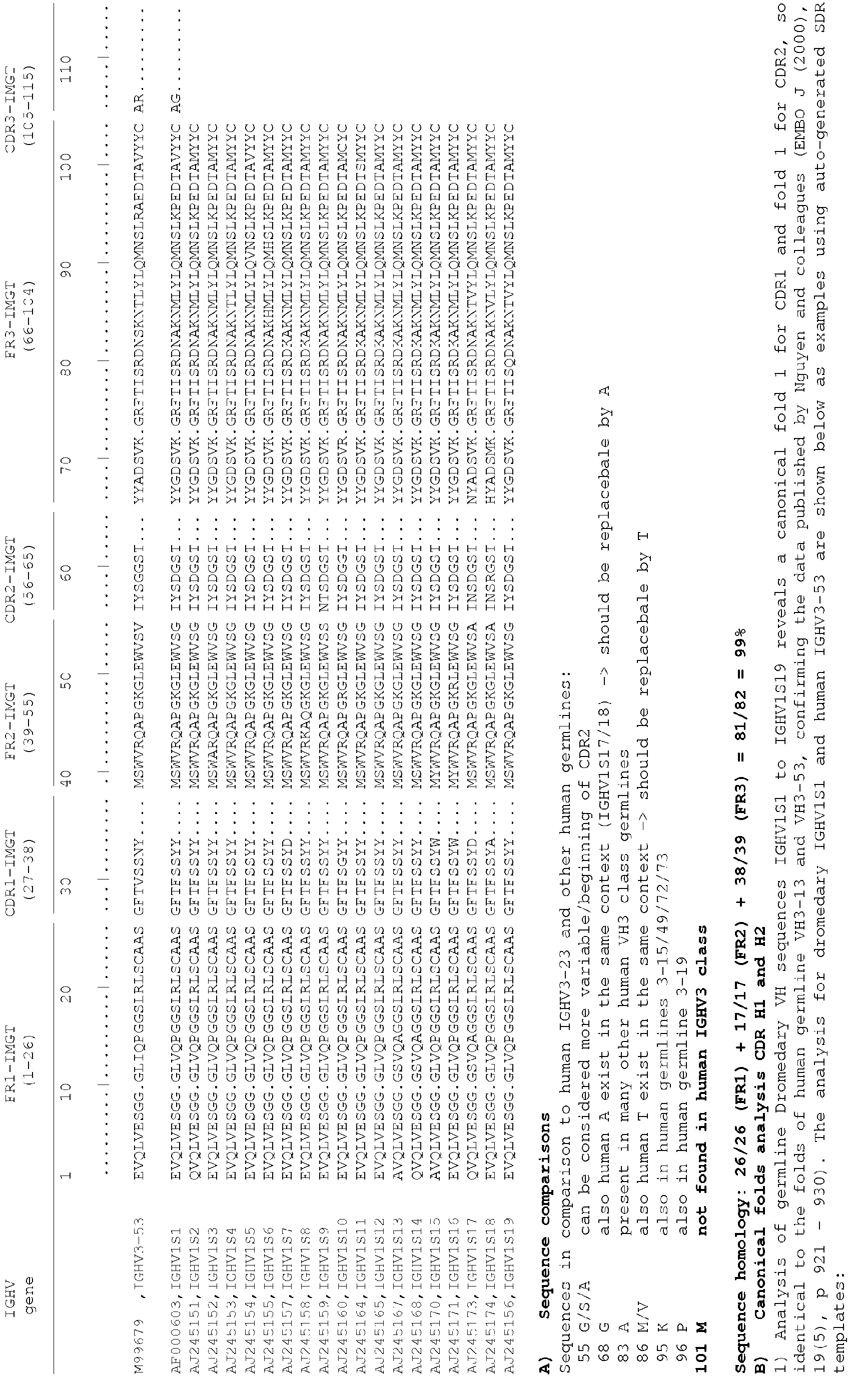

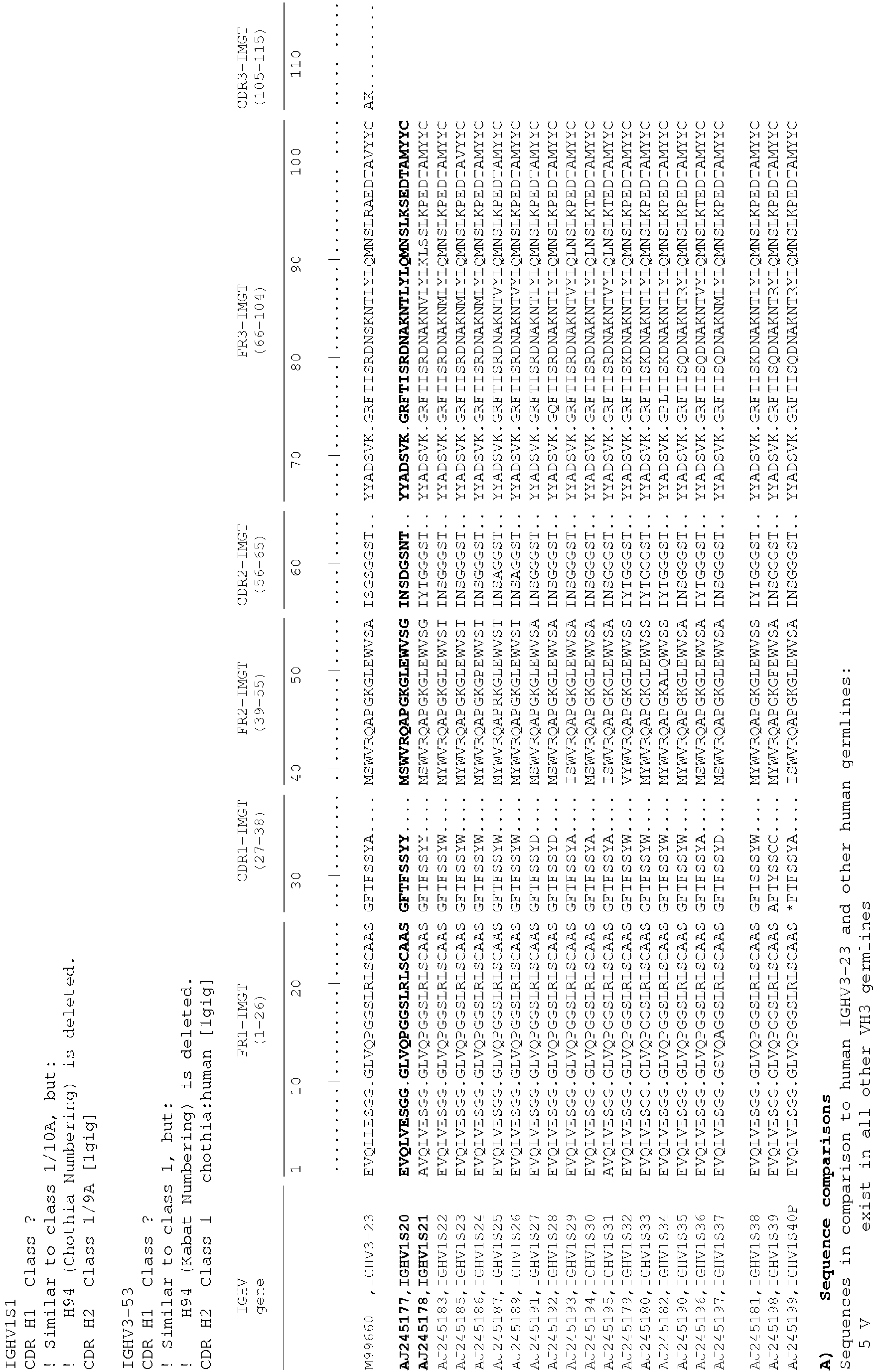

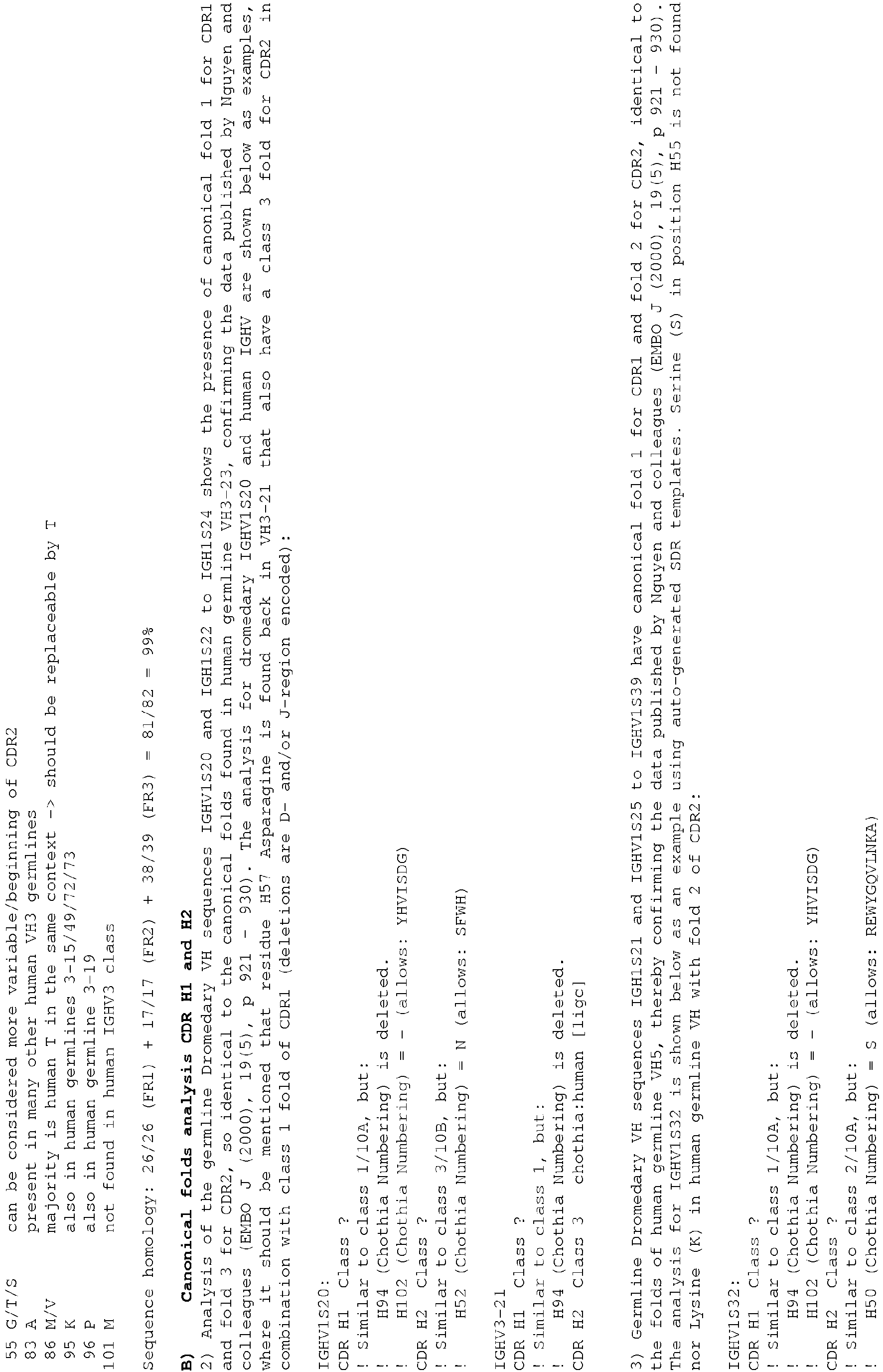

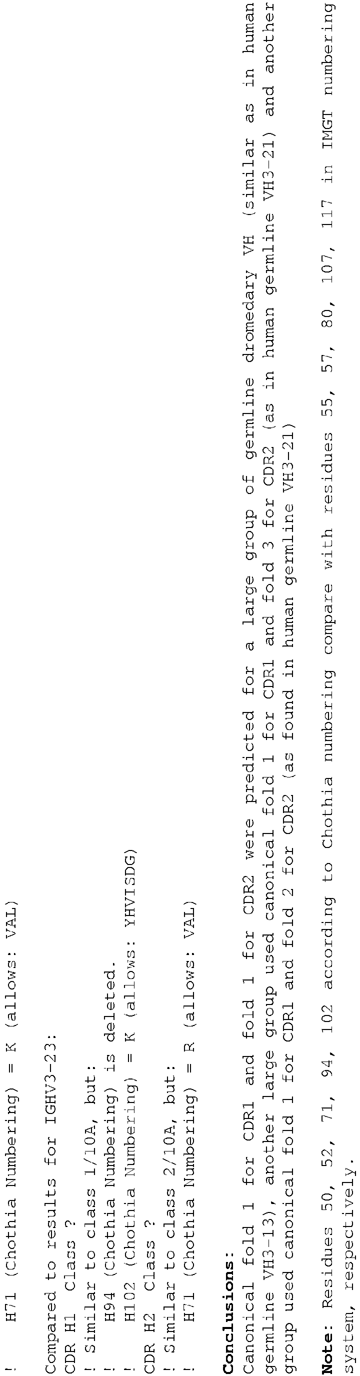

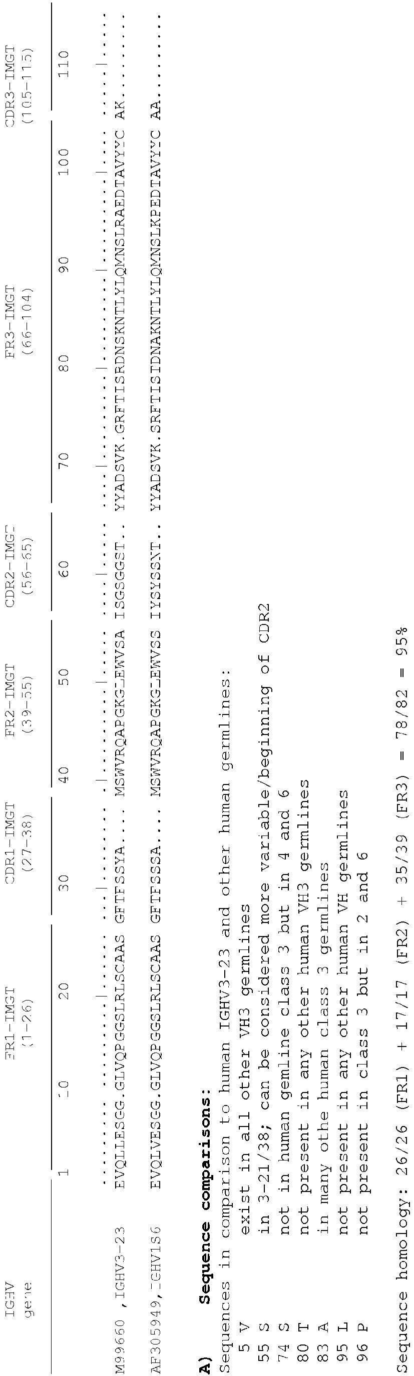

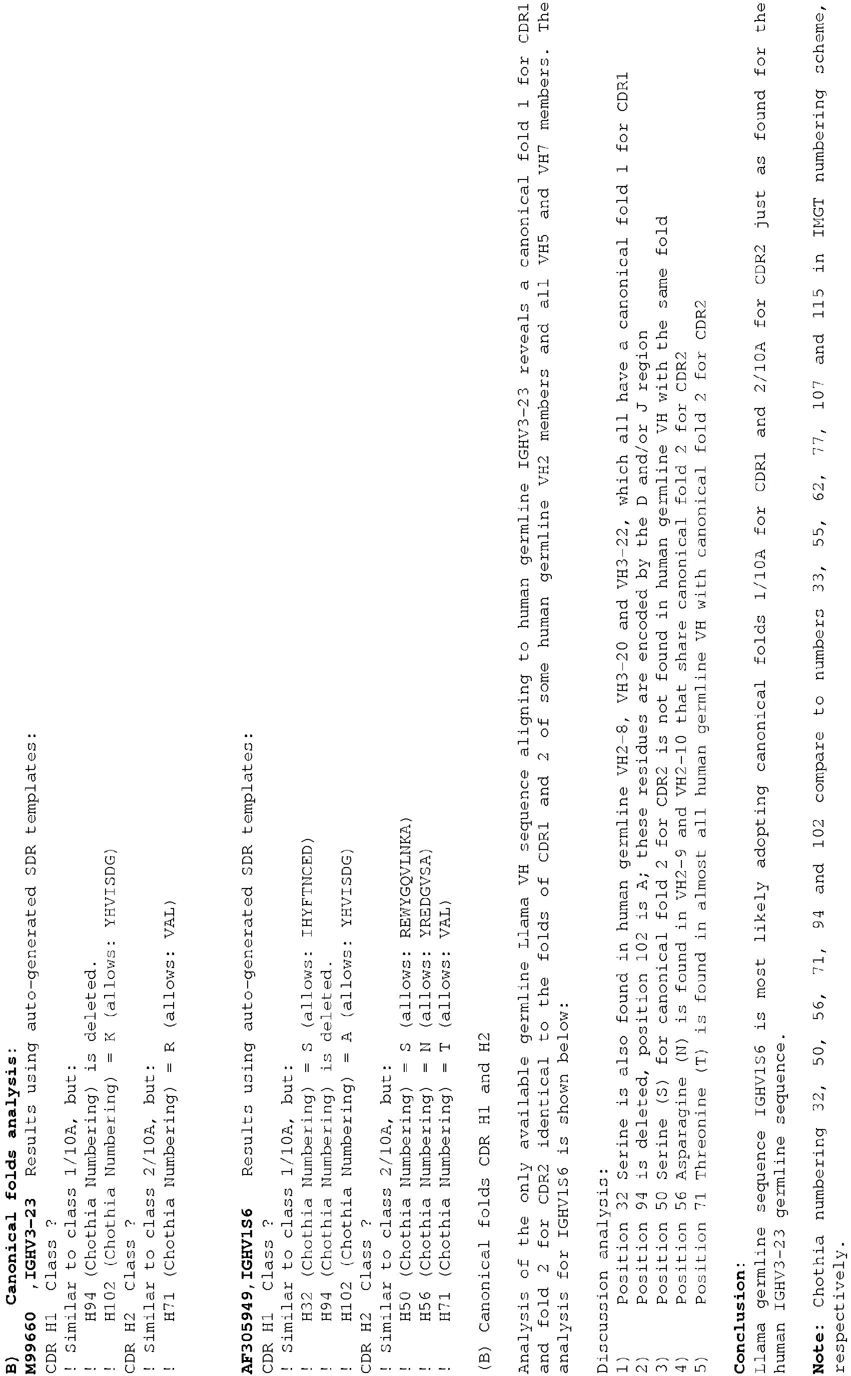

### 10.3 VL 1-40

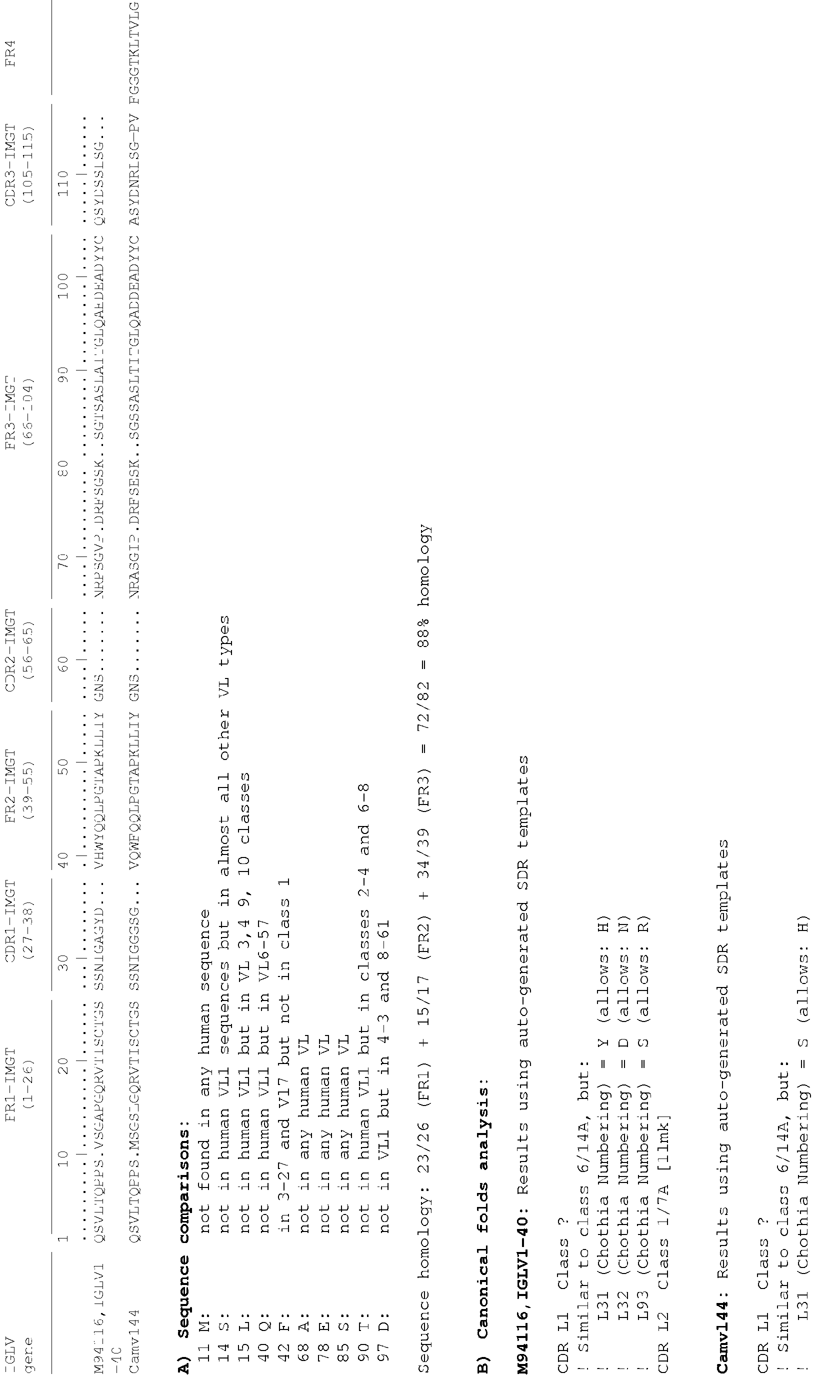

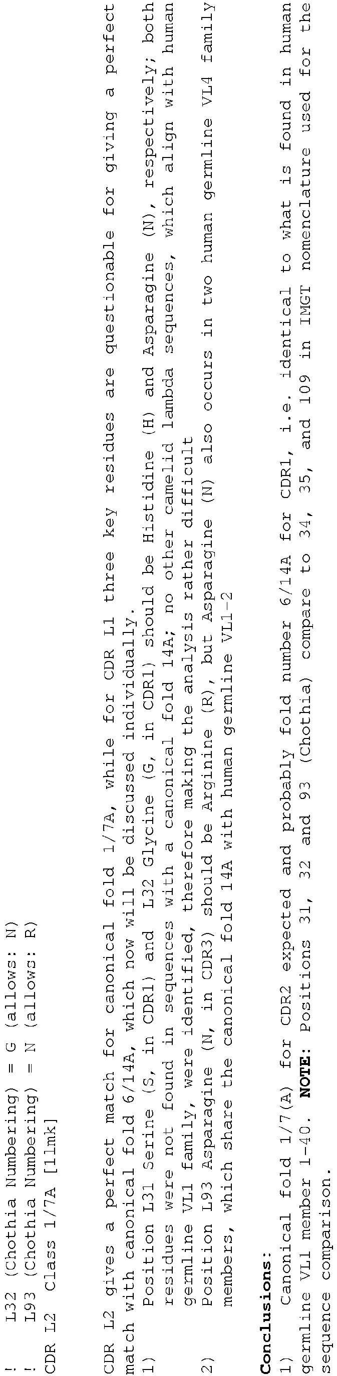

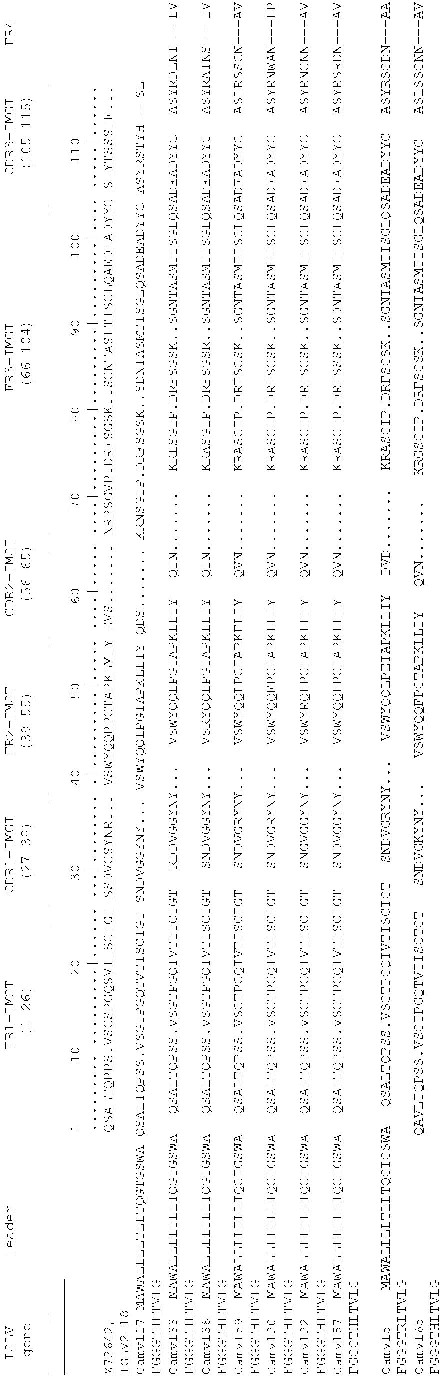

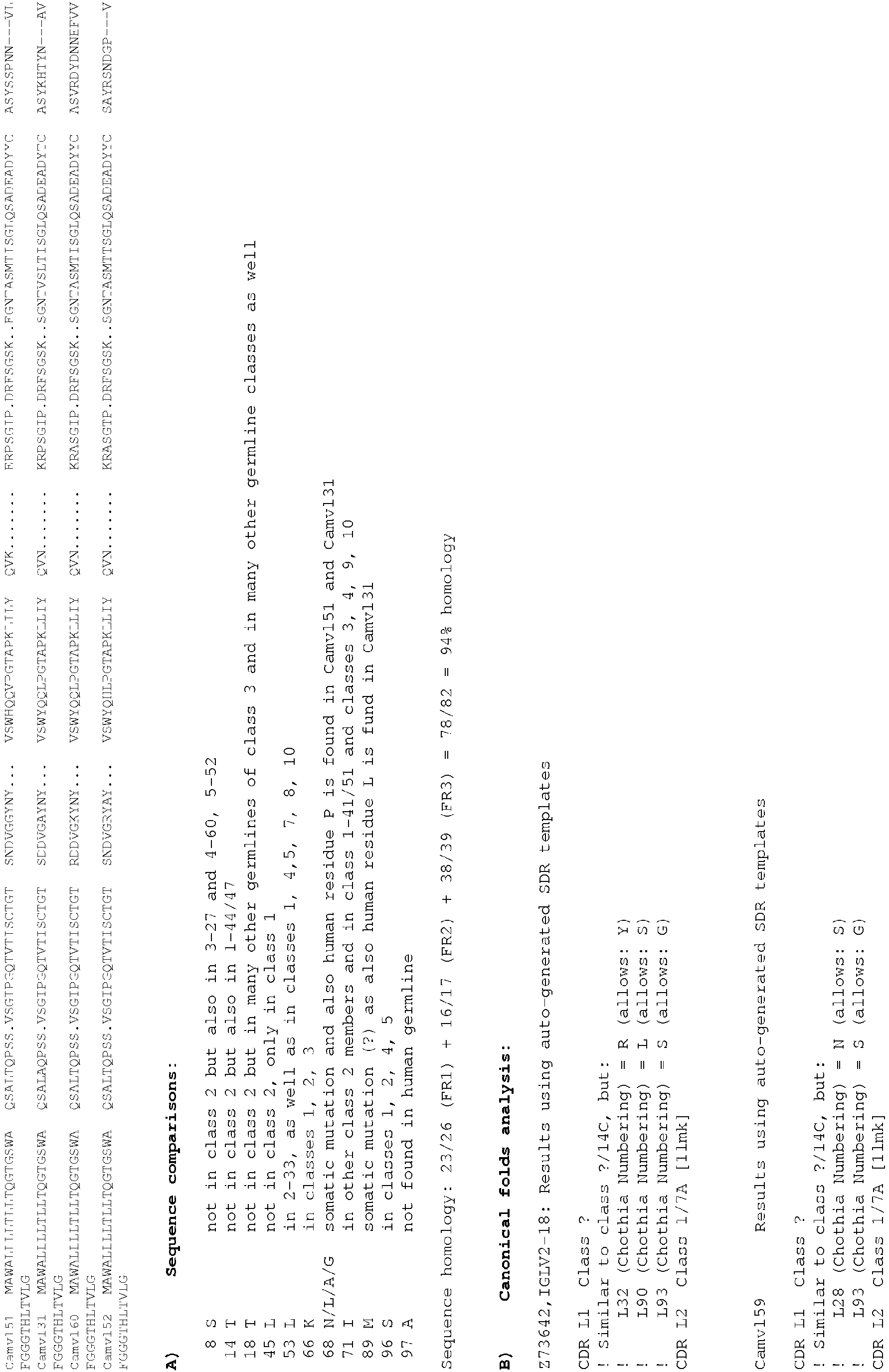

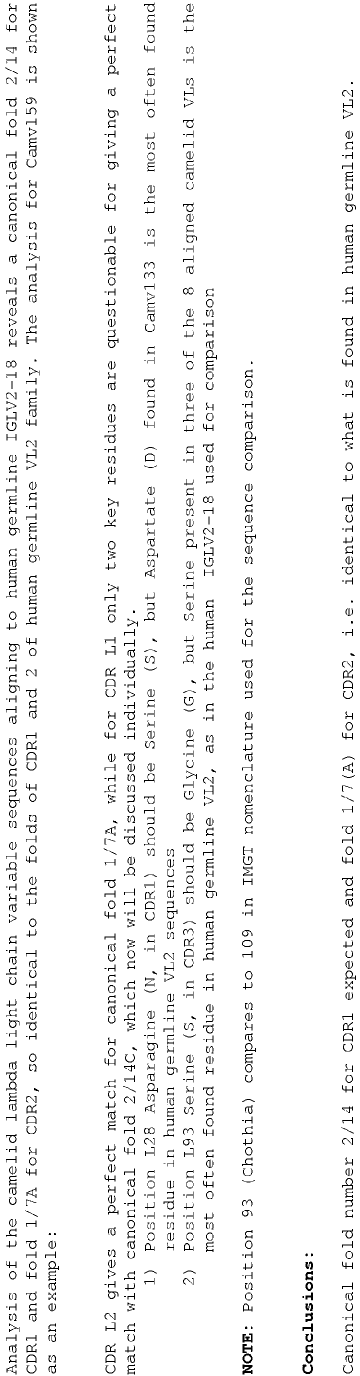

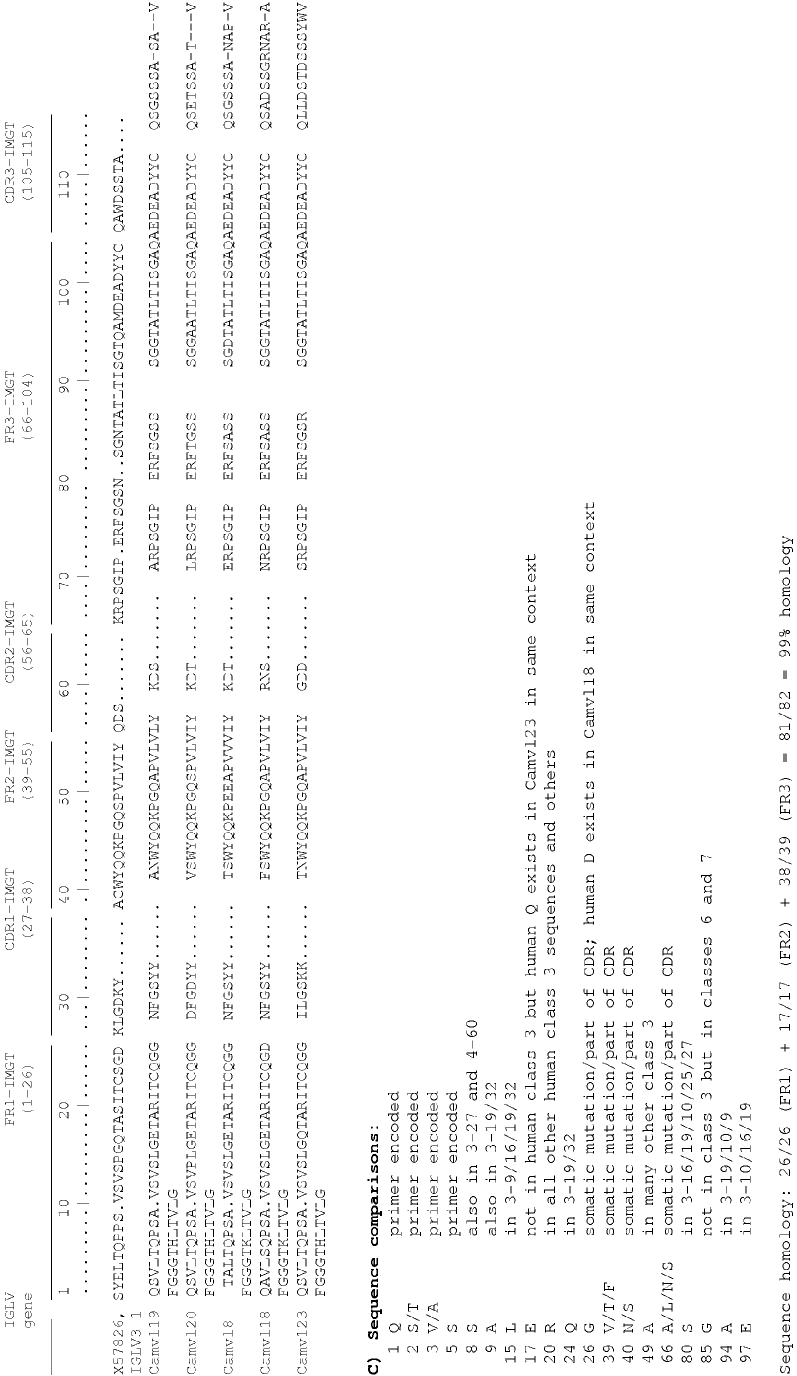

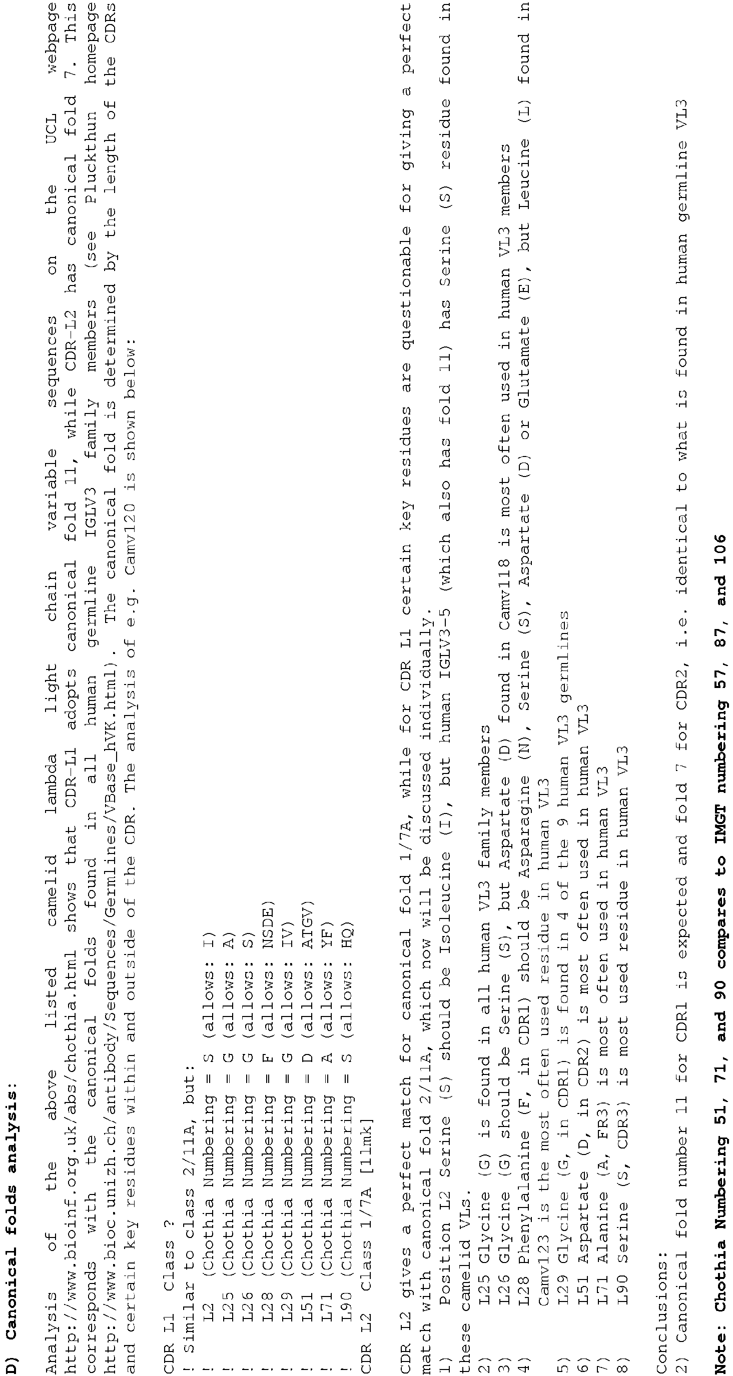

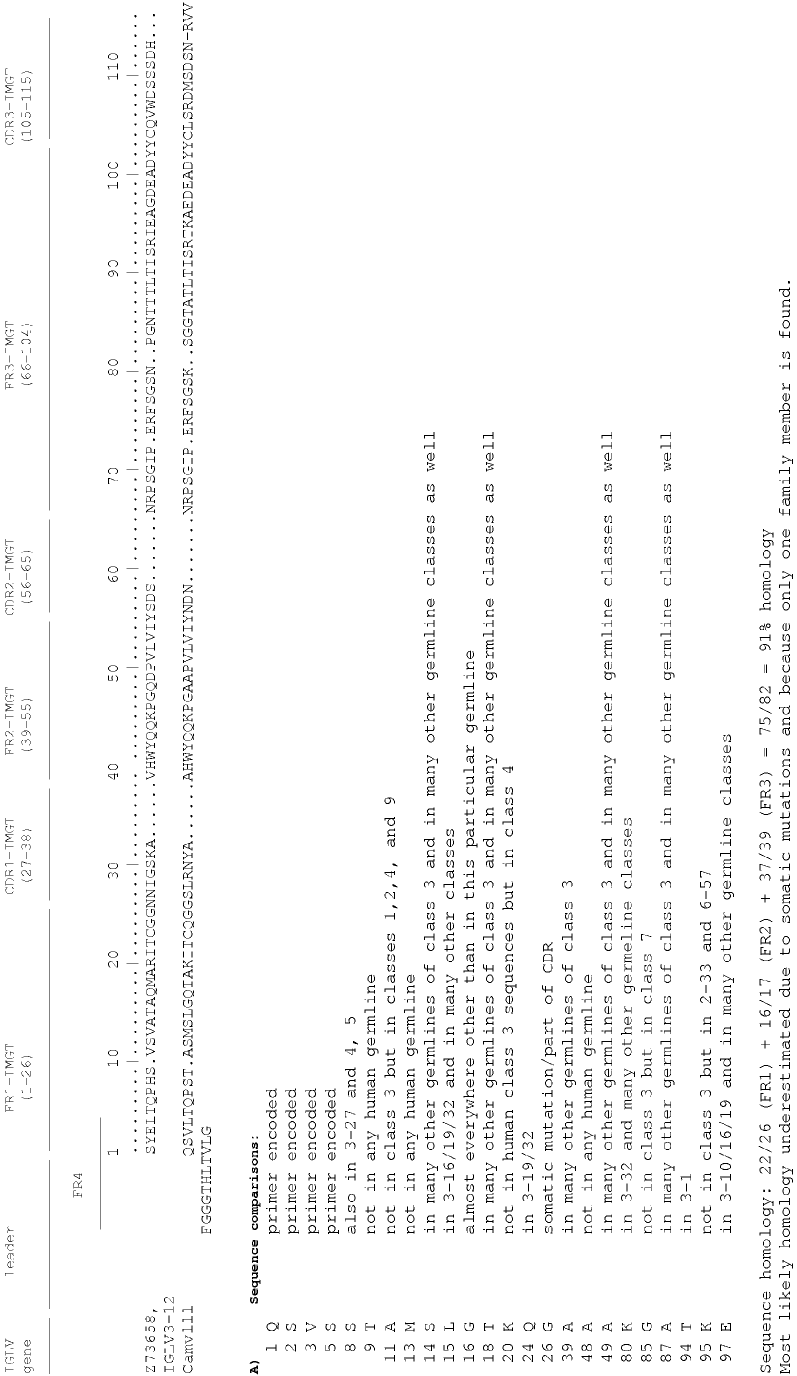

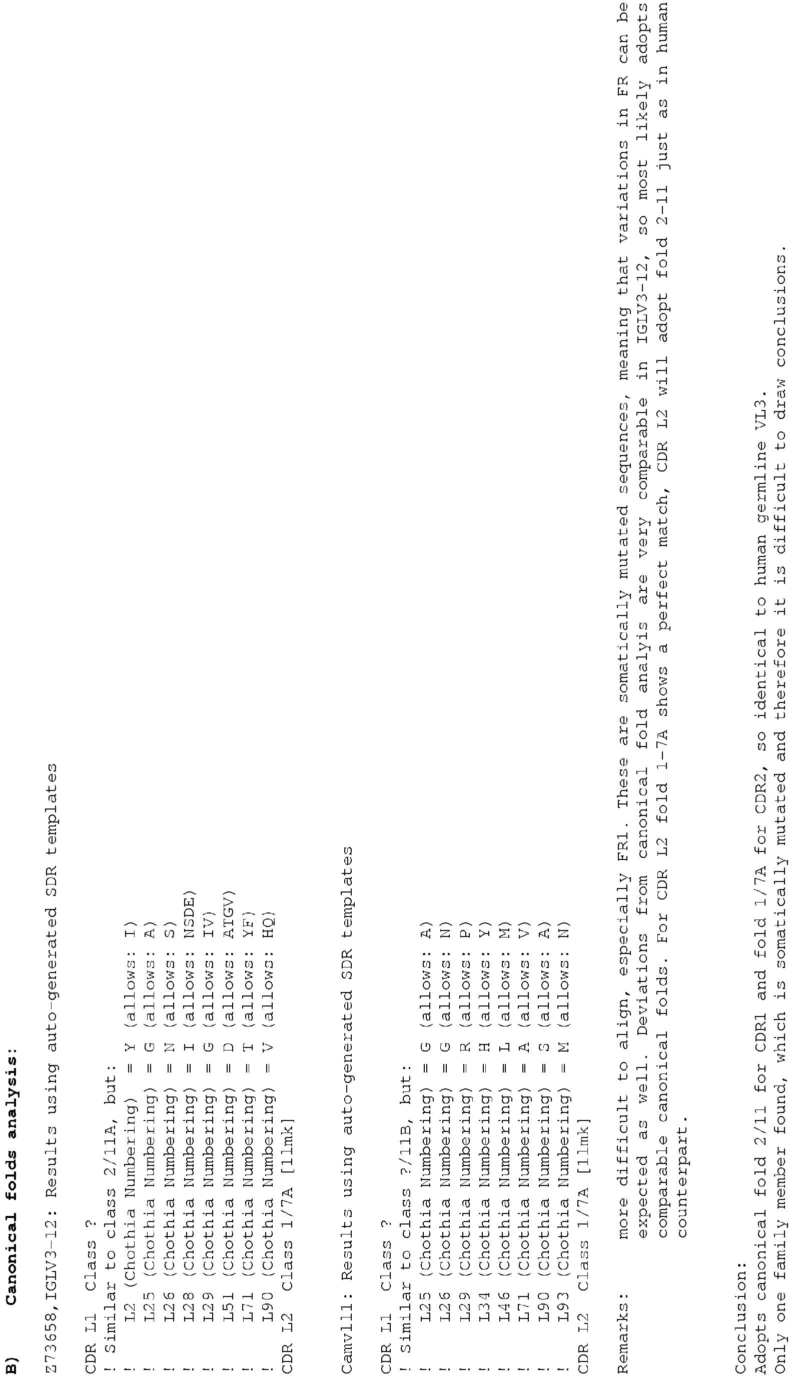

### 10.7 VKappa 2-40

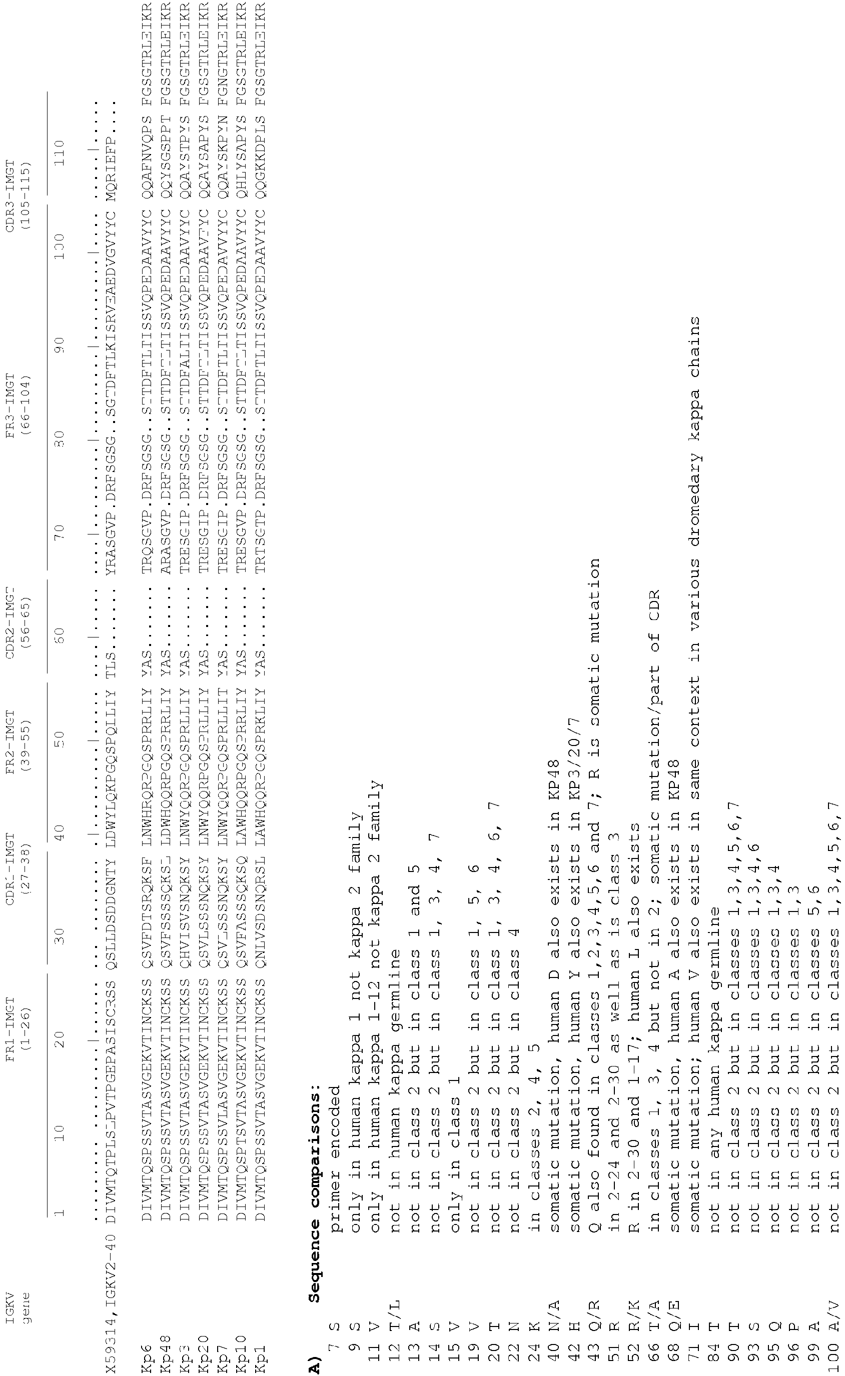

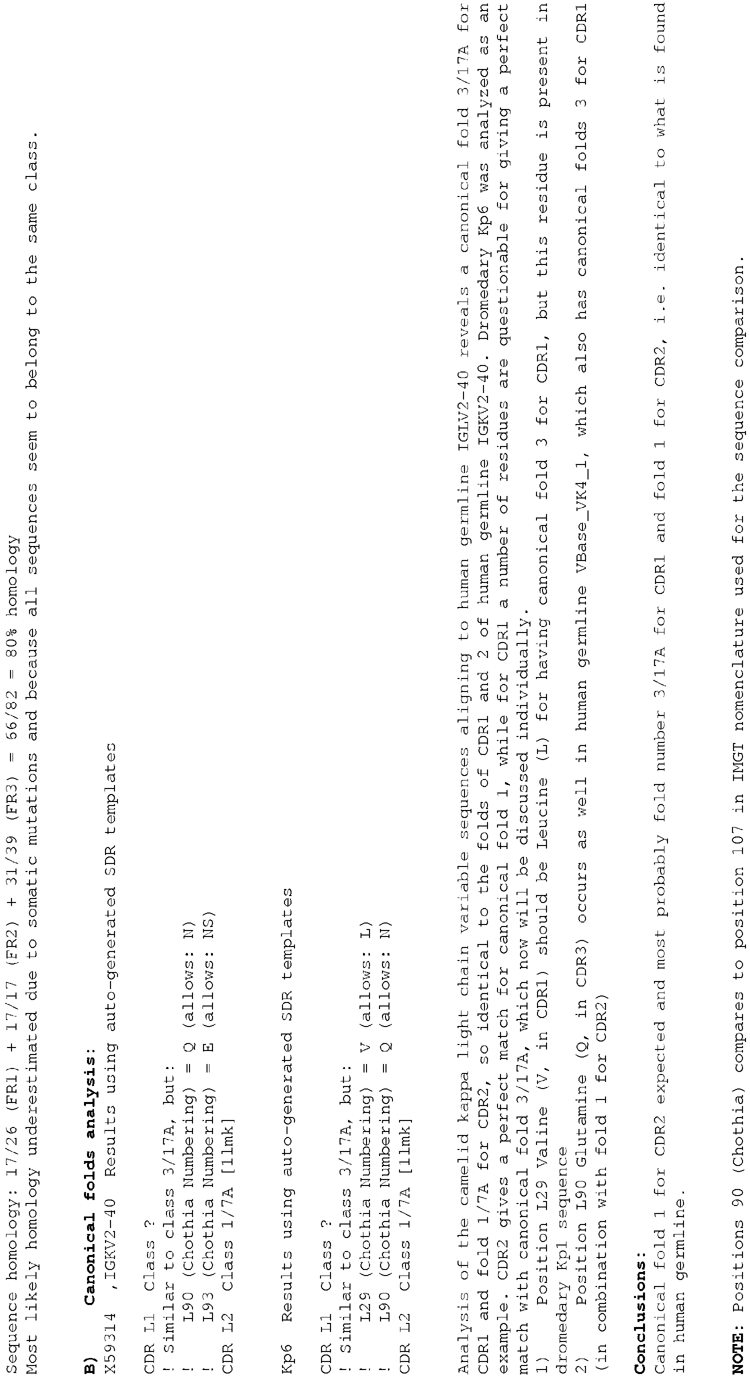

### 10.8 J(H) region comparisons of human and llama

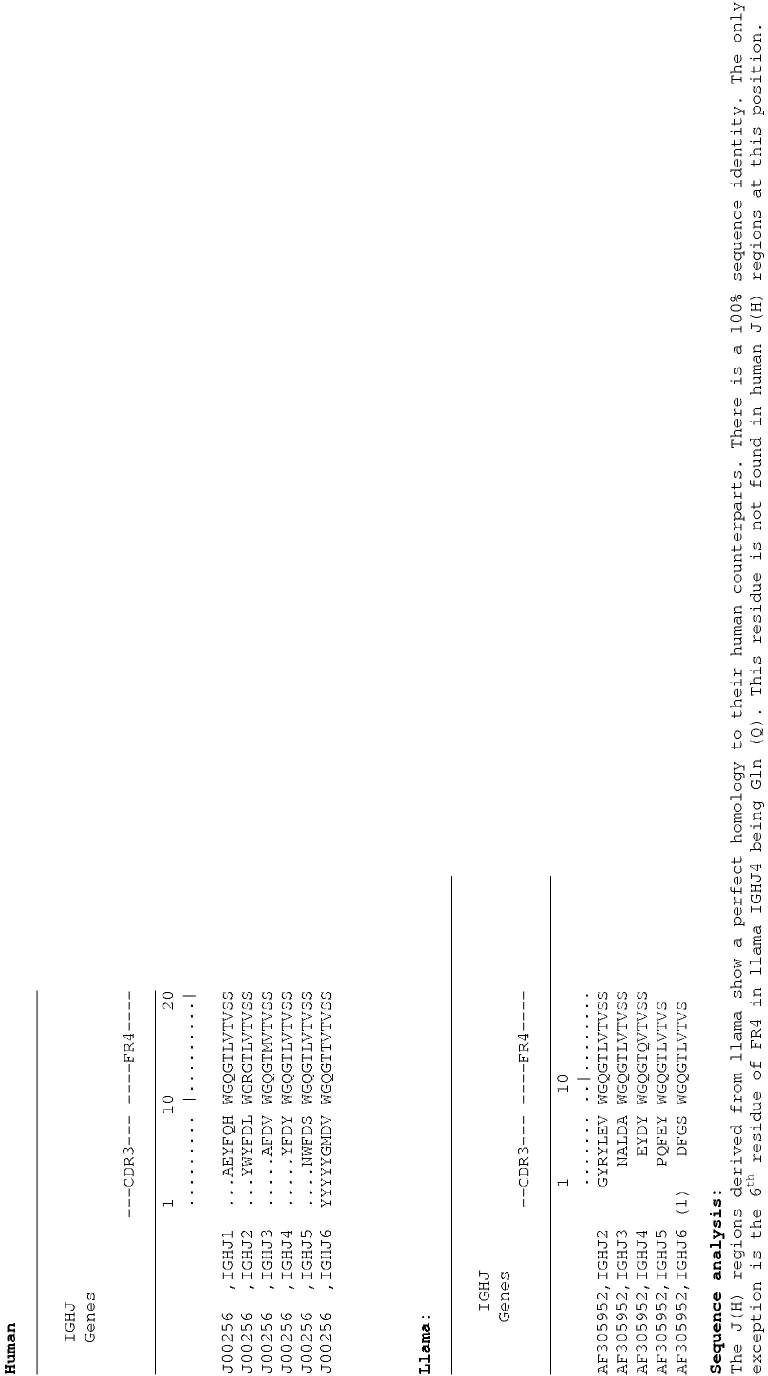

### 10.9 Comparison of light chain J region

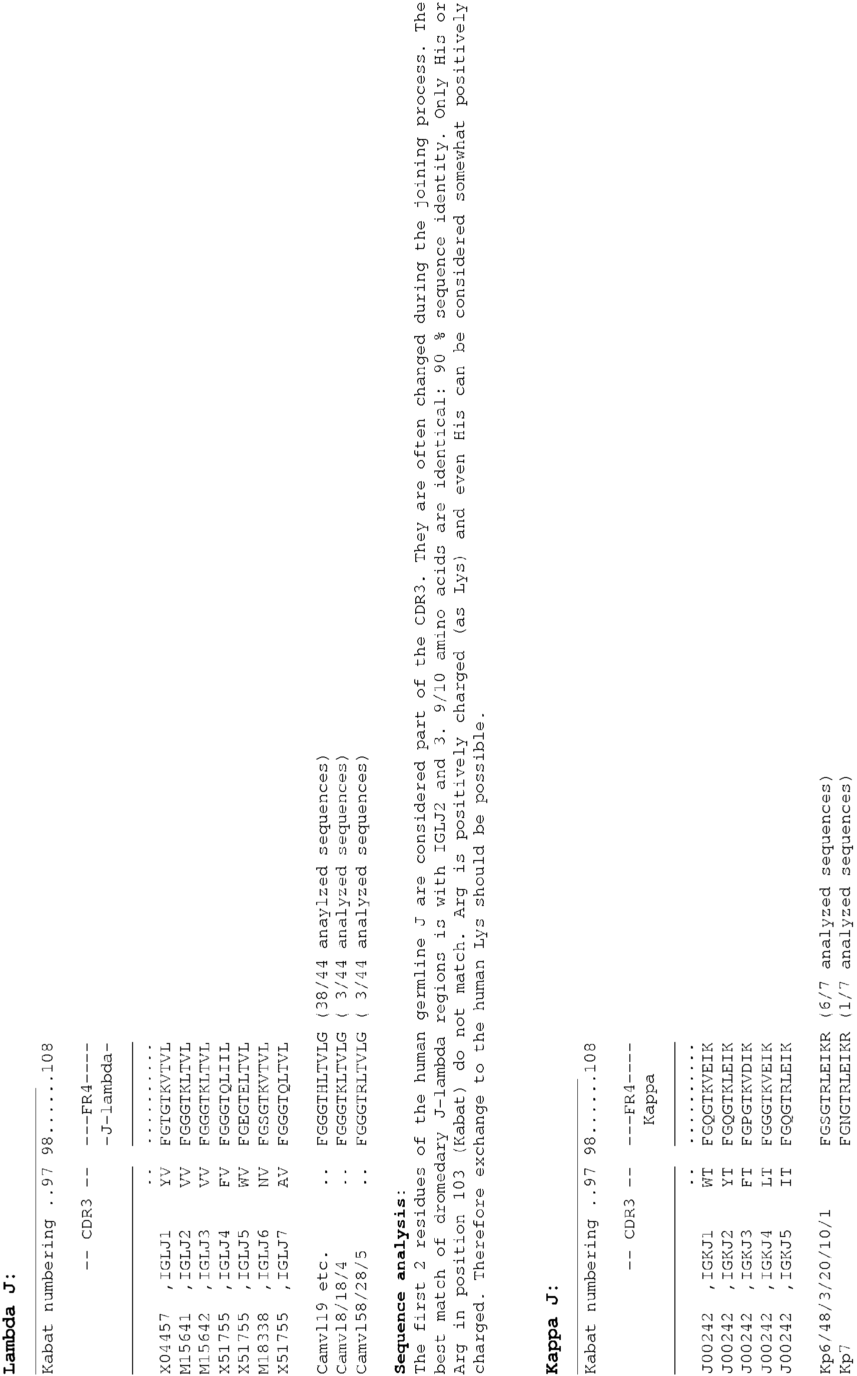

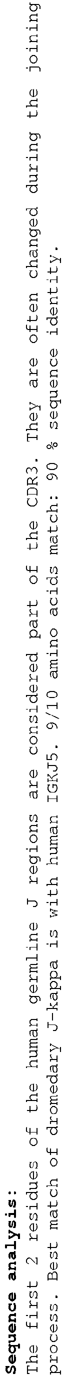

### 10.10 Lama pacos VH germaline analysis

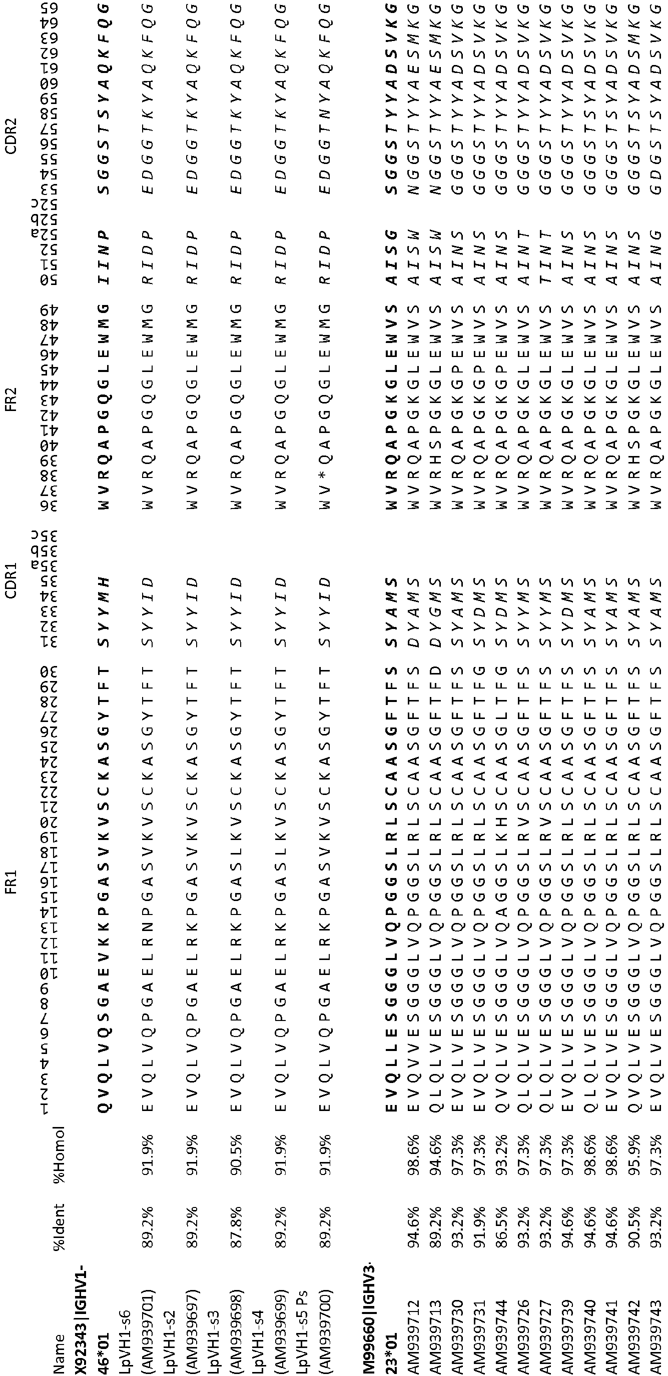

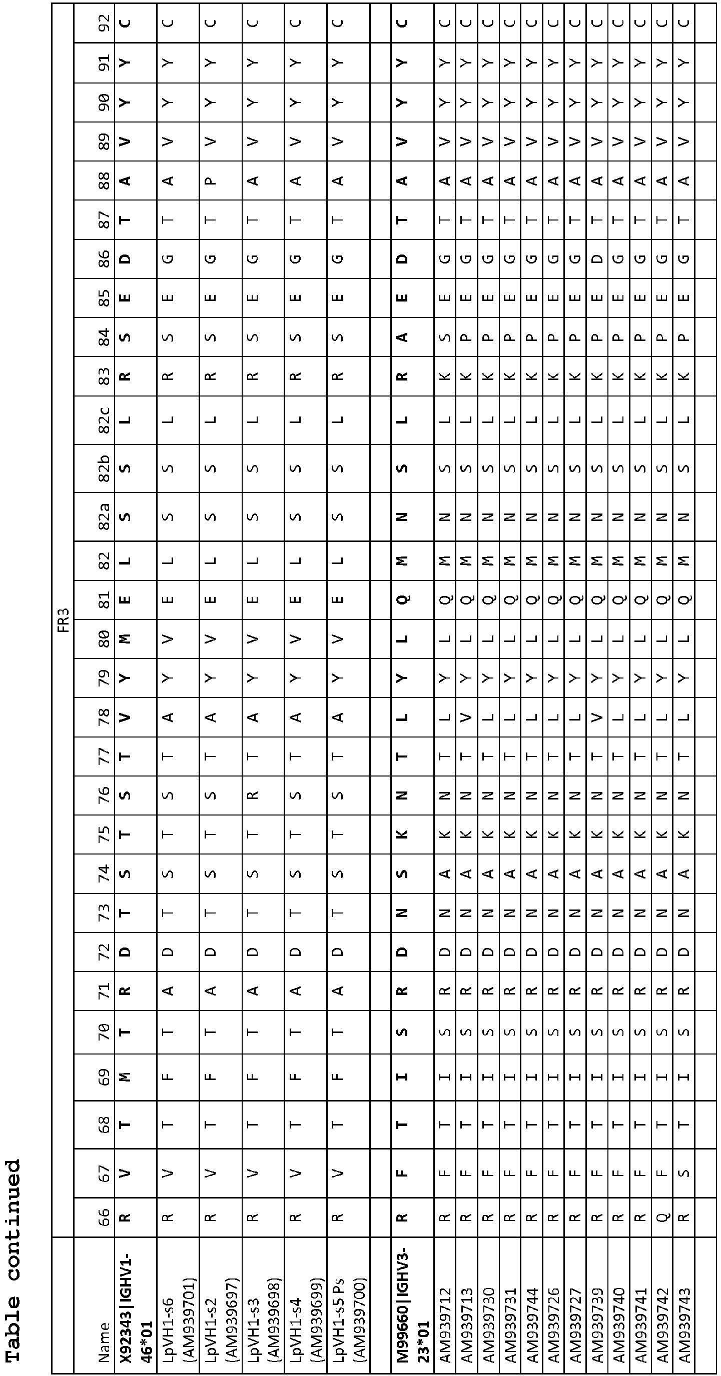

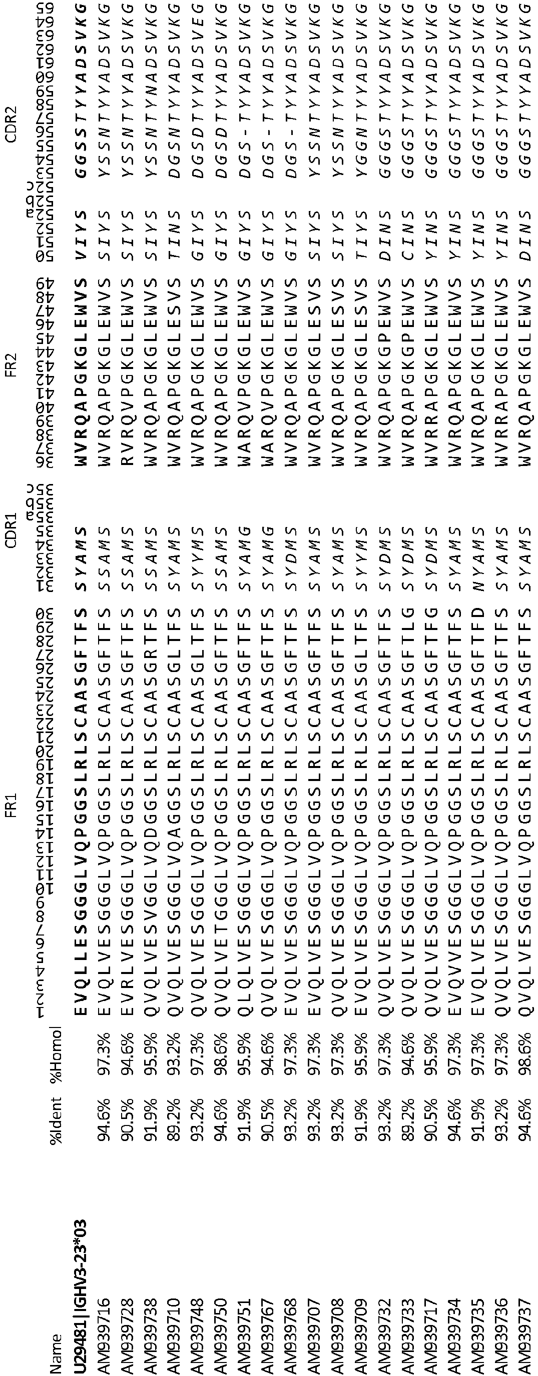

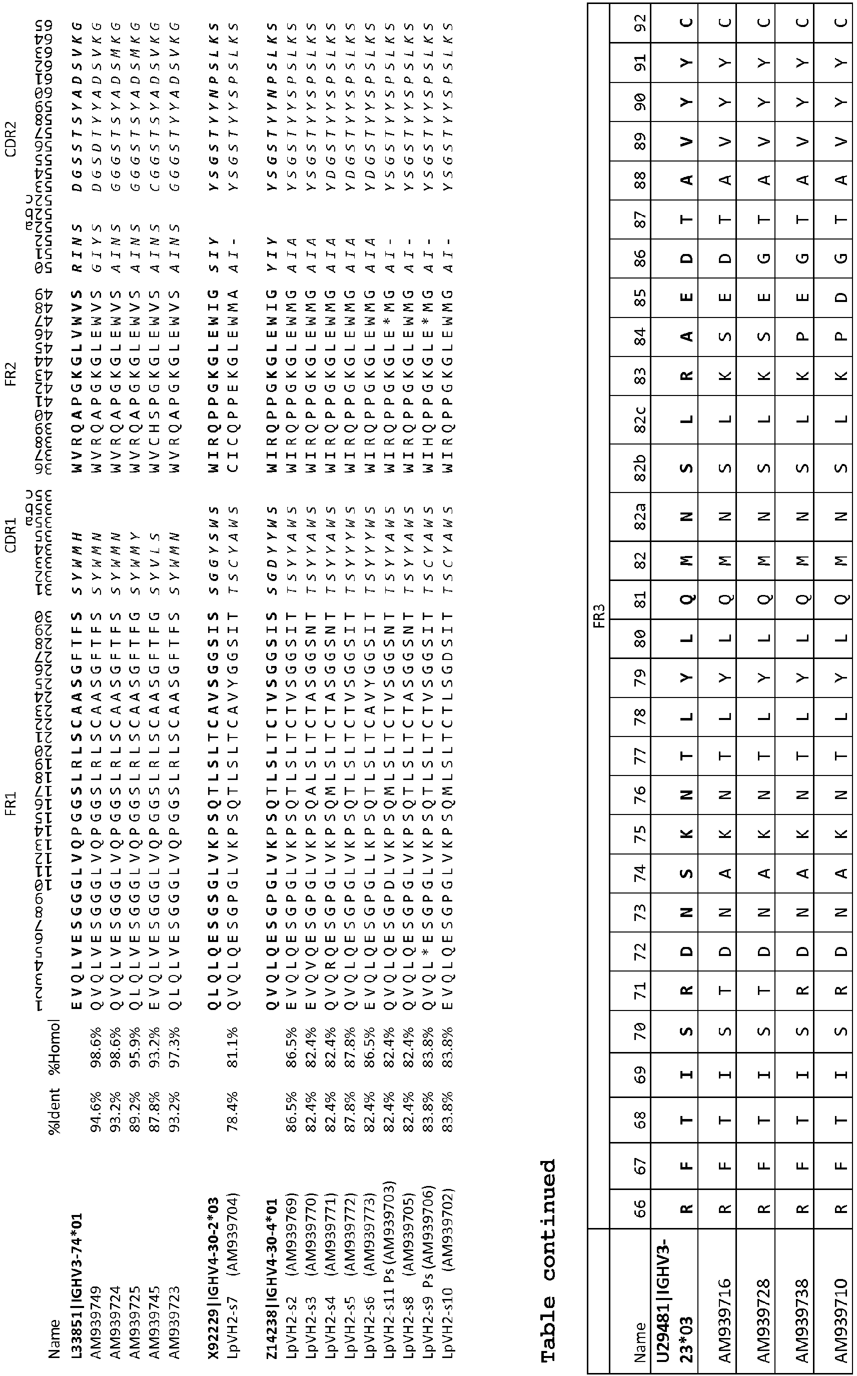

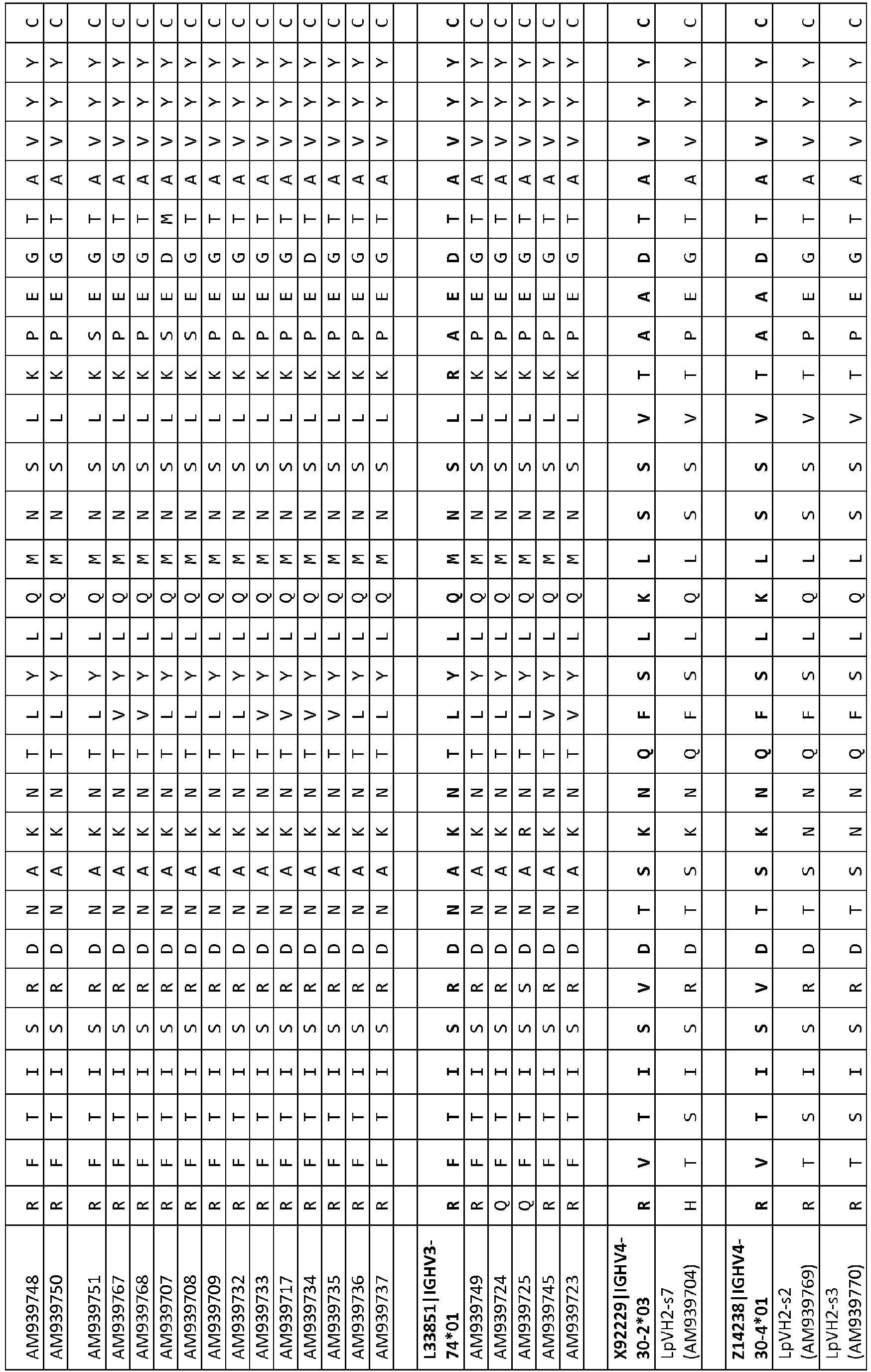

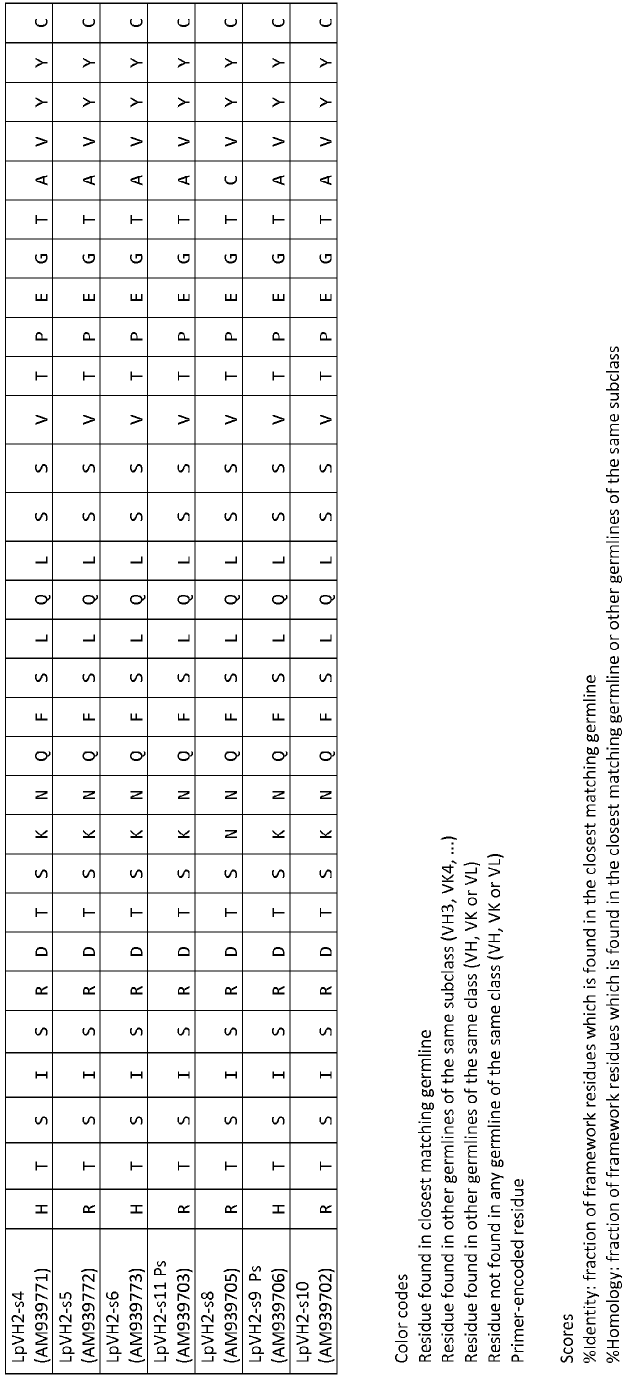

### 10.11 Lama glama derived VH analysis

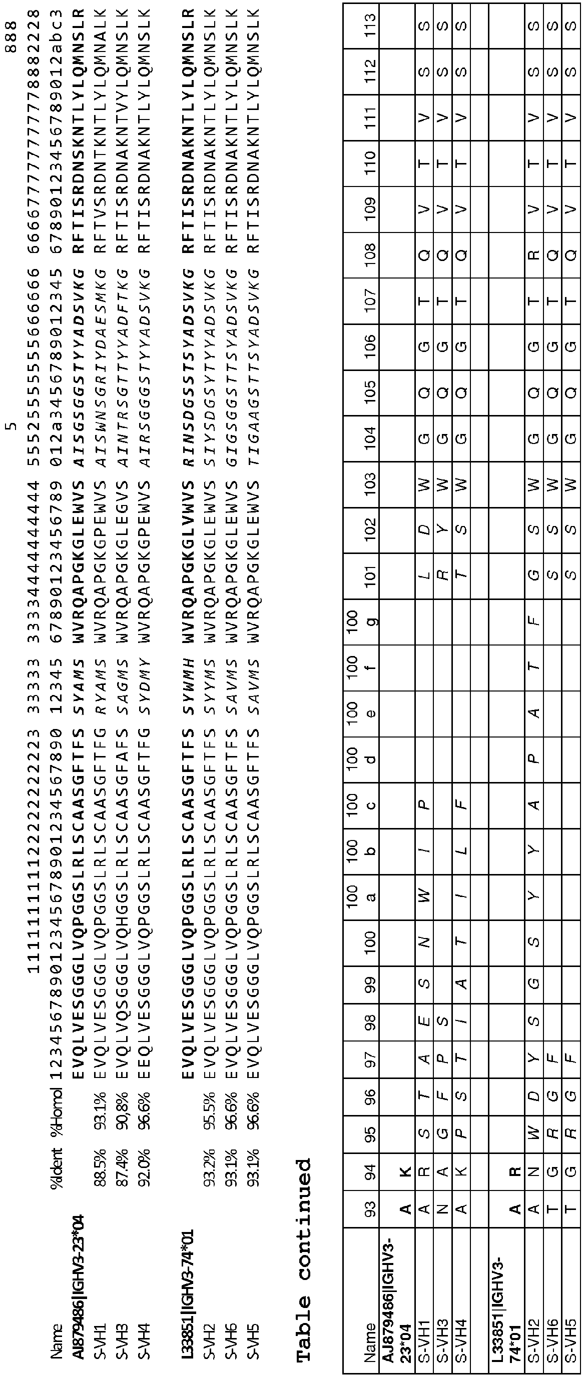

### 10.12 Lama glama derived VL analysis

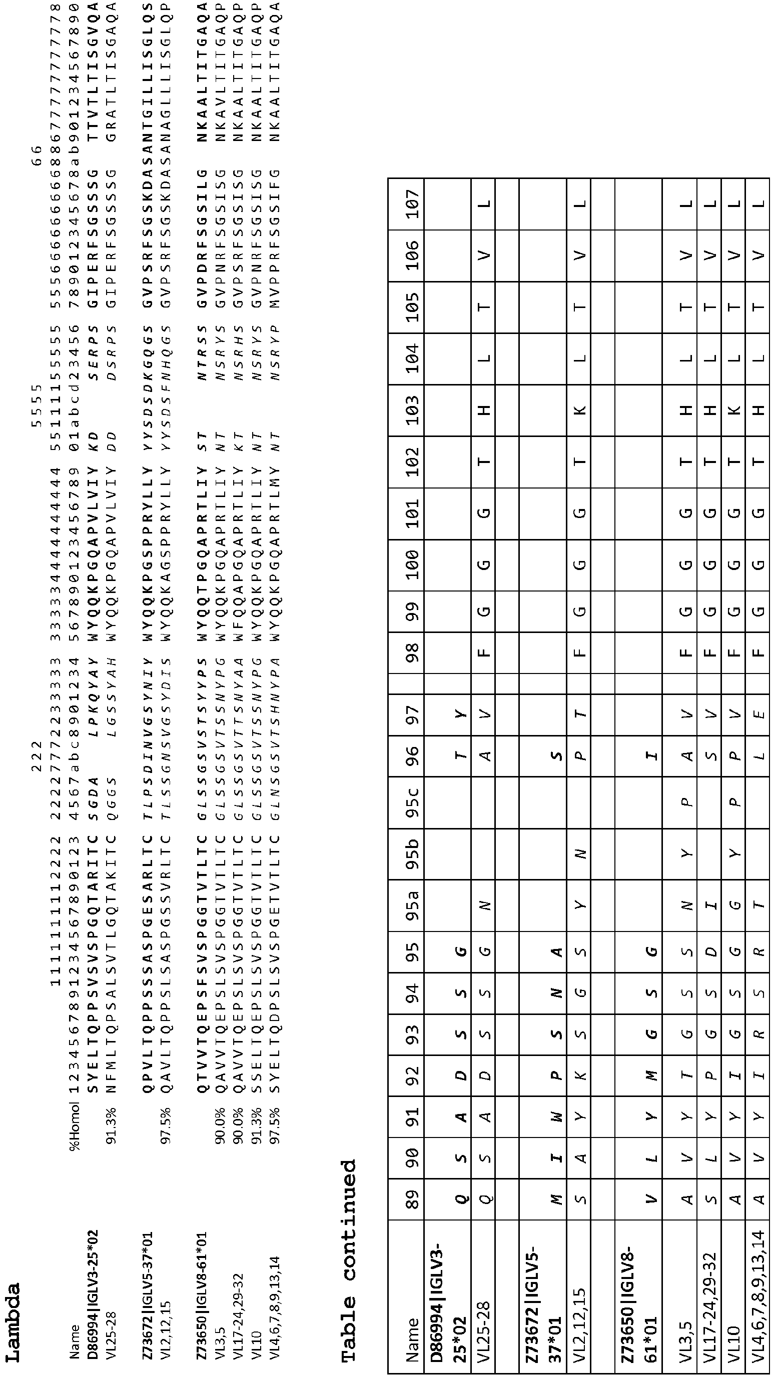

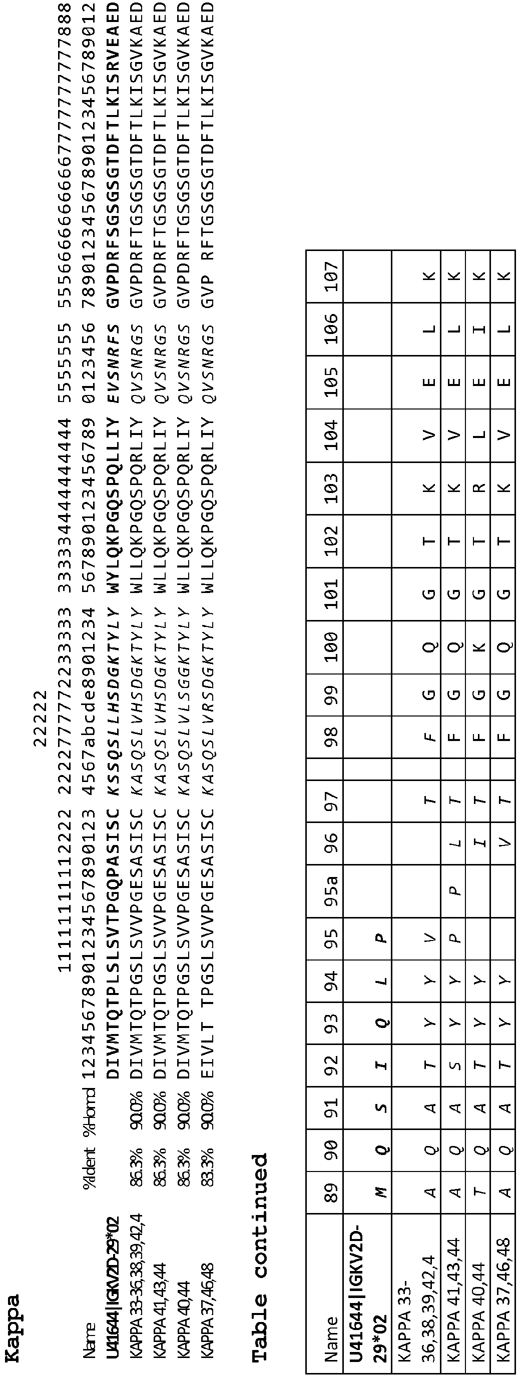

### Example 11 - Analysis of key residues for canonical folds of H1 and H2 and comparison of H1 and H2 residues with human germline

Structural analysis of antibodies revealed the relationship between the sequence and the shape of the binding site formed by the complementarity determining regions (Chothia and Lesk, J. Mol. Biol. 196:901-917 (1987); Tramontano et al., J. Mol. Biol. 215:175-82 (1990)). Despite their high sequence variability, five of the six loops adopt just a small repertoire of main-chain conformations, called "canonical structures". These conformations are first of all determined by the length of the loops and secondly by the presence of key residues at certain positions in the loops and in the framework regions that determine the conformation through their packing, hydrogen bonding or the ability to assume unusual main-chain conformations.

We have analyzed the predicted canonical structures of H1 and H2 for the germline dromedary and Ilama VH segments based on the length of these loops and the presence of the previously mentioned key residues. The comparison was made with the key residues as they occur in the closest matching human germline in terms of the presence of identical combination of canonical folds and overall sequence homology (Table 1); in addition the amino acids compatible with the corresponding canonical fold as proposed by Morea and colleagues (Morea et al., Methods 20:267-279 (2000)) are shown. For the dromedary germline VH family IGHV1S(1-19), which has canonical fold 1 for H1 (coded as H1: 1 in Table 1) and fold 1 for H2 (H2: 1), and family IGHV1S(20, 22, 23, 24) with fold 1 for H1 (H1: 1) and fold 3 for H2 (H2: 3), and family IGHV1S(21, 25-39) with fold 1 for H1 (H1: 1) and fold 2 for H2 (H2: 2) and Ilama germline IGHV1S8 with fold 1 for H1 (H1: 1) and fold 2 for H2 (H2: 2), the key residues 24, 26, 27, 29, 34 and 94 for the canonical fold of H1 are shown along with these from the analogue human germline family with the same canonical fold combination (upper part of Table 1). Also for dromedary germline VH family IGHV1S(1-19) with fold 1 for H1 (H1: 1) and fold 1 for H2 (H2: 1), and family IGHV1S(20, 22, 23, 24) with fold 1 for H1 (H1: 1) and fold 3 for H2 (H2: 3), and family IGHV1S(21, 25-39) with fold 1 for H1 (H1: 1) and fold 2 for H2 (H2: 2) and Ilama germline IGHV1 S8 with fold 1 for H1 (H1: 1) and fold 2 for H2 (H2: 1) the key residues 52a or 54 or 55 in combination with residue 71 are shown along with these from the analogue human germline families with the same canonical fold combinations (lower part Table 1).

The analysis clearly demonstrates the "human nature" of the canonical loops, since the key residues found in the camelid VH segments are identical to what is found in the corresponding human VH segments. For example, all 19 germline VH segments from dromedary coded IGHV1S(1-19) have Alanine on position 19, Glycine on 26, Phenylalanine on 27 and 29 and Methionine on 34 as they predominantly occur in human germline family 3 members, which like these dromedary germline sequences have a canonical fold type 1 for H1 and fold type 1 for H2. Position 94 is encoded only in a single germline dromedary (out of the total of 39 germline VH segments) meaning that a proper analysis is not possible. Nguyen and colleagues (Nguyen et al., EMBO J. 19:921-930 (2000)) noticed that the dromedary germline VH and VHH segments "span from the conserved octamer (i.e. recombination signal) to the Cysteine residue 92 of FR3" (end of citation), whereas the human segments encode two additional residues (93 and 94). However, we will discuss this residue during the analysis of the only six known somatically mutated VH segments derived from Ilama derived conventional antibodies.

There are virtually no exceptions for the perfect match with the human germline segments and the key residues proposed by Morea and colleagues (Morea et al., Methods 20:267-279 (2000)), besides residue 52a for Ilama germline IGHV1S6 for canonical fold type 2 for loop H2, which has Serine on this position, while the human analogue uses Threonine. It is encouraging to observe that in four of the six published Ilama VH derived from somatically mutated conventional antibodies (Vu et al., Mol. Immunol. 34:1121-31 (1997) Threonine is found on position 52a. It is worthwhile to mention that Serine and Threonine are closely related, since they both have a polar hydroxyl group and small sidegroups, suggesting that it might be possible to exchange both residues during humanization.

Glutamine on position 71, which occurs in 1 out of 19 dromedary germline VH segments with a canonical fold type 1 for loop H2 and in 4 out of 16 dromedary germline VH with a canonical fold type 2 for H2, seems to be rather extraordinary. The H2 loop packs against the residue at site 71 and the position of the loop relative to the framework is mainly determined by the size of the residue at this site. Canonical structures 2 and 3 are found in H2 loops with 6 residues. Structure 2 occurs when residues 52a and 71 are small or medium sized hydrophobic residues, while canonical fold 3 occurs when residue 71 is Arginine or Lysine. It can not be predicted how often the dromedary germline segments with Glutamine on position 71 will be used in somatically mutated conventional antibodies, but the humanization of this residue in antibody leads with this particular residue needs to be carefully examined. The presence of Arginine and Glutamine on position 71 in the dromedary IGHV1S(20, 22, 23, 24) family members with canonical fold 2 for H2 is rather unexpected, but on the other hand human germline VH1 family members VH1-9, VH1-10 and VH1-11 with canonical fold 2 for H2 have Arginine as well, while VH5 member 5-1 with fold 2 for H2 carries Glutamine on this position.

As Chothia and colleagues did when discussing the structural repertoire of the human VH segments (Chothia et al., J. Mol. Biol. 227:799-817 (1992)) we examined the individual amino acid residues of the H1 and H2 loops of the dromedary and Ilama VH segments along with the key residues and compared these with the human counterparts having the same canonical fold combination (Table 2). For dromedary germline VH family IGHV1S(1-19) with canonical fold 1 for H1 and fold 1 for H2, and family IGHV1S(20, 22, 23, 24) with fold 1 for H1 (H1: 1) and fold 3 for H2 (H2: 3), and family IGHV1S(21, 25-39) with fold 1 for H1 (H1: 1) and fold 2 for H2 (H2: 2) and Ilama germline IGHV1 S8 with fold 1 for H1 and fold 2 for H2 the H1 residues 26 to 33 and the are shown together with the key residues 24 and 94 located outside of H1 (Table 2A). In addition, for dromedary germline VH family IGHV1S(1-19) with canonical fold 1 for H1 (H1: 1) and fold 1 for H2 (H2: 1), and family IGHV1S(20, 22, 23, 24) with fold 1 for H1 (H1: 1) and fold 3 for H2 (H2: 3), and family IGHV1S(21, 25-39) with fold 1 for H1 (H1: 1) and fold 2 for H2 (H2: 2) and Ilama germline IGHV1S8 with fold 1 for H1 (H1: 1) and fold 2 for H2 (H2: 2) the H2 residues 52 to 56 were analyzed with key residue 71, which is located outside H2 (Table 2B).

It is surprising to see the very high degree of sequence homology in the variable loops, especially in H1 there are hardly differences with the relevant human sequences. For instance, germline dromedary family IGHV1 S(1-19) with canonical fold combination H1: 1 / H2: 1 contains predominantly Alanine on position 24, Glycine on 26, Phenylalanine on 27, Threonine on 28, Phenylalanine on 29, Serine on 30 and 31, Tyrosine on 33, Methionine on 34 and Serine on 35, which completely match the human germline family 3 members that share the same combination of H1 and H2 canonical folds. Exceptions are residue 27 (Phenylalanine) and 32 (Serine) of the only publicly known Ilama germline VH segment, but again in 4 out of 6 somatically mutated Ilama VH which are publicly known (Vu et al., Mol. Immunol. 34:1121-1131 (1997)). Tyrosine is present on 32 as is found in the analogue human germlines. The same high degree of sequence homology is found for the H2 loops of dromedary germline VH segments with the exception of residue 54 of family IGHV1S(1-19). Especially dromedary family IGHV1S(21, 25-39) deviates on a number positions in the H2 loop (i.e. 53, 54, 56 and 58). The H2 loop of the Ilama germline VH segment with the same fold scores much better, but as well contains a number of deviating residues on position 50, 52, 52a, 54, 55 and 58, but the interpretation is rather difficult, since the analysis was performed with the only known germline segment. The analysis of somatically mutated VH derived from Ilama shows that certain residues on these positions occur, which also appear in the corresponding human germline sequences, although infrequently (f.i. Glycine on 50, Asparagine on 52 and 58, Threonine on 52a and Glycine on 55).

We also analyzed the panel of six publicly known somatically mutated VH sequences from Ilama (Vu et al., Mol. Immunol. 34:1121-31 (1997)). Below the alignment with human VH3 member 3-23 is shown, demonstrating a very high degree of sequence homology: overall, only 3 deviating residues were observed, one of which is encoded by the primer used for amplification, while the other two occur in human germlines of the same class. Even the CDRs show to have a very high degree of sequence homology: CDR1 is probably identical, while only three residues of CDR2 are different. Canonical fold analysis reveals that two VH have fold 1 for H1 and fold 2 for H2, as was observed for the only available Ilama derived germline VH, but the other four have fold 1 for H1 and fold 3 for H2 as occurs in 3-23 and the majority of the human family VH3 germline segments. This might be suggesting that these are derived from other, not yet known germline VH segments. Examination of the key residues supporting the canonical folds gives the perfect match with those occurring in human germline with the same canonical fold combination as was already observed for the Ilama germline VH segment listed in Table 1. It is very interesting to see that key residue 94 in these somatically mutated sequences is Lysine (2 out of 5), Serine (1 out of 5) and Arginine (1 out of 5), which are all found in the human germlines with the same fold combination or are proposed by Morea and colleagues (Morea et al., Methods 20:267-279 (2000)).

### 11.1 Somatically mutated Llama VH from conventional antibodies

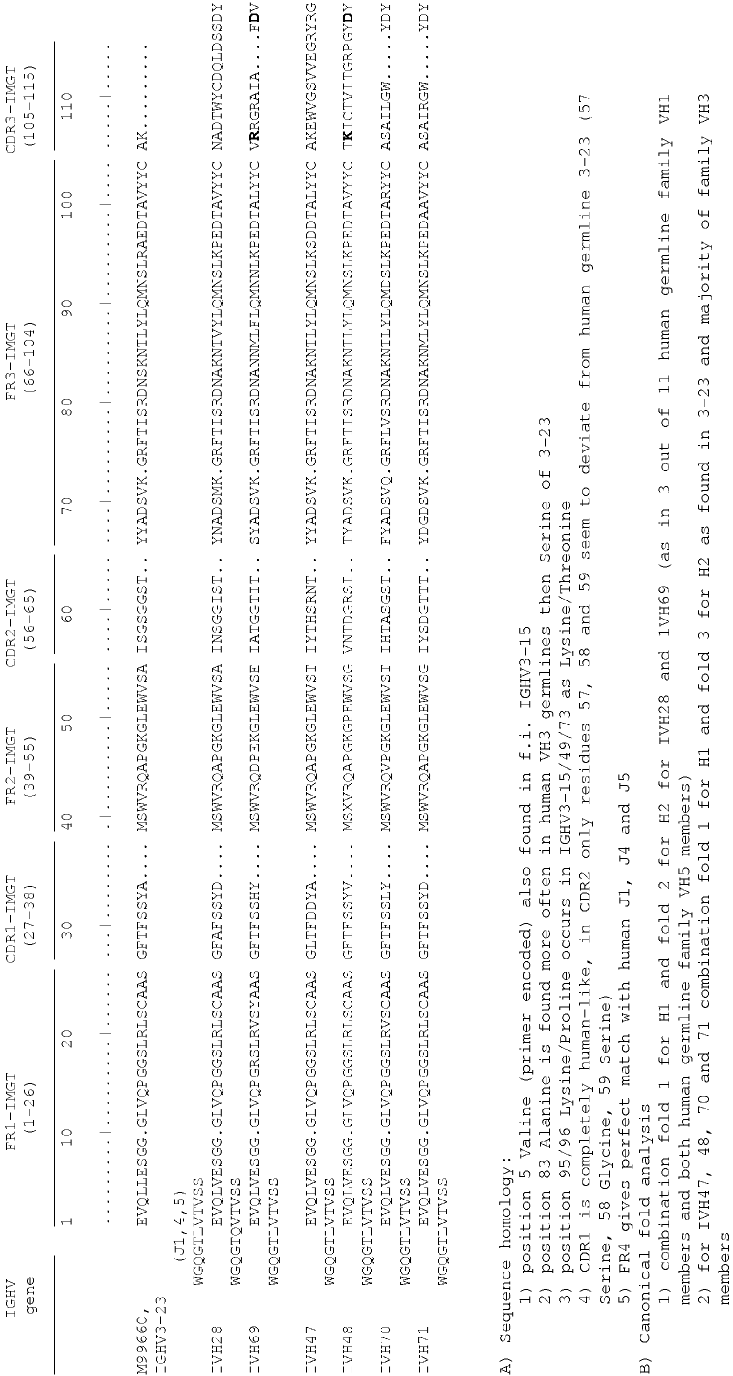

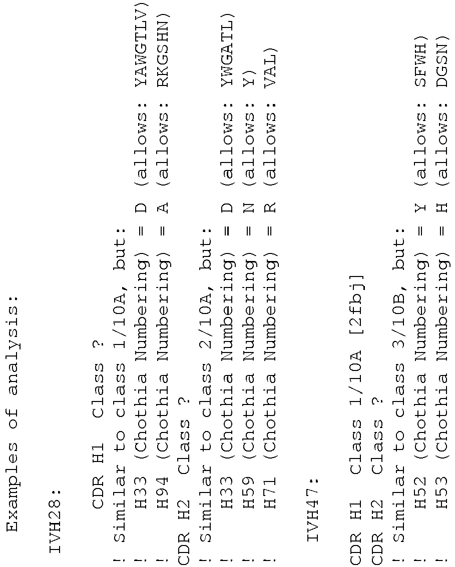

### Example 12 - Analysis key residues for canonical folds of L1 (λ) and L2(λ) and comparison of L1(λ) and L2(λ) residues with human germline

Also the predicted canonical structures of L1 and L2 were analyzed for the somatically mutated dromedary VLambda segments based on length of the loops and the presence of the key residues relevant for the canonical folds. The comparison was made with the key residues occurring in the closest matching human germline with the same combination of canonical folds and overall sequence homology (Table 3). For the somatically mutated dromedary VL family VL3-1 (Camvl8, 18, 19, 20 and 23), with fold 11 for L1 (L1: 11) and fold 7 for L2 (L2: 7), and somatically mutated dromedary VL family VL3-12/32 (Camvl11) with fold 11 for L1 (L1: 11) and fold 7 for L2 (L2: 7), and somatically mutated dromedary VL1-40 (Camvl44) with fold 14 for L1 (L1: 14) and fold 7 for L2 (L2: 7), and somatically mutated dromedary VL2-18 (Camvl5, 17, 30-33, 36, 52, 57, 59, 60 and 65) with fold 14 for L1 (L1: 14) and fold 7 for L2 (L2: 7) the key residues 2, 25, 29, 30, 33 and 71 relevant for the canonical fold of L1 are shown along with these from the analogue human germline family with the same canonical fold combination (upper part of Table 3). For somatically mutated dromedary VL family VL3-1 (Camvl8, 18, 19, 20 and 23), with fold 11 for L1 (L1: 11) and fold 7 for L2 (L2: 7), and somatically mutated dromedary VL family VL3-12/32 (Camvl11) with fold 11 for L1 (L1: 11) and fold 7 for L2 (L2: 7), and somatically mutated dromedary VL1-40 (Camvl44) with fold 14 for L1 (L1: 14) and fold 7 for L2 (L2: 7), and somatically mutated dromedary VL2-18 (Camvl5, 17, 30-33, 36, 52, 57, 59, 60 and 65) with fold 14 for L1 (L1: 14) and fold 7 for L2 (L2: 7) the key residues 48 and 64 important for the canonical fold of L2 are shown along with the key residues from the analogue human germline families having the same canonical fold combinations (lower part Table 3).

As observed for VH the analysis reveals the "human nature" of both canonical loops L1 and L2, because here also the key residues of the camelid VLambda segments are identical to those of the corresponding human VLambda segments. For example, in dromedary VL3-12/32, VL1-40 and VL2-18 all key residues of L1 are identical to those occurring in the corresponding human germlines, and in dromedary VL3-1, VL3-12/32 and VL2-18 the key residues of L2 completely match with the corresponding human germline VL segments. Basically there are only two exceptions. First of all, L2 key residue 64 of VL1-40 is Glutamate, which is rather different from Glycine that is present in human germline Vλ1 with the same L1 / L2 combination of canonical folds as VL1-40. It is difficult to draw general conclusions, since VL1-40 only consists of an orphan (i.e. Camvl44). The second exception is VL3-1, where Phenylalanine is the most dominantly occurring L1 key residue on position 30, whereas Leucine is frequently found in human Vλ family 3 members that share fold 11 for L1 and fold 7 for L2 with VL3-1. However, Leucine is also present in one out of the five VL3-1 members.

We analyzed the individual amino acid residues of the L1 and L2 loops of the somatically mutated dromedary VLambda segments along with the key residues and made the comparison with the human counterparts sharing the same canonical fold combination (Table 4). For the somatically mutated dromedary VL family VL3-1 (Camvl8, 18, 19, 20 and 23), with fold 11 for L1 (L1: 11) and fold 7 for L2 (L2: 7), and somatically mutated dromedary VL family VL3-12/32 (Camvl11) with fold 11 for L1 (L1: 11) and fold 7 for L2 (L2: 7), the L1 residues 27 to 33 together with key residue 71 outside L1 are compared with the same residues present in the corresponding human germline Vλ with same combination of folds for L1 and L2 (upper part of Table 4A). For dromedary somatically mutated dromedary VL1-40 (Camvl44) with fold 14 for L1 (L1: 14) and fold 7 for L2 (L2: 7), and somatically mutated dromedary VL2-18 (Camvl5, 17, 30-33, 36, 52, 57, 59, 60 and 65) with fold 14 for L1 (L1: 14) and fold 7 for L2 (L2: 7) the L1 residues 26 to 33 together with key residues 2 and 71 outside L1 are compared with the same residues present in the corresponding human germline Vλ family with same combination of folds for L1 and L2 (lower part of Table A). In addition, for somatically mutated dromedary VL family VL3-1 (Camvl8, 18, 19, 20 and 23), with fold 11 for L1 (L1: 11) and fold 7 for L2 (L2: 7), and somatically mutated dromedary VL family VL3-12/32 (Camvl11) with fold 11 for L1 (L1: 11) and fold 7 for L2 (L2: 7), and somatically mutated dromedary VL1-40 (Camvl44) with fold 14 for L1 (L1: 14) and fold 7 for L2 (L2: 7), and somatically mutated dromedary VL2-18 (Camvl5, 17, 30-33, 36, 52, 57, 59, 60 and 65) with fold 14 for L1 (L1: 14) and fold 7 for L2 (L2: 7) the L2 residues 49 to 53 together with key residues 48 and 64 located outside L2 are compared with the same residues present in the corresponding human germline Vλ family with the same combination of canonical folds for L1 and L2 (Table 4B). There is a high degree of sequence homology between the L1, L2 and key residues with the human germline sequences and only few exceptions exist, which mainly can be found in the orphan members of the VL3-12/32 and VL1-40 families. For L1 of VL3-12/32 residues 27, 28, 30, 30a and 30b deviate from the corresponding human germline Vλ family 3, while for the same loop residues 30b, 31 and 32 of VL1-40 are different from the matching human germline Vλ family 1. In the orphan member of VL3-12/32 L2 residue 50 differs from the human analogue, while for the only member of VL1-40 key residue 64 differs from the human analogue. A fundamental difference can be observed for L1 residue 28 of dromedary family VL2-18 (Asparagine or Glutamate versus Serine in analogue human Vλ family 2).

### Example 13 - Analysis key residues for canonical folds of L1 (K) and L2(K)) and comparison of L1 (K)) and L2(K)) residues with human germline

The predicted canonical structures of L1 (K)) and L2(K)) were analyzed for the somatically mutated dromedary VKappa segments based on loop length and presence of key residues. As before the comparison was made with the key residues occurring in the closest matching human germline with the same combination of canonical folds and overall sequence homology (Table 5). For the somatically mutated dromedary VK family VK2-40 (Kp1, 3, 6, 7, 10, 20 and 48), with fold 3 for L1 (L1: 3) and fold 1 for L2 (L2: 1) the key residues 2, 25, 29, 30e, 33 and 71 relevant for the canonical fold of L1 are shown along with these from the analogue human VKappa germline family 2 with the same canonical fold combination. In addition the key residues compatible with the corresponding canonical fold as proposed by Morea *et al.* are shown in the bottom line. There is a perfect match for key residues 2, 25, 33 and 71 and to a certain degree for residue 29. Residue 30e of the dromedary VKappa is Glutamine in stead of Glycine, but Morea and colleagues (Morea et al., Methods 20:267-279 (2000)) suggested this residue to be perfectly compatible with the fold 3 for L1.

For the same somatically mutated dromedary VK family VK2-40 (Kp1, 3, 6, 7, 10, 20 and 48) with fold 3 for L1 (L1: 3) and fold 1 for L2 (L2: 1) the key residues 48 and 64 determining the canonical fold of L1 are shown together with these from the analogue human VKappa germline family 2. Here again the match is perfect.

The individual amino acid residues of the L1 and L2 loops of the somatically mutated dromedary VKappa segments along with the key residues were compared with those occurring in the human counterpart (VK family 2) that shares the same canonical fold combination (Table 6). For the somatically mutated dromedary VK family VK2-40 (Kpl, 3, 6, 7, 10, 20 and 48) with fold 3 for L1 (L1: 3) and fold 1 for L2 (L2: 1) the L1 and L2 residues were compared with those found in germline VK family 2 that has the identical canonical loop combination. The majority of the residues are shared between the dromedary somatically mutated VK and the human germline, such as Isoleucine on position 2, Serine on 25, 26 28 and 30b, Glutamine on 27, Tyrosine on 32, Leucine on 33 and Phenylalanine on 71. However, a few differ from the human analogue, i.e. Valine on 29 (although Leucine of human germline also occurs in the dromedary VK), Phenylalanine on 30 (again human residue Leucine is found as well in dromedary VK), Serine on 30a (human residue Glutamate occurs infrequently in dromedary VK), Serine on 30c, Asparagine on 30d, Glutamine on 30e, Lysine on 30f and finally Serine on 31.

Together with the key residues the residues of the L2 loop of the somatically mutated dromedary VKappa segments were compared those occurring in the human counterpart (VK family 2) that shares the same canonical fold combination (Table 6). Here again a perfect match was observed from residue Isoleucine on 48, Tyrosine on 49, Serine on 52 and Glycine on 64, while deviations were found on position 50 (Tyrosine in stead of Threonine from human VK family 2), and 51 (Alanine on 51 instead of Leucine).

### Overall conclusion

This analysis of the camelid VH and VL sequences demonstrates a very high homology if not identity to the human key residues defining the canonical folds as well as to the residues found in the hypervariable loops themselves. This suggests that the vast majority of the camelid immunoglobulin sequences will adopt the canonical folds as found in the human germlines, not only for the individual hypervariable loops but as well as for the combination of canonical folds found in human VH and VL.

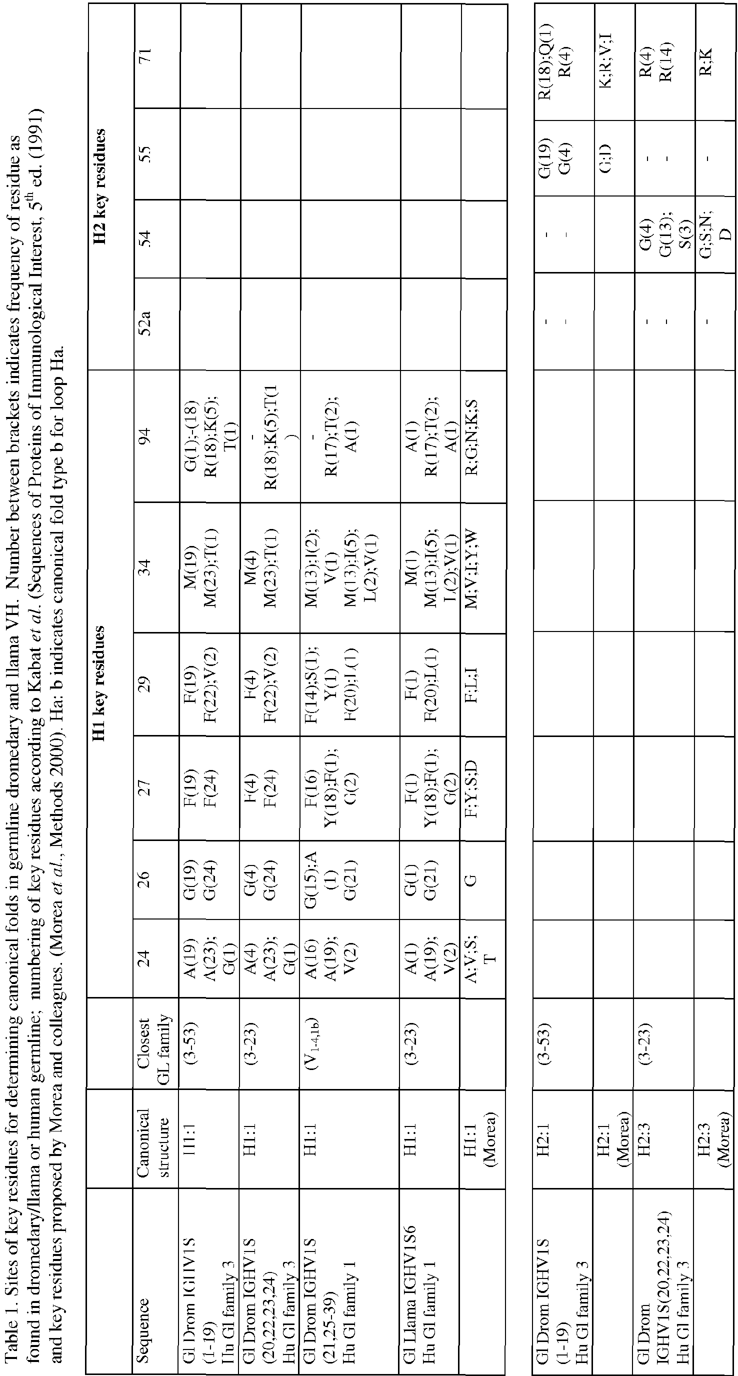

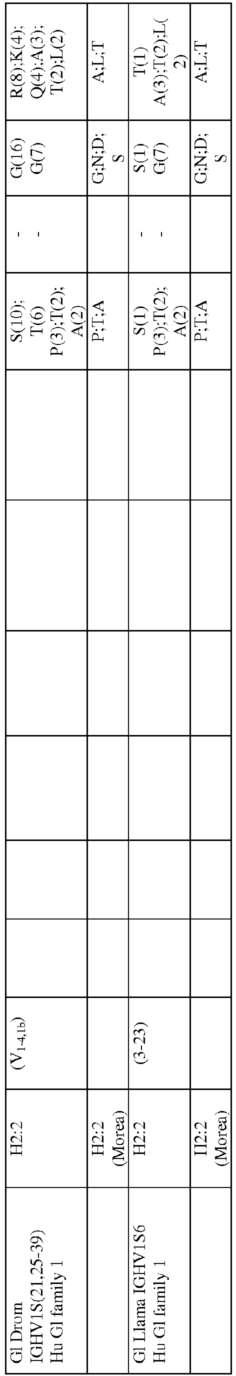

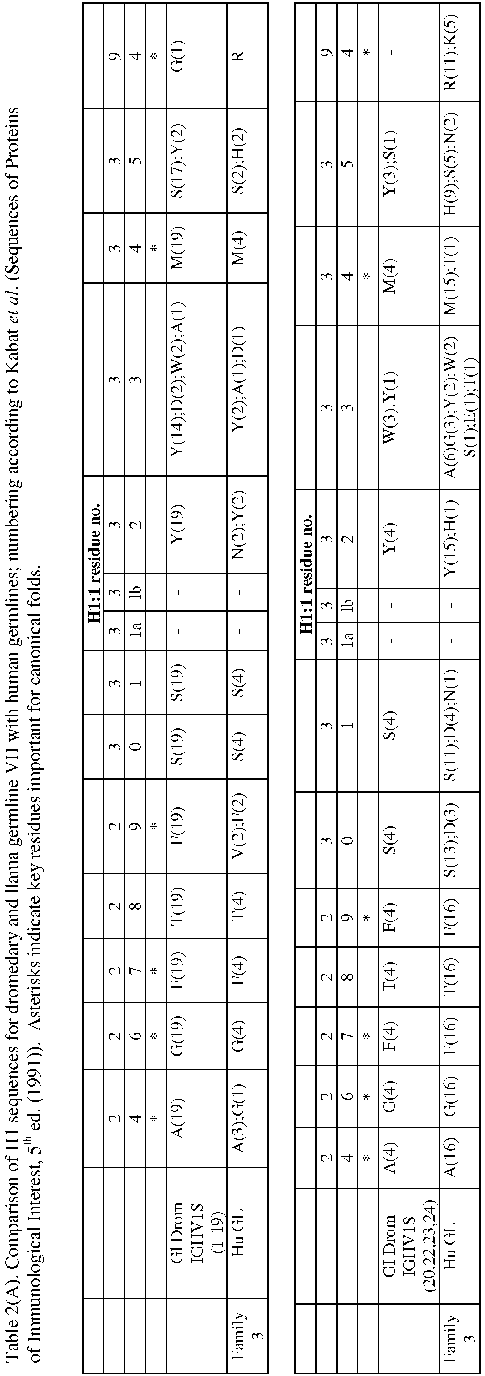

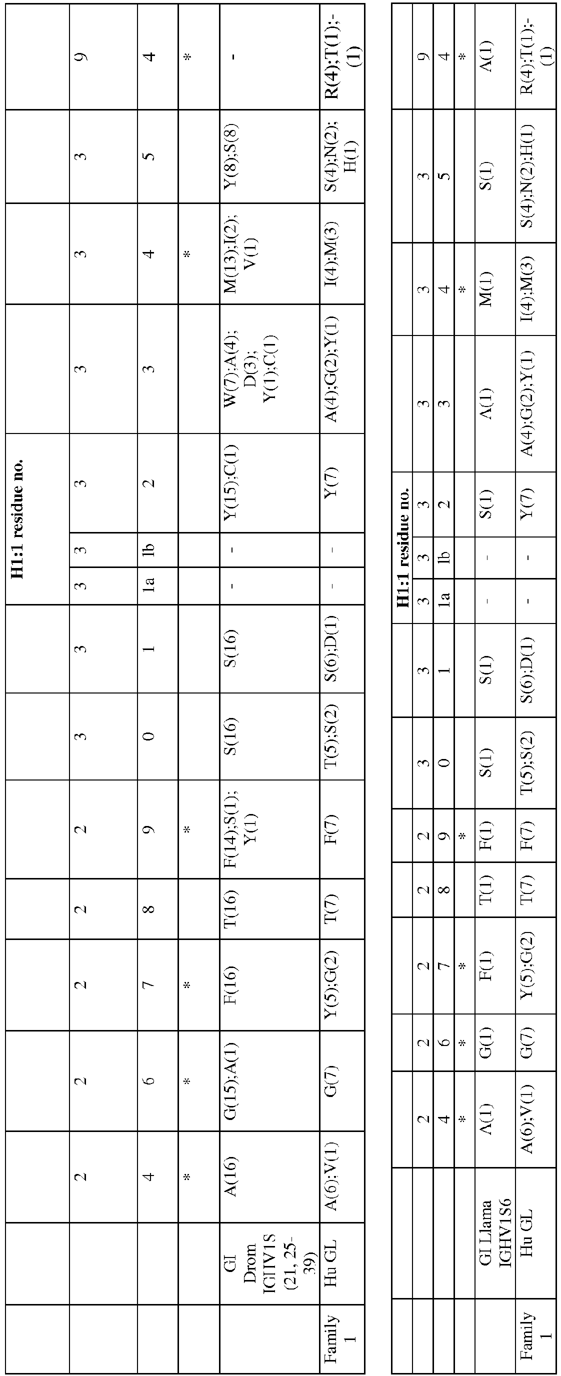

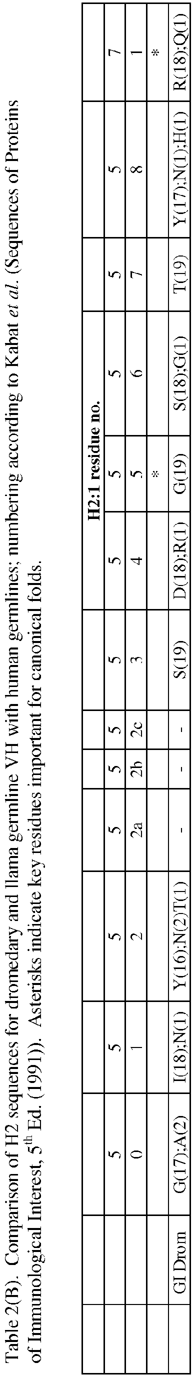

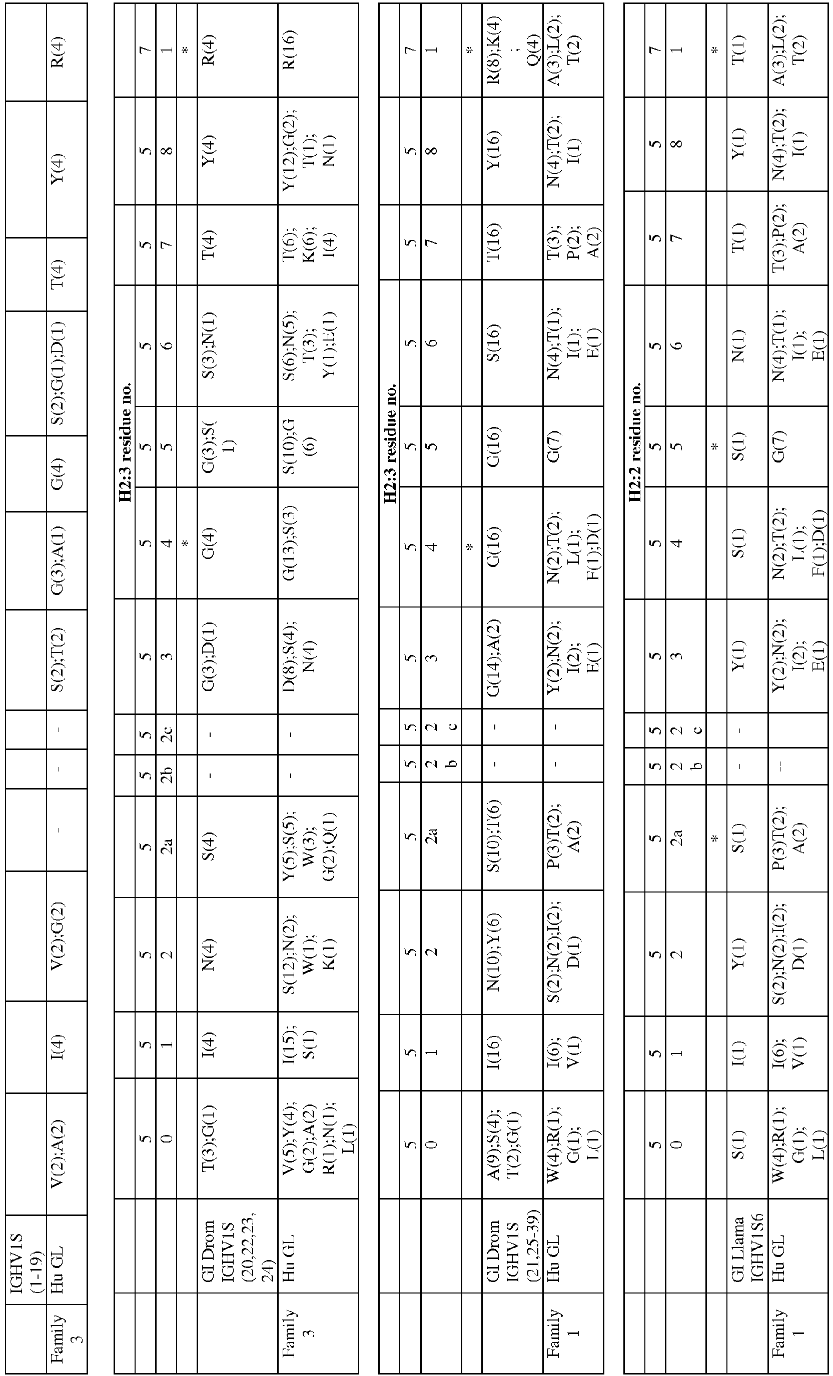

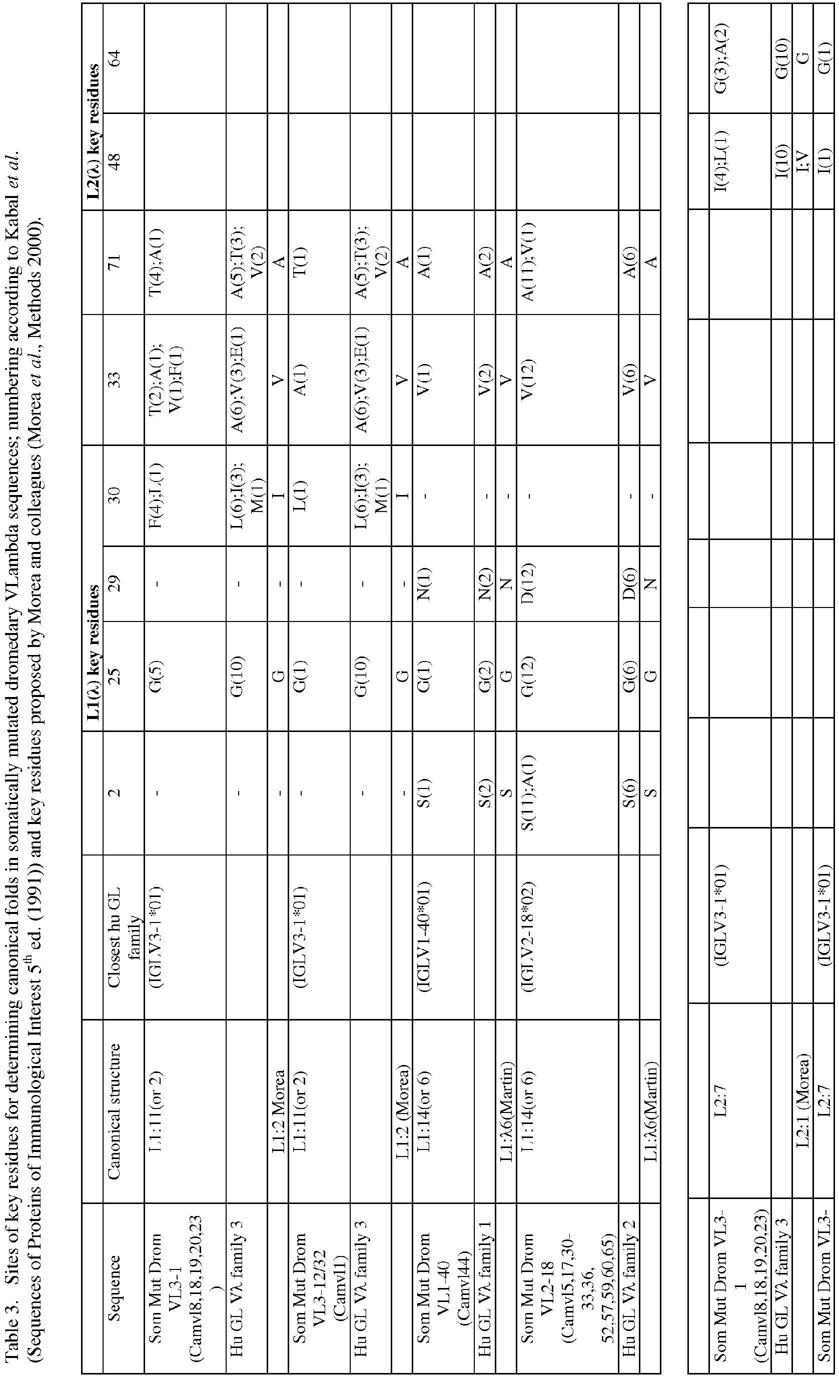

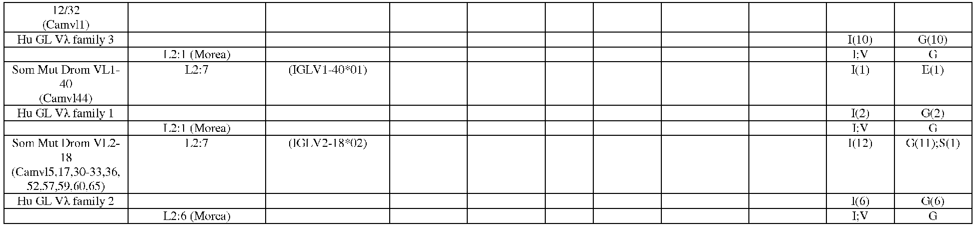

**Table 4(A). Comparison of L1 sequences of dromedary VLambda with human germlines; numbering according to Kabat et al. (Sequences of Proteins of Immunological Interest, 5^{th} ed. (1991)). Asterisks indicate key residues important for canonical folds.**

| | | | | | | **L1:11 residue no.** | | | | | | | | |
|---|---|---|---|---|---|---|---|---|---|---|---|---|---|---|
| | | 0 | 2 | 2 | 2 | 2 | 3 | 3 | 3 | 3 | 3 | 3 | 3 | 7 |
| | | 2 | 6 | 7 | 8 | 9 | 0 | 0a | 0b | 0c | 1 | 2 | 3 | 1 |
| | | | | | | | * | | | | | | * | * |
| | Som Mut Drom VL3-1(Camv18,18,19,20, 23) | - | - | G(4); D(1) | N(3);D(1); I(1) | - | F(4);L(1) | G(5) | S(4);D(1) | Y(4);K(1) | - | Y(4); K(1) | T(2); A(1); V(1); F(1) | A(5) |
| Family 3 | Hu GL Vλ family 3 | - | - | D(6); N(3); E(1) | N(3);A(3); S(2); K(1);V(1) | - | L(6);I(3); M(1) | G(5); P(3); R(1); E(1) | S(4);K(3); D(1); E(1);G(1) | K(6);Y(1); N(1); Q(1):S(1) | - | Y(7); A(1); S(1); N(1); | A(6); V(3); E(1) | A(3); T(3); V(2) |

| | | | | | | **L1:11 residue no.** | | | | | | | | |
|---|---|---|---|---|---|---|---|---|---|---|---|---|---|---|
| | | 0 | 2 | 2 | 2 | 2 | 3 | 3 | 3 | 3 | 3 | 3 | 3 | 7 |
| | | 2 | 6 | 7 | 8 | 9 | 0 | 0a | 0b | 0c | 1 | 2 | 3 | 1 |
| | | | | | | | * | | | | | | * | * |
| | Som Mut Drom VL3-12/32 | - | - | S(1) | L(1) | - | R(1) | N(1) | Y(1) | Y(19);C(1) | - | A(1) | A(1) | A(1) |
| | (Camvl1) | | | | | | | | | | | | | |
| Family 3 | Hu GL Vλ family 3 | - | - | D(6); N(3); E(1) | N(3);A(3); S(2); K(1);V(1) | - | L(6);I(3); M(1) | G(5); P(3); R(1); E1l) | S(4);K(3); D(1); E(1);G(1) | K(6);Y(1); N(1); Q(1);S(1) | - | Y(7); A(1); S(1); N(1) | A(6); V(3); E(1) | A(3); T(3); V(2) |

| | | | | | | **L1:14 residue no.** | | | | | | | | |
|---|---|---|---|---|---|---|---|---|---|---|---|---|---|---|
| | | 0 | 2 | 2 | 2 | 2 | 3 | 3 | 3 | 3 | 3 | 3 | 3 | 7 |
| | | 2 | 6 | 7 | 8 | 9 | 0 | 0a | 0b | 0c | 1 | 2 | 3 | 1 |
| | | * | | | | * | | | | | | | * | * |
| | Som Mut Drom VL1-40 (Camv144) | S(1) | S(1) | S(1) | S(1) | N(1) | I(1) | G(1) | G(1) | G(1) | S(1) | G(1) | V(1) | A(1) |
| Family 1 | Hu GL Vλ family 1 | S(2) | S(2) | S(2) | S(2) | N(2) | 1(2) | G(2) | A(2) | G(2) | Y(2) | D(1); V(1) | V(2) | A(2) |

| | | | | | | **L1:14 residue no.** | | | | | | | | |
|---|---|---|---|---|---|---|---|---|---|---|---|---|---|---|
| | | 0 | 2 | 2 | 2 | 2 | 3 | 3 | 3 | 3 | 3 | 3 | 3 | 7 |
| | | 2 | 6 | 7 | 8 | 9 | 0 | 0a | 0b | 0c | 1 | 2 | 3 | 1 |
| | | * | | | | * | | | | | | | * | * |
| | Som Mut Drom VL2-18 (Camv15,17,30-33,36,52,57,59,60,6 5) | S (11); A(1) | T(12) | S(10); R(2) | N(9);D(3) | D(11); G(1) | V(12) | G(12) | G(5);R(4); K(2); A(1) | Y(12) | N(11); A(1) | Y(12) | V(12) | A(11); V(1) |
| Family 2 | Hu GL Vλ family 2 | S(6) | T(6) | S(6) | S(6) | D(6) | V(6) | G(6) | G(3);S(2); D(1) | Y(6) | N(5); D(1) | Y(3); R(1); L(1); V(1) | V(6) | A(6) |

**Table 4(B). Comparison of L2 sequences of dromedary Vlambda with human germlines; numbering according to Kabat et al. (Sequences of Proteins of Immunological Interest, 5^{th} ed. (1991)). Asterisks indicate key residues important for canonical folds.**

| | | | | L2: 7 residue no. | | | | |
|---|---|---|---|---|---|---|---|---|
| | | 4 | 4 | 5 | 5 | 5 | 5 | 6 |
| | | 8 | 9 | 0 | 1 | 2 | 3 | 4 |
| | | * | | | | | | * |
| | Som Mut Drom VL3-1(Camv18,18,19,20,23) | I(4);L(1) | Y(5) | K(3);R(1);G(1) | D(4);N(1) | S(2);T(2);D(1) | A(1);L(1);E(1);N(1);S(1) | G(3);A(2) |
| Family | Hu GL Vλ family 3 | I(10) | Y(10) | E(2);K(2);D(1);Q(1);R(1);S(1);G(1);Y(1) | D(8);K(1);S(1) | S(9);N(1); | E(3);N(3);K(2);D(2) | G(10) |
| 3 | | | | | | | | |
| | | | | | | | | |

| | | | | L2: 7 residue no. | | | | |
|---|---|---|---|---|---|---|---|---|
| | | 4 | 4 | 5 | 5 | 5 | 5 | 6 |
| | | 8 | 9 | 0 | 1 | 2 | 3 | 4 |
| | | * | | | | | | * |
| | Som Mut Drom VL3-12/32 (Camvll) | I(1) | Y(1) | N(1) | D(1) | N(1) | N(1) | G(1) |
| Family 3 | Hu GL Vλ family 3 | I(10) | Y(10) | E(2);K(2);D(1);Q(1);R(1);S(1);G(1);Y(1) | D(8);K(1);S(1) | S(9);N(1) | E(3);N(3);K(2);D(2) | G(10) |
| | | | | | | | | |

| | | | | L2: 7 residue no. | | | | |
|---|---|---|---|---|---|---|---|---|
| | | 4 | 4 | 5 | 5 | 5 | 5 | 6 |
| | | 8 | 9 | 0 | 1 | 2 | 3 | 4 |
| | | * | | | | | | * |
| | Som Mut Drom VL1-40 (Camv144) | I(1) | Y(1) | G(1) | N(1) | S(1) | N(1) | E(1) |
| Family 1 | Hu GL Vλ family 1 | I(2) | Y(2) | G(2) | N(2) | S(2) | N(2) | G(2) |
| | | | | | | | | |

| | | | | L2: 7 residue no. | | | | |
|---|---|---|---|---|---|---|---|---|
| | | 4 | 4 | 5 | 5 | 5 | 5 | 6 |
| | | 8 | 9 | 0 | 1 | 2 | 3 | 4 |
| | | * | | | | | | * |
| | Som Mut Drom VL2-18 (Camvl5,17,30-33,36,52,57,59,60,65) | I(12) | Y(12) | Q(11);D(1) | V(9);I(2);D(1) | N(10);S(1);D(1) | K(12) | G(11);S(1) |
| Family 2 | Hu GL Vλ family 2 | I(6) | Y(6) | E(4);D(1);N(1) | V(6) | S(5);N(1) | K(3);N(2);T(1) | G(6) |

**Table 5. Sites of key residues for determining canonical folds in somatically mutated dromedary Vkappa sequences; numbering according to Kabat et al. (Sequences of Proteins of Immunological Interest, 5^{th} ed. (1991)) and key residues proposed by Morea and colleagues (Morea et al., Methods 2000).**

| | | | **L1(κ) key residues** | | | | | | **L2(κ) key residues** | |
|---|---|---|---|---|---|---|---|---|---|---|
| Sequence | Canonical structure | Closest hu GL family | 2 | 25 | 29 | 30e | 33 | 71 | 48 | 64 |
| Som Mut Drom vK2-40 (Kp1,3,6,7,10,20,48) | K1:3 | IGKV2-40 (2_1 or 011*/01) | I(7) | S(7) | V(6);L(1) | Q(7) | L(7) | F(7) | | |
| Hu GL Vκ family 2 | | | I(1) | S(1) | L(1) | G(1) | L(1) | F(1) | | |
| | | | | | | | | | | |
| | K1: 3 (Morea) | | I | S | L;V | E;Q;S | L | F | | |
| Som Mut Drom vK2-40 (Kp1,3,6,7,10,20,48) | K2: 1 | IGKV2-40 (2_1 or 011*/01) | | | | | | | I(7) | G(7) |
| Hu GL Vκ family 2 | | | | | | | | | I(1) | G(1) |
| | H2: 1 (Morea) | | | | | | | | I;V | G |

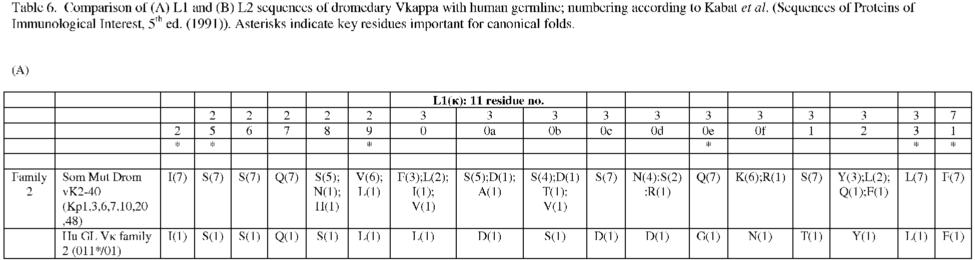

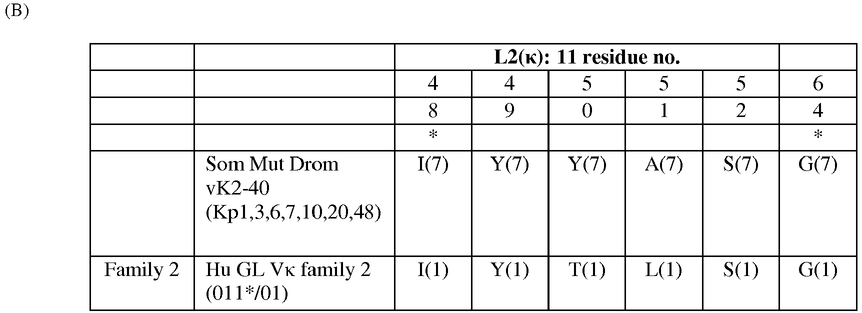

### Example 14 - Sequence analysis of somatically mutated VH, VK and VL from Ilama

From four Ilamas peripheral blood lymphocytes were isolated, RNA extracted and random primed cDNA synthesized as described before (de Haard et al, JBC 1999). Amplification of VHCH1, VLCL and VKCK was performed and the amplicons were cloned in vector pCB3 yielding heavy chain or light chain libraries. After screening of clones with PCR to check for the presence of the antibody domain insert, individual clones were grown and plasmid DNA was purified for sequence analysis.

Section 14.A shows the lambda light chain variable regions grouped into families according to the closest human germline analogue with the same CDR1 and CDR2 length and presumable having the same canonical fold combination. The lambda germlines most frequently used in humans, i.e. VL1, VL2 and VL3, are also often found in the analyzed Ilama sequences and in addition VL4, VL5, VL6 (not shown in section 14.A) and VL8, meaning that 7 out of the 10 lambda families as found in humans are used as well in the Ilama. Section 14.B shows two of the three kappa light chain variable regions(i.e. VK1, and VK4; VK2 is not shown), which occur in about 50% of kappa containing human antibodies. Members of the VK3 family, which is used most frequently (50%) in human antibodies, were not identified, but it can well be that the used primers for amplification are responsible for this and that these have to be adapted. Section 14.C shows the alignment of the VH sequences revealing a high sequence homology to the most often used human VH3 segment (occurs 34% in human antibodies) and VH1 (17%). It must be noted that the recent publication of Achour et al (J Immunol 2008) mentions the presence of a VH2 family in the germline of Alpaca which is most closely related to the human VH 4 family (see example 10.10).

Overall it can be concluded that camelids use a high diversity of heavy chain and light chain families similar to what is found in the human immune system, meaning that by active immunization with human disease targets an excellent choice of lead antibodies can be expected with a high sequence homology to human antibodies, which therefore can be easily engineered for therapeutic applications.

### 14. (A) VLAMBDA

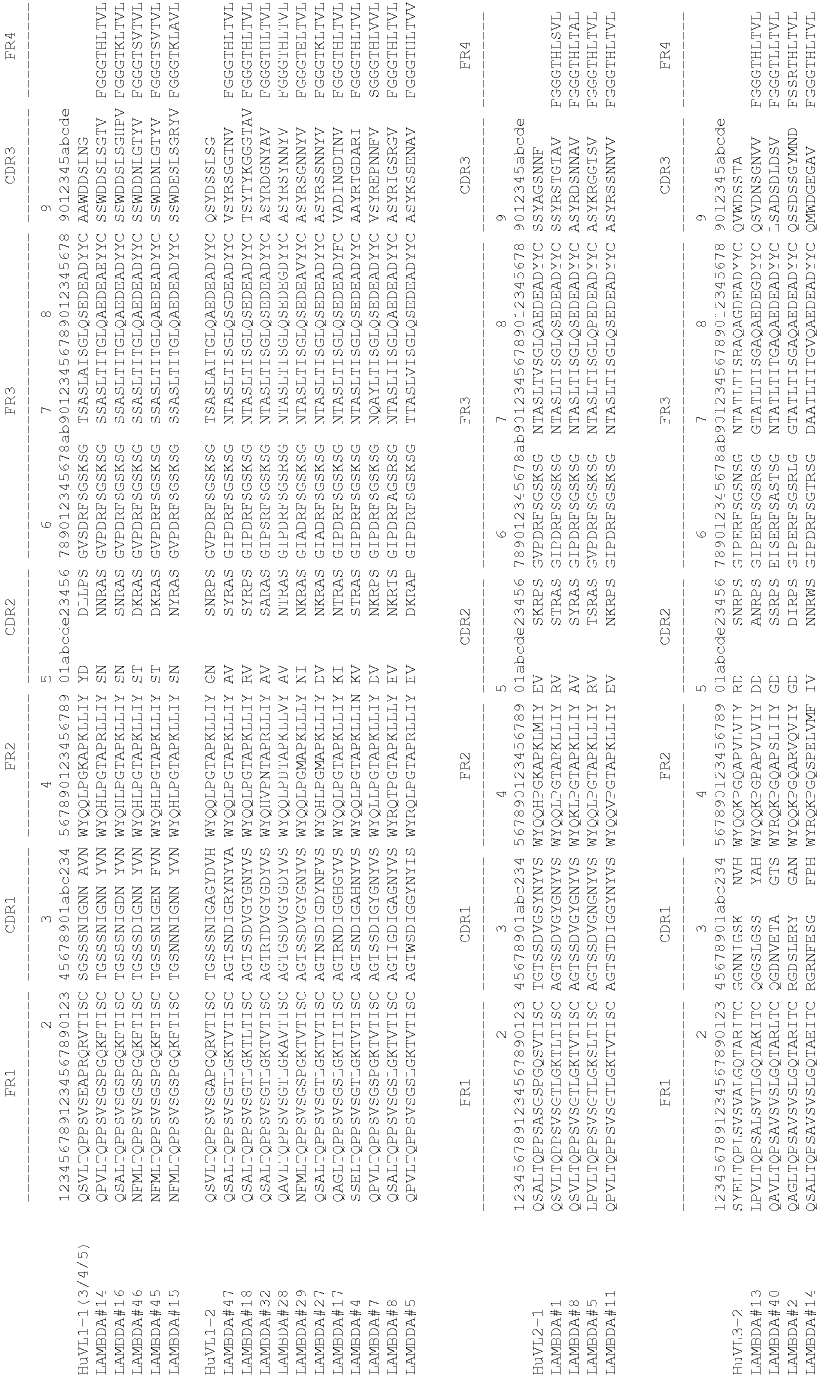

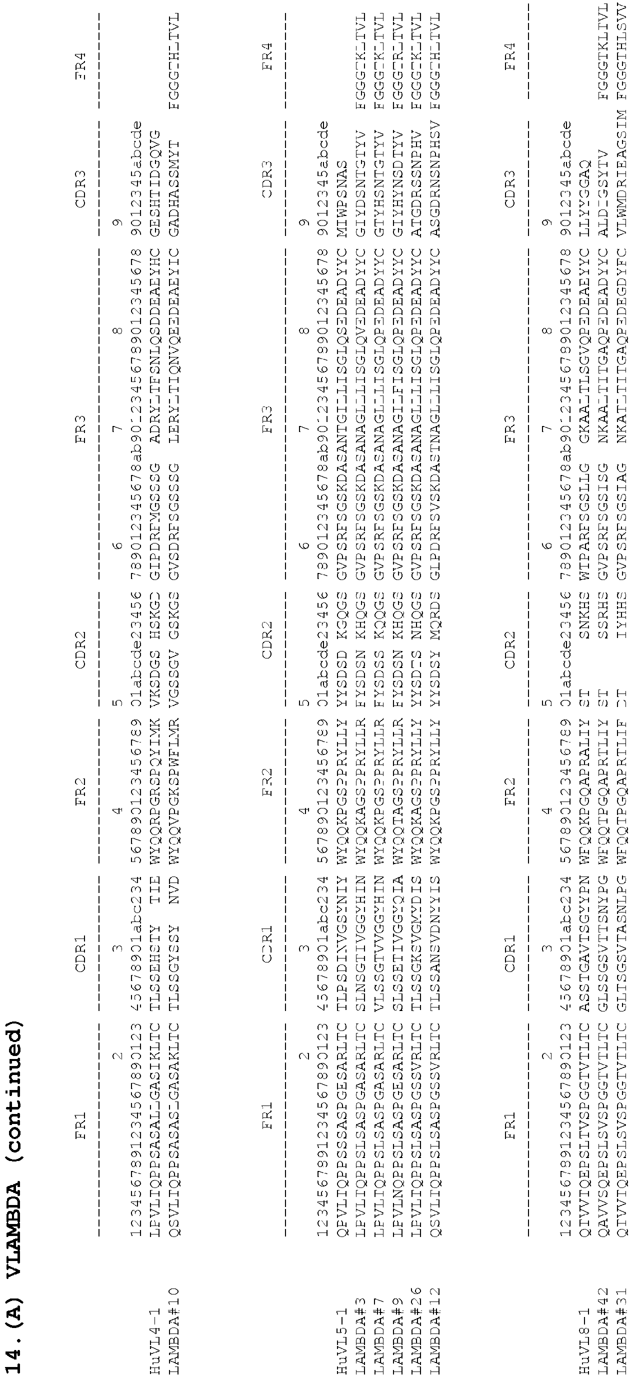

### 14.(B) VKAPPA

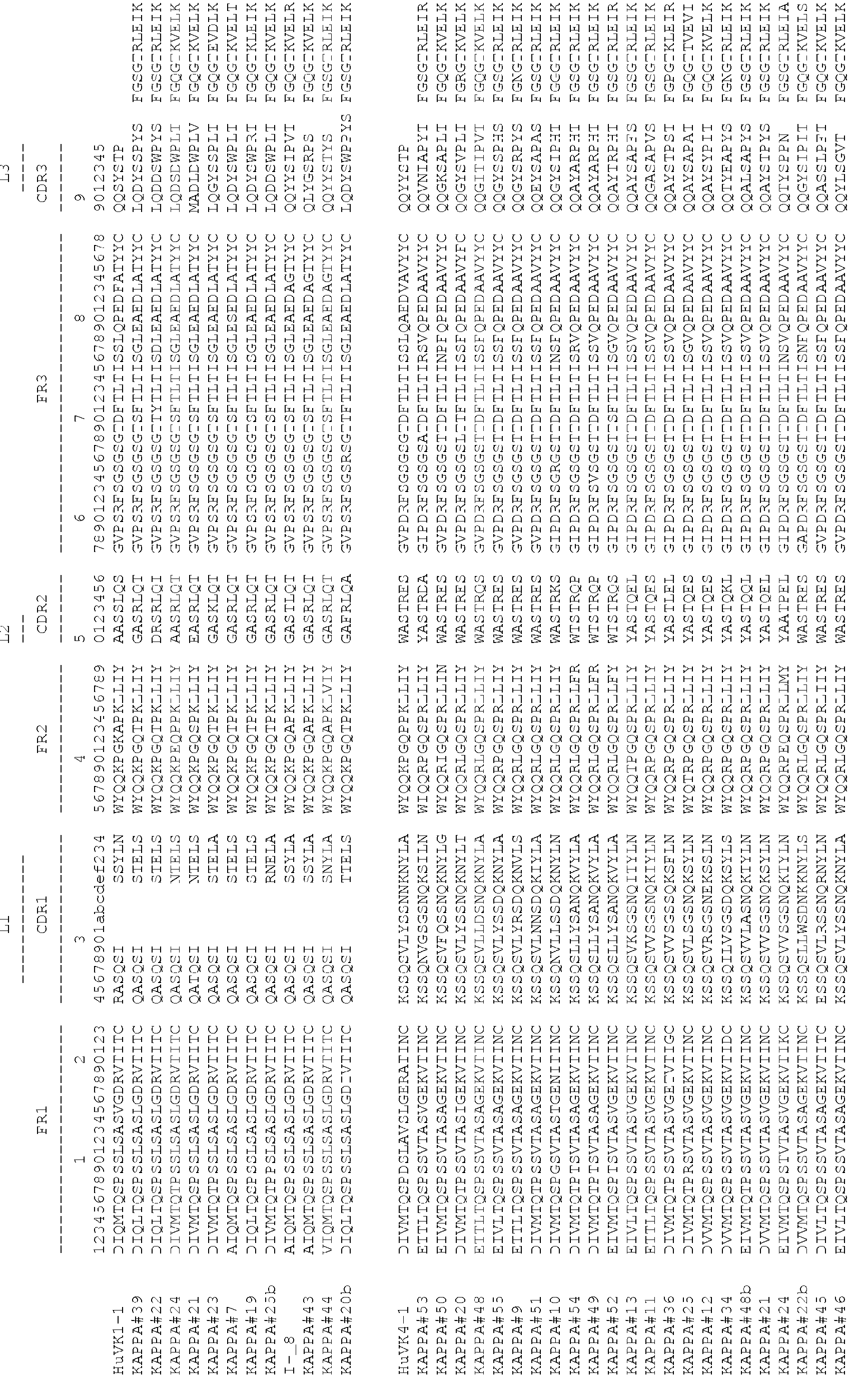

### 14. (C) VH

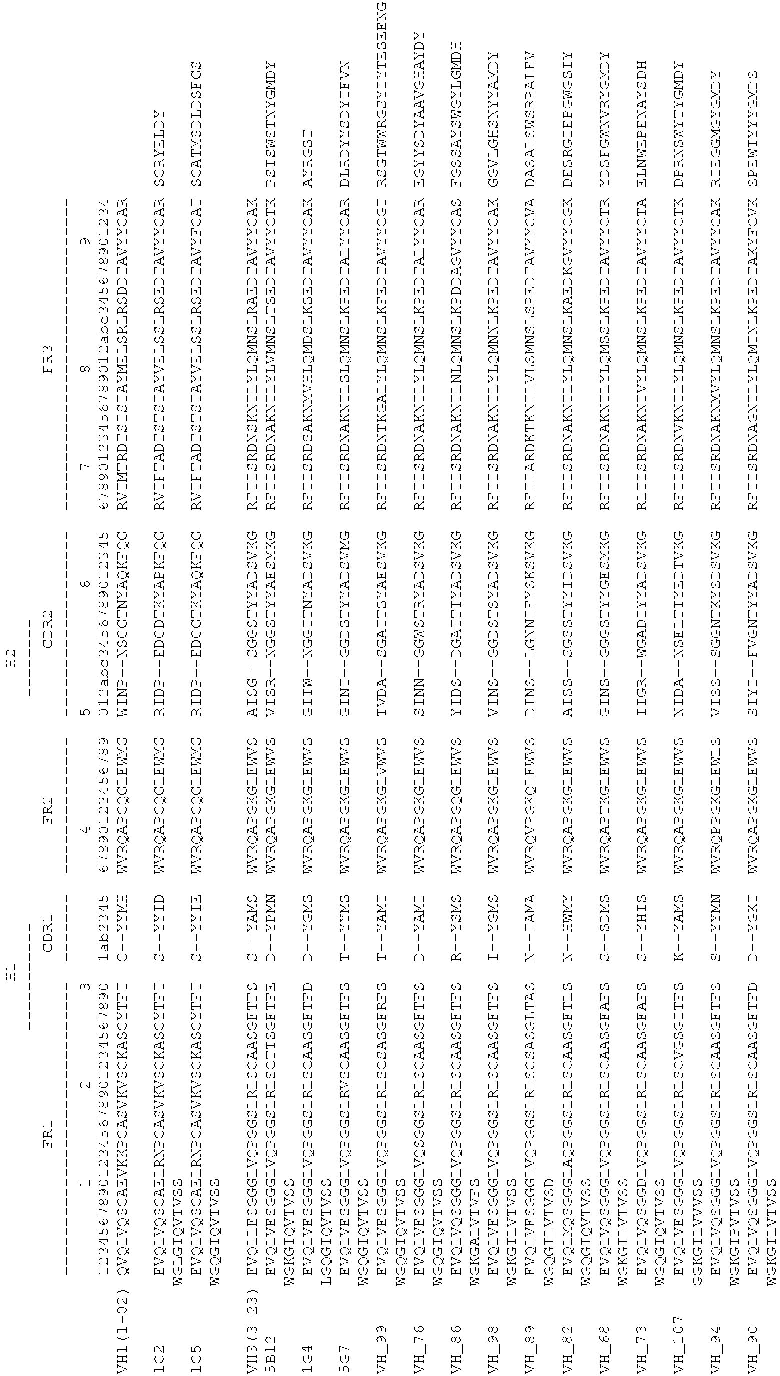

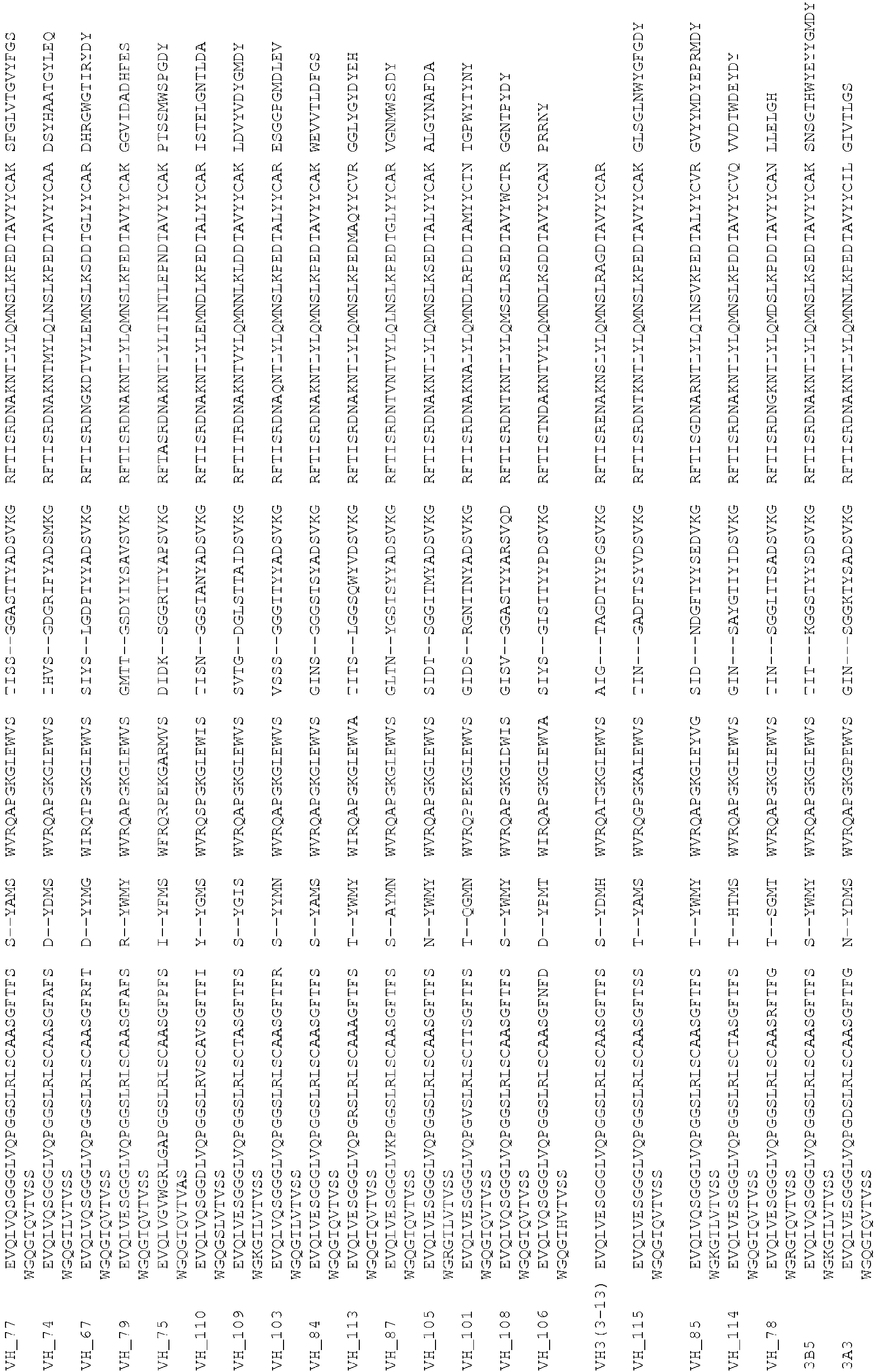

### Example 15 - Generating Fabs against IL-1 Beta

Unless otherwise indicated, the materials and protocols used in the following study were analogous to those used in examples 1-9.

### LIamas were successfully immunized with IL-1 Beta

Two Ilamas (*Lama glama*) were immunized with human IL-1 Beta according to a standard protocol (as described in Example 1).

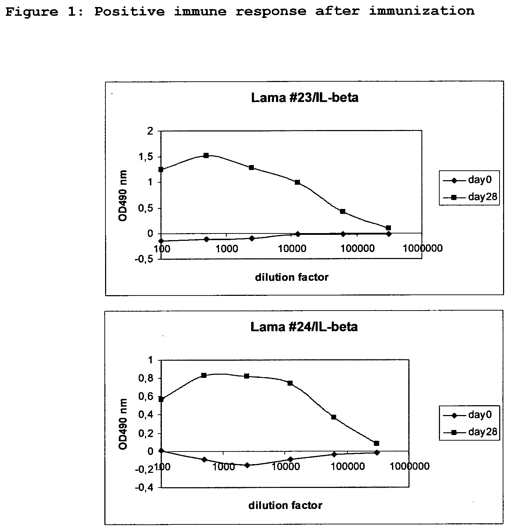

Sera from both Ilamas were tested for the presence of antibodies against IL-1 Beta by ELISA prior (day 0) and after immunization (day 28). As shown in Figure 1, a specific signal against IL-1 Beta was observed in ELISA after immunization, even after 10,000 fold dilution of the serum. This high antibody titer indicates a specific and appropriate immune response.

### Fab Libraries with good diversity were constructed

PBLs isolated from both immunized Ilamas were used for RNA extraction, RT-PCR and PCR-cloning of Fab in a phagemid using the strategy described by de Haard et al (JBC 1999), to obtain a diverse library of good diversity (2-5x10⁸).

The following primers were used:

| **Primers for cloning of lambda light chain** | |
|---|---|
| Name | Sequence |
| HuVl1A-BACK-ApaLI | GCC TCC ACC AGT GCA CAGTCTGTGYTGACKCAGCC |
| HuVl1 B-BACK-ApaLI | GCC TCC ACC AGT GCA CAGTCTGTGYTGACGCAGCC |
| HuVl1C-BACK-ApaLI | GCC TCC ACC AGT GCA CAGTCTGTCGTGACGCAGCC |
| HuVl2-BACK-ApaLI | GCC TCC ACC AGT GCA CAGTCTGCCCTGACTCAGCC |
| HuVl3A-BACK-ApaLI | GCC TCC ACC AGT GCA CTT TCCTATGAGCTGACWCAGCC |
| HuVl3B-BACK-ApaLI | GCC TCC ACC AGT GCA CTT TCTTCTGAGCTGACTCAGGA |
| HuVl4-BACK-ApaLI | GCC TCC ACC AGT GCA CAGCYTGTGCTGACTCAATC |
| HuVl5-BACK-ApaLI | GCC TCC ACC AGT GCA CAGGCTGTGCTGACTCAGCC |
| HuVl6-BACK-ApaLI | GCC TCC ACC AGT GCA CTT AATTTTATGCTGACTCAGCC |
| HuVl7/8-BACK-ApaLI | GCC TCC ACC AGT GCA CAGRCTGTGGTGACYCAGGA |
| HuVl9-BACK-ApaLI | GCC TCC ACC AGT GCA CWGCCTGTGCTGACTCAGCC |
| HuVI10-BACK-ApaLI | GCC TCC ACC AGT GCA CAGGCAGGGCTGACTCAGCC |
| caClambda1-FOR | CTAACACTGGGAGGGGGACACCGTCTTCTC |
| caClambda2-FOR | CTAACACTGGGAGGGNCTCACNGTCTTCTC |

| **Primers for cloning of kappa light chain** | |
|---|---|
| Name | Sequence |
| HuVk1B-BACK-ApaLI | GCC TCC ACC AGT GCA CTT GACATCCAGWTGACCCAGTCTCC |
| HuVk2-BACK-ApaLI | GCC TCC ACC AGT GCA CTT GATGTTGTGATGACTCAGTCTCC |
| HuVk3B-BACK-ApaLI | GCC TCC ACC AGT GCA CTT GAAATTGTGWTGACRCAGTCTCC |
| HuVk2/4-BACK-ApaLI | GCC TCC ACC AGT GCA CTT GAYATYGTGATGACCCAGWCTCC |
| HuVk5-BACK-ApaLI | GCC TCC ACC AGT GCA CTT GAAACGACACTCACGCAGTCTCC |
| HuVk6-BACK-ApaLI | GCC TCC ACC AGT GCA CTT GAAATTGTGCTGACTCAGTCTCC |
| HuVk4B-BACK-ApaLI | GCC TCC ACC AGT GCA CTT GATATTGTGATGACCCAGACTCC |
| caCHkapFOR-AscI | GCC TCC ACC GGG CGC GCC TTA TTAGCAGTGTCTCCGGTCGAAGCTCCT |
| caCHkap2FOR-AscI | GCC TCC ACC GGG CGC GCC TTA TTARCARTGYCTNCGRTCRAA |
| | |

| **Non-tagged primers for cloning of Heavy chain (step 1)** | |
|---|---|
| Name | Sequence |
| VH1a-BACK | CAGGTKCAGCTGGTGCAGTCTGG |
| VH5a-BACK | GARGTGCAGCTGGTGCAGTCTGG |
| VH4a-BACK | CAGSTGCAGCTGCAGGAGTCTGG |
| VH4b-BACK | CAGGTGCAGCTACAGCAGTCTGG |
| VH2b-BACK | CAGGTCACCTTGARGGAGTCTGG |
| | |

| **Tagged primers for cloning of Heavy chain (step 2)** | |
|---|---|
| Name | Sequence |
| VH1a-BACK-SfiI | CTC GCA ACT GCG GCC CAG CCG GCC ATG GCCCAGGTKCAGCTGGTGCAGTCTGG |
| VH5a-BACK-SfiI | CTC GCA ACT GCG GCC CAG CCG GCC ATG GCCGARGTGCAGCTGGTGCAGTCTGG |
| VH4a-BACK-SfiI | CTC GCA ACT GCG GCC CAG CCG GCC ATG GCCCAGSTGCAGCTGCAGGAGTCTGG |
| VH4b-BACK-SfiI | CTC GCA ACT GCG GCC CAG CCG GCC ATG GCCCAGGTGCAGCTACAGCAGTCTGG |
| VH2b-BACK-SfiI | CTC GCA ACT GCG GCC CAG CCG GCC ATG GCCCAGGTCACCTTGARGGAGTCTGG |

Independent VλCλ and VκCκ libraries were constructed using a single (tagged)-PCR step (30 cycles) to conserve a greater clonal diversity.

The VHCH1 libraries were built in parallel using a two step PCR (25 cycles with non tagged primers (step 1) followed by 10 cycles of tagged primers (step 2)).

Next, the light chain from the VλCλ and VκCκ libraries are re-cloned separately in the VHCH1-expressing vector to create the "Lambda" and "Kappa" Ilama Fab-library respectively (two for each immunized Ilama). Quality control of the libraries was routinely performed using PCR.

Up to 93% of the clones tested randomly contained full length Fab sequences, indicating a high quality of the libraries.

### Human IL-1 Beta specific Fabs were selected

Phage display was used to identify a large diversity of Ilama Fabs binding to biotinylated IL-1 Beta. Biotinylated IL-1 Beta was used for capturing to conserve the active conformation of the protein. After two rounds of selection, a good enrichment compared to control was observed. Phage ELISA revealed presence of clones expressing cytokine specific Fabs (data not shown).

The phage binding to biotinylated IL-1 Beta were eluted by pH shock. Sequential dilutions of the output (10⁻¹ to 10⁻⁵) were used to infect fresh *E. coli TG1* cells. The number of colonies obtained indicate the number of phage bound during the selection. In the example above, 5µl of output gave around 10⁵ phage when selection was done with 100nM and 1nM of biot-IL-1 Beta. Compared to the 10² phages obtained by non-specific binding, this gives a 1000 fold enrichment.

94 Single clones were grown and used to produce monoclonal phage. These phage were used in a phage ELISA. Many phage showed good binding to biot-IL-1 Beta after two rounds of selection on biotinylated IL-1 Beta.

### Human IL-1 Beta specific Fabs have high starting homology to human germline

Target specific VH and Vλ domains were matched with those common human germlines showing an identical CDR1 and CDR2 length and corresponding canonical folds. Subsequently the closest human germline was selected based on sequence homology in their framework regions. Non-matching amino acid residues were checked for their presence in other, related human germlines. In case there was no match, these residues were counted as foreign.

**Table 7: Overall sequence homology of llama VH to human germline**

| **Closest Human Germline** | **Matching Clones** | **% Sequence Homology** |
|---|---|---|
| IGHV1-2 | 1C2/2B7/2C12 | 93 |
| | 2D8 | 94 |
| | 1G5/2D7 | 93 |
| | 2E12/2G7 | 94 |
| IGHV3-23 | 1F2 | 98 |
| | 1G4 | 92 |
| | 5G7 | 95 |
| | 5B12 | 92 |
| | 1A1/2B8/2B9 | 98 |
| | 1C3/1E3/2A7 | 98 |
| IGHV3-13 | 1E2 | 94 |
| | 3A3/3B6/3E2/3E3 | 95 |
| | 3B5/4F1 | 98 |
| IGHV3-20 | 4H1 | 94 |
| | 4H4 | 93 |

**Table 8: Overall sequence homology of llama VL to human germline**

| **Closest Human Germline** | **Matching Clones** | **% Sequence Homology** |
|---|---|---|
| IGLV8-61 | 1E2 | 90 |
| | 1F2 | 90 |
| | 3E2/3E3/3A3 | 86 |
| | 3B5/4F1 | 86 |
| IGLV2-18 | 3B6 | 91 |
| IGLV5-52 | 1G4 VL | 96 |
| IGLV3-19 | 1C2/2B7/2D7 | 95 |
| | 2E12/2G7 | 96 |
| | 2D8 | 95 |

### Discussion and conclusions:

- A total of 14 target specific VH families, 9 target specific Vλ families and 3 V_{K} families were identified based on this very first selection
- This initial panel of 14 anti-IL-1 Beta WT VH's and 12 anti-IL-1 Beta WT VL's shows a remarkably high sequence homology to the human germline.
- 33% of those VH domains have a starting homology of 95% or more to the human situation and about 44% of the VL domains have a starting homology of 95% or more to the human situation, eliminating the need for further humanization.
- VH domain 2D8 is a humanized version of VH 1 C2 because it has one deviating amino acid residue less as compared to the closest human germline. Its corresponding VL domain (VL 2D8), had a starting homology of 95% which was further increased to 96% by 1 back mutation (VL 2G7) to the closest human germline.
- All VH and VL domains, without a single exception, exhibited human 3-D binding site structures (i.e. identical combinations of canonical folds for CDR1 and CDR2 as occurring in the matching human germline segments) when assessed using the methodology described above (data not shown).

### Humanization of Fabs 1E2 and 1F2

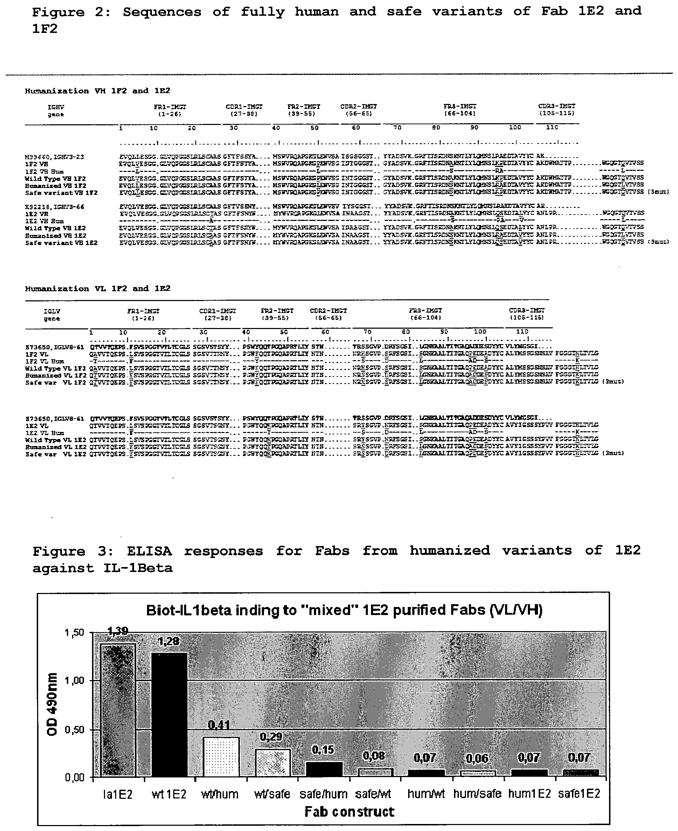

Humanization was performed on two IL-1 Beta specific Fabs coded 1 E2 and 1 F2. Based on the alignment against the closest human germlines, mutations in their VH and Vλ framework regions were proposed (Figure 2). The germlining of VH matching to the human VH3 family will often involve a number of residues, which already deviate in publically known *Lama glama, Lama pacos* or *Camelus dromedarius* derived germline sequences. For instance, Alanine on position 71 (numbering according to Kabat) and Lysine on 83 and Proline on 84 might be changed into Serine (although Alanine exists in certain human germline VH3 members), Arginine (although Lysine is used by a number of human VH3 germlines) and Alanine, respectively. For light chain variable sequences no germline sequences are available for Camelids, but it is very likely that a number of deviations in FRs from human germline exists that will be changed in the majority of lead antibodies. Besides the fully humanized (hum) and the wild type (wt) V regions, also a "safe variant" with only three wild type residues remaining was proposed (safe).

Fab 1 E2 was formatted in a step-by-step approach, whereby the different versions (wt, safe and fully humanized) of the Vλ fused to the human constant domain were combined with various versions of the VH fused to the human constant CH1 domain to generate the Fabs indicated in Table 9.

**Table 9: Fab 1E2 formats**

| | **VH** | **VH 1E2 + hum CH1** | | |
|---|---|---|---|---|
| | **wt** | **wt** | **safe** | **hum** |
| **VL 1E2 + humCL** | **wt** | wt 1E2 | wt/safe | wt/hum |
| | **safe** | safe/wt | safe 1E2 | safe/hum |
| | **hum** | hum/wt | hum/safe | hum 1E2 |

The genes of these Fabs were ordered as synthetic genes with GeneArt (Germany) and were subsequently produced in *E. coli,* purified and tested for their ability to bind biot-IL-1 Beta. For this the Fabs were captured on an anti-myc coated Maxisorp plate.

Biotinylated human IL-1 Beta was added and bound cytokine was detected using HRP-conjugated streptavidin. The read out of this assay is represented in Figure 3 below.
- The replacement of the wild type constant domains CH1 and Cλ by their human counterpart did not affect the binding capacity.
- Partial (wt Vλ/safe VH) and complete (wt Vλ/hum VH) humanization of the VH domain of 1 E2 generated a functional Fab.

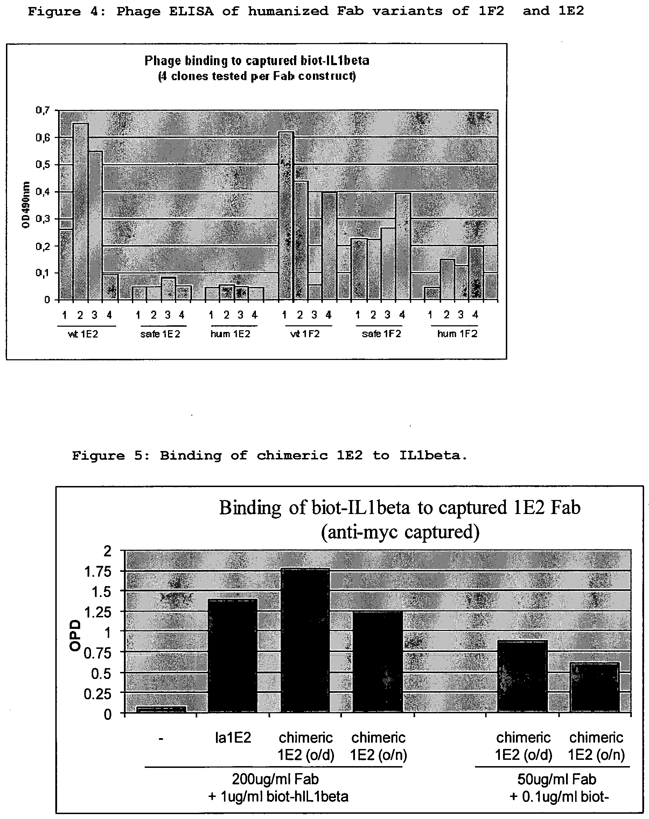

The humanized variants of clone 1 F2 were tested with phage expressing gene3-Fabs fusions (Figure 4). Phage were produced from 4 independent clones for each construct:
- wt 1F2 and wt 1 E2 (llama Vλ and VH fused to human Cλ and CH1)
- safe variant 1 F2 and safe variant 1 E2 (partially humanized Vλ fused to human Cλ and CH1)
- hum 1 F2 and hum 1 E2 (fully humanized Vλ and VH fused to human Cλ and CH1) Four clones for each Fab were tested to overcome clonal variation (due to bacterial growth, phage production efficiency and toxicity etc...). Phage ELISA was performed by capturing of biotinylated IL-1 Beta on neutravidin coated Maxisorp plate and subsequent incubation of crude phage extract (i.e. bacterial medium). After extensive washing, bound phages were detected with an anti-M13-HRP monoclonal antibody. The same phage preparations when tested on neutravidin coated wells (without biotinylated IL-1 Beta) did not give signals (data not shown).
- Back mutations in the framework regions of 1 F2 VL and VH domains to the closest human germline successfully yield partially (safe) and fully (hum) humanized variants, maintaining antigen specificity.

### Successful formatting of camelid variable domains with human constant domains

The VL and VH variable domains of the IL-1 Beta specific clone 1 E2 were successfully fused to the human Cλ and CH1 constant domains, resulting in a "chimeric" Fab which was produced and purified.

This chimeric 1 E2 Fab was produced by performing the induction for 4h at 37°C (o/d) or for 16h and 28°C (o/n) from the pCB5 phagemid (Δgene3). The wild type Ilama 1 E2 Fab was produced by performing induction for 16h at 30°C from pCB3 (gene3 containing phagemid). After purification, these Fabs were loaded on SDS-PAGE with (reducing) or without (non-reducing) DTT. Coomassie staining was performed, nicely showing the presence of these Fabs or their composing light and heavy chains at the expected molecular weight band (not shown).

The purified Ilama and chimeric 1 E2 Fabs described above were captured on anti-myc coated maxisorp plates. After incubation with biotinylated human IL-1 Beta and extensive washing, the biotinylated IL-1 Beta bound to the Fab was detected using HRP-conjugated streptavidin. Both the purified Ilama and chimeric 1 E2 Fabs exhibited functional target binding (Figure 5). This finding demonstrates the feasibility to associate *Camelid* derived variable domains with the constant domains of human IgGs

### A subset of Fabs showed functional inhibition of the target

The table below shows the OD values resulting from the following ELISA experiment. Wells were coated with a mouse monoclonal antibody known to inhibit the binding of IL1-Beta with its receptor (provided by Diaclone SAS).

Biotinylated IL-1 Beta was added to the wells together with periplasmic extracts of Fabs identified after 2 rounds of selection against biotinylated IL-1 Beta. Detection of the bound biotinylated IL-1 Beta happened through HRP labeled streptavidin. A reduced signal indicated the competition of a specific Fab with the blocking mouse monoclonal antibody, suggesting antagonism.

A positive control was included in well G12 by spiking in a large amount of the competing mouse monoclonal (well 12G in Table 10).

**Table 10: Results of competition assay**

| | 6A | | | | | | 6C | | | | | |
|---|---|---|---|---|---|---|---|---|---|---|---|---|
| | 1 | 2 | 3 | 4 | 5 | 6 | 7 | 8 | 9 | 10 | 11 | 12 |
| A | 0.205 | 0.621 | 0.670 | 0.691 | 0.704 | 0.823 | 0.353 | 0.737 | 0.726 | 0.544 | 0.551 | 0.626 |
| B | 0.668 | 0.546 | 0.444 | 0.632 | 0.614 | 0.627 | 0.442 | 0.641 | 0.558 | 0.693 | 0.565 | 0.548 |
| C | 0.756 | 0.541 | 0.386 | 0.572 | 0.763 | 0.593 | 0.517 | 0.537 | 0.518 | 0.588 | 0.473 | 0.435 |
| D | 0.521 | 0.790 | 0.203 | 0.792 | 0.801 | 0.263 | 0.619 | 0.483 | 0.631 | 0.592 | 0.455 | 0.603 |
| E | 0.798 | 0.545 | 0.359 | 0.528 | 0.281 | 0.655 | 0.618 | 0.516 | 0.651 | 0.493 | 0.575 | 0.367 |
| F | 0.747 | 0.611 | 0.520 | 0.599 | 0.203 | 0.204 | 0.605 | 0.373 | 0.550 | 0.516 | 0.602 | 0.392 |
| G | 0.498 | 0.477 | 0.539 | 0.693 | 0.663 | 0.197 | 0.554 | 0.589 | 0.807 | 0.528 | 0.634 | 0.063 |
| H | 0.628 | 0.493 | 0.552 | 0.900 | 0.858 | 0.635 | 0.170 | 0.574 | 0.559 | 0.598 | 0.593 | 0.378 |

A number of Fabs were identified which successfully compete with the blocking mouse monoclonal antibody (indicated by underlined cells in Table 10). Sequence analysis of the competing clones revealed the presence of three Fabs with different VH, which were present in 48 screened clones (part of the plate coded 6A in Table 10). The sequence alignments against the closest human germline and the structural homology analysis of the VH of the antagonistic Fab 1A1 (giving a signal of 0.205 in the competition assay of Table 10), 1 B3 (signal of 0.444) and the related clone 1G1 (signal of 0.498) and finally 1 C3 (signal of 0.386) are shown below.

All three have a very high degree of sequence homology with the matching human germline and have the identical canonical fold combinations as found in the human germline. This was also observed for the lambda light chain in the three antagonistic leads (data not shown). Fab 1A1 competes strongly with the antagonistic reference monoclonal antibody (IC50 of 12 µg/ml in ELISA based competition assay), whereas Fab 1 C3 hardly shows competition (only at concentrations of more than 50 µg/ml). However, in the bioassay 1 C3 is more potent (IC50 of 3 µg/ml) than 1A1 (IC50 of 10 µg/ml), which suggests a different epitope recognition. The high frequency of different antagonistic Fabs (3 different antibodies in 48 screened clones) and the difference in epitope recognition as found for two of these illustrates the high diversity of antibodies as the result of the outbred nature of the lama. The high degree of sequence homology with human germline V regions combined with the high diversity of (potent) antibodies and the broad epitope coverage enables the identification of panels of therapeutic antibodies from immunized camelids.

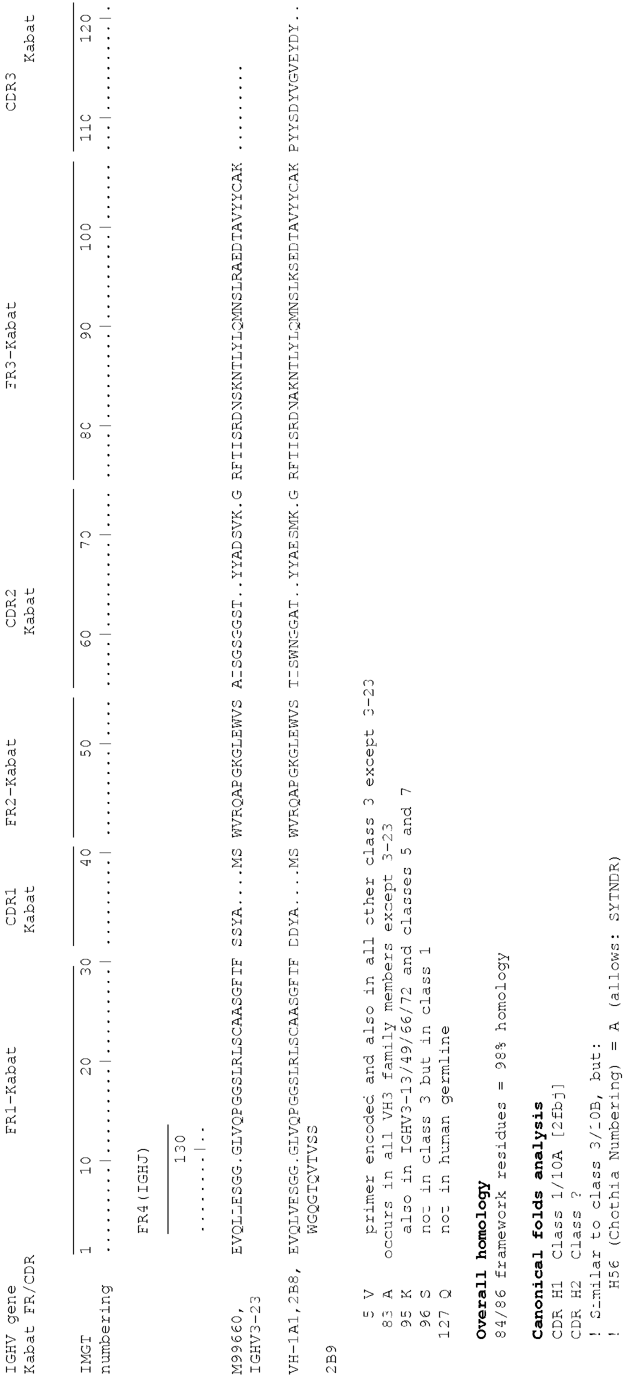

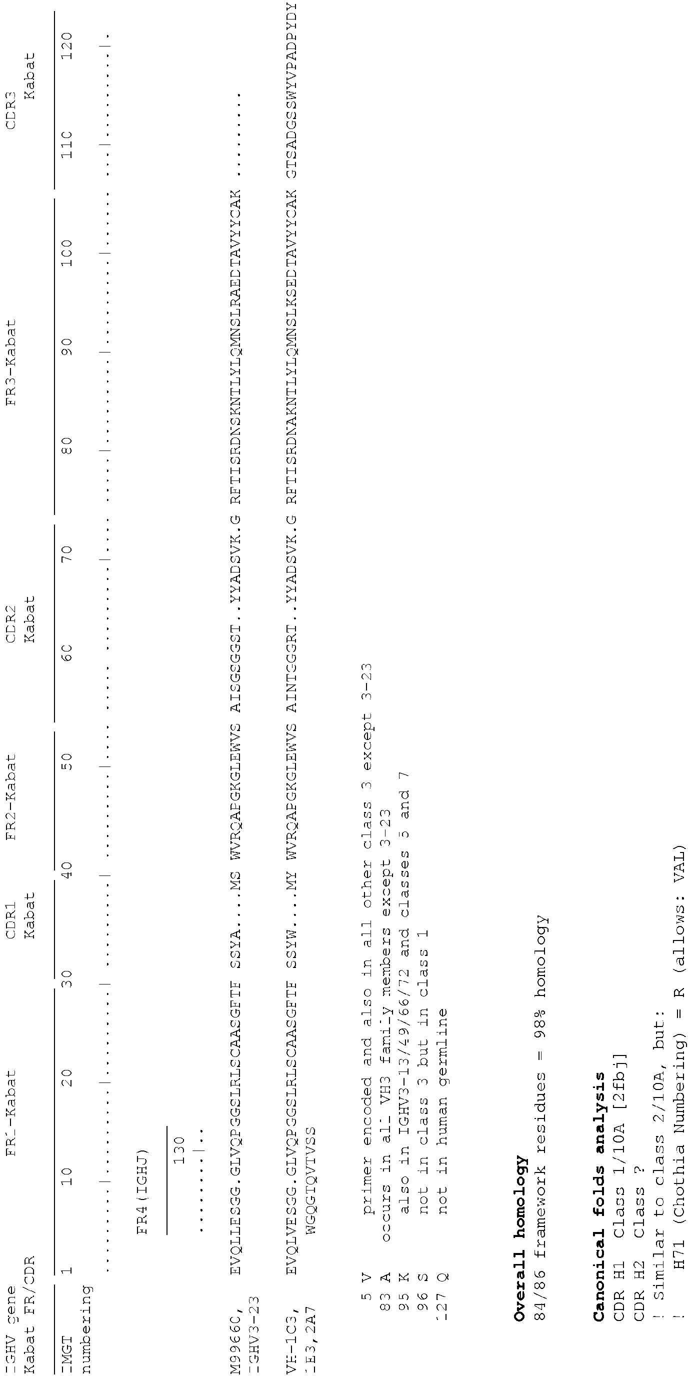

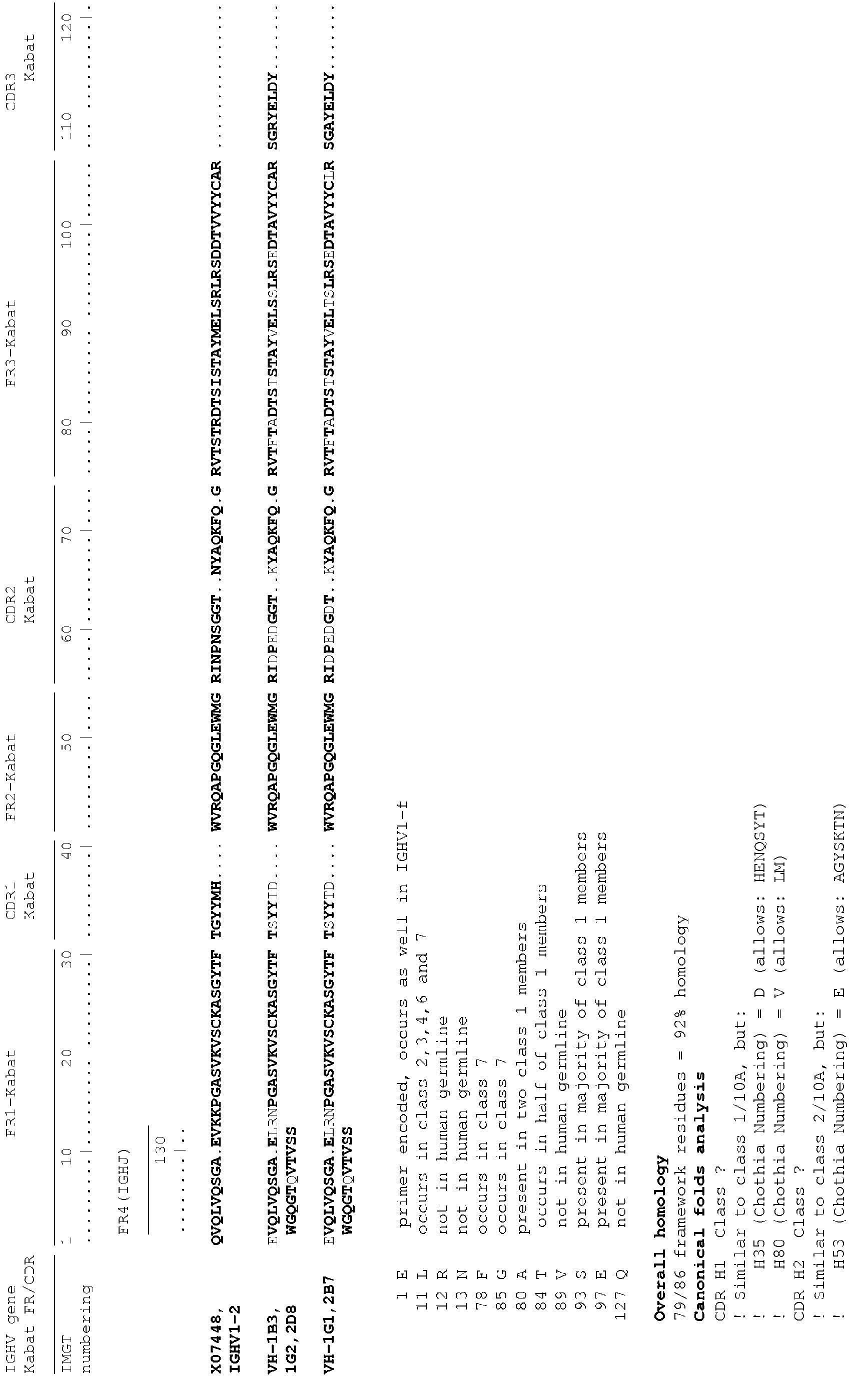

### Example 16

The following example demonstrates the functional diversity which can be achieved with the current invention, in comparison with the established mouse monoclonal antibody approach.

10 BALB/c mice were immunized with a recombinant produced cytokine with a small molecular weight. After completion of the immunization protocol, the animals were sacrificed, and hybridomas were generated by immortalizing their spleen cells. Supernatant of the resulting hybridomas was tested in the cytokine binding ELISA and subsequently in a suitable bioassay. One highly potent antagonist and one weaker antagonist could be identified.

Also, 4 Ilamas were immunized with the same recombinant produced cytokine, using the general protocol described herein. After completion of the immunization protocol, peripheral B lymphocytes were harvested and their RNA was extracted and purified. Using a set of Ilama specific primers, Fab libraries were generated using the phage display technique. Those Fabs were tested in the cytokine/cytokine receptor binding ELISA. 5 different VH families could be identified from the first 2 Ilamas, and 6 additional different VH families from the next 2 Ilamas, which blocked the cytokine/receptor interaction with high potency, meaning that those VH domains contained uniquely different CDRs, both in length and amino acid sequence.

Thus a higher functional diversity could be achieved from a small number of outbred Ilamas as opposed to a higher number of inbred BALB/c mice. All VH families obtained by active immunisation of Ilamas exhibited an extraordinary sequence homology as compared to the closest human germline and had the same canonical fold combinations for CDR1 and CDR2 as the matching human germlines.

### Example 17

The following table summarises the results of amino acid sequence homology comparisons between germline VH domains of alpaca (*Lama pacos*) and the closest matching human germline VH domains. % homology was calculated using the same algorithm as described herein for *Lama glama.* Raw VH sequence data for *Lama pacos* is not shown:

**Table 11: amino acid sequence homology germline VH of Lama pacos vs human**

| **Alpaca (*Lama pacos*) germline VH family** | **Frequency** | **% amino acid sequence homology with closest matching human germline VH** |
|---|---|---|
| VH3 | 70% (36/51) | ≥ 95% |
| VH1 | 10% (5/51) | 90-92% |
| VH2 (NB *Lama pacos* VH2 aligns to human VH4) | 20% (10/51) | 81-88% |

The following table is provided to cross-reference nucleotide and amino acid sequences listed herein with the sequence listing submitted in ST.25 format for searching purposes.

**Table 12: cross-reference to sequence identifiers**

| **SEQ ID No.** | **Sequence name** | **SEQ ID No.** | **Sequence name** |
|---|---|---|---|
| **1** | M99679, IGHV3-53 | **44** | M94116, IGLV1-40 |
| **2** | AF000603, IGHV1S1 | **45** | Camvl44 |
| **3** | AJ245151, IGHV1S2 | **46** | Z73642, IGLV2-18 |
| **4** | AJ245152, IGHV1S3 | **47** | Camv117 |
| **5** | AJ245153, IGHV1S4 | **48** | Camvl33 |
| **6** | AJ245154, IGHV1S5 | **49** | Camv136 |
| **7** | AJ245155, IGHV1S6 | **50** | Camv159 |
| **8** | AJ245157, IGHV1S7 | **51** | Cams130 |
| **9** | AJ245158, IGHV1S8 | **52** | Camv132 |
| **10** | AJ245159, IGHV1S9 | **53** | Camvl57 |
| **11** | AJ245160, IGHV1S10 | **54** | Camvl5 |
| **12** | AJ245164, IGHV1S11 | **55** | Camv165 |
| **13** | AJ245165, IGHV1S12 | **56** | Camv151 |
| **14** | AJ245167, IGHV1S13 | **57** | Camv131 |
| **15** | AJ245168, IGHV1S14 | **58** | Camvl60 |
| **16** | AJ245170, IGHV1S15 | **59** | Camv152 |
| **17** | AJ245171, IGHV1S16 | **60** | X57826, IGLV3-1 |
| **18** | AJ245173, IGHV1S17 | **61** | Camvl19 |
| **19** | AJ245174, IGHV1S18 | **62** | Camvl20 |
| **20** | AJ245156, IGHV1S19 | **63** | Camvl8 |
| **21** | M99660, IGHV3-23 | **64** | Camvl18 |
| **22** | AJ245177, IGHV1S20 | **65** | Camv123 |
| **23** | AJ245178, IGHV1S21 | **66** | Z73658, IGLV3-12 |
| **24** | AJ245183, IGHV1S22 | **67** | Camvlll |
| **25** | AJ245185, IGHV1S23 | **68** | X59314, IGKV2-40 |
| **26** | AJ245186, IGHV1S24 | **69** | Kp6 |
| **27** | AJ245187, IGHV1S25 | **70** | Kp48 |
| **28** | AJ245189, IGHV1S26 | **71** | Kp3 |
| **29** | AJ245191, IGHV1S27 | **72** | Kp20 |
| **30** | AJ245192, IGHV1S28 | **73** | Kp7 |
| **31** | AJ245193, IGHV1S29 | **74** | Kp10 |
| **32** | AJ245194, IGHV1S30 | **75** | Kp1 |
| **33** | AJ245195, IGHV1S31 | **76** | J00256, IGHJ1 |
| **34** | AJ245179, IGHV1S32 | **77** | J00256, IGHJ2 |
| **35** | AJ245180, IGHV1S33 | **78** | J00256, IGHJ3 |
| **36** | AJ245182, IGHV1S34 | **79** | J00256, IGHJ4 |
| **37** | AJ245190, IGHV1S35 | **80** | J00256, IGHJS |
| **38** | AJ245196, IGHV1S36 | **81** | J00256, IGHJ6 |
| **39** | AJ245197, IGHV1S37 | **82** | AF305952, IGHJ2 |
| **40** | AJ245181, IGHV1S38 | **83** | AF305952, IGHJ3 |
| **41** | AJ245198, IGHV1S39 | **84** | AF305952, IGHJ4 |
| **42** | AJ245199, IGHV1S40P | **85** | AF305952, IGHJ5 |
| **43** | AF305949, IGHV1S6 | **86** | AF305952, IGHJ6 (1) |

| **SEQ ID No.** | **Sequence name** | **SEQ ID No.** | **Sequence name** |
|---|---|---|---|
| **87** | X04457, IGLJ1 | **130** | LAMBDA#1 |
| **88** | M15641, IGLJ2 | **131** | LAMBDA#8 (2) |
| **89** | M15642, IGLJ3 | **132** | LAMBDA#5 (2) |
| **90** | X51755, IGLJ4 | **133** | LAMBDA#11 |
| **91** | X51755, IGLJ5 | **134** | HuVL3-2 |
| **92** | M18338, IGLJ6 | **135** | LAMBDA#13 |
| **93** | X51755, IGLJ7 | **136** | LAMBDA#40 |
| **94** | Camvl19, etc. | **137** | LAMBDA#2 |
| **95** | Camvl8/18/4 | **138** | LAMBDA#14 (2) |
| **96** | Camvl58/28/5 | **139** | HuVL4-1 |
| **97** | J00242, IGKJ1 | **140** | LAMBDA#10 |
| **98** | J00242, IGKJ2 | **141** | HuVL5-1 |
| **99** | J00242, IGKJ3 | **142** | LAMBDA#3 |
| **100** | J00242, IGKJ4 | **143** | LAMBDA#7 (2) |
| **101** | J00242, IGKJ5 | **144** | LAMBDA#9 |
| **102** | Kp6/48/3/20/10/1 | **145** | LAMBDA#26 |
| **103** | Kp7 (1/7 analyzed) | **146** | LAMBDA#12 |
| **104** | M99660, IGHV3-23 | **147** | HuVL8-1 |
| **105** | IVH28 | **148** | LAMBDA#42 |
| **106** | IVH69 | **149** | LAMBDA#31 |
| **107** | IVH47 | **150** | HuVK1-1 |
| **108** | IVH48 | **151** | KAPPA#39 |
| **109** | IVH70 | **152** | KAPPA#22 |
| **110** | IVH71 | **153** | KAPPA#24 |
| **111** | HuVL1-1(3/4/5) | **154** | KAPPA#21 |
| **112** | LAMBDA#14 | **155** | KAPPA#23 |
| **113** | LAMBDA#16 | **156** | KAPPA#7 |
| **114** | LAMBDA#46 | **157** | KAPPA#19 |
| **115** | LAMBDA#45 | **158** | KAPPA#25b |
| **116** | LAMBDA#15 | **159** | I-_8 |
| **117** | HuVL1-2 | **160** | KAPPA#43 |
| **118** | LAMBDA#47 | **161** | KAPPA#44 |
| **119** | LAMBDA#18 | **162** | KAPPA#20b |
| **120** | LAMBDA#32 | **163** | HuVK4-1 |
| **121** | LAMBDA#28 | **164** | KAPPA#53 |
| **122** | LAMBDA#29 | **165** | KAPPA#50 |
| **123** | LAMBDA#27 | **166** | KAPPA#20 |
| **124** | LAMBDA#17 | **167** | KAPPA#48 |
| **125** | LAMBDA#4 | **168** | KAPPA#55 |
| **126** | LAMBDA#7 | **169** | KAPPA#9 |
| **127** | LAMBDA#8 | **170** | KAPPA#51 |
| **128** | LAMBDA#5 | **171** | KAPPA#10 |
| **129** | HuVL2-1 | **172** | KAPPA#54 |

| **SEQ ID No.** | **Sequence name** | **SEQ ID No.** | **Sequence name** |
|---|---|---|---|
| **173** | KAPPA#49 | **216** | VH_105 |
| **174** | KAPPA#52 | **217** | VH_101 |
| **175** | KAPPA#13 | **218** | VH_108 |
| **176** | KAPPA#11 | **219** | VH_106 |
| **177** | KAPPA#36 | **220** | VH3(3-13) |
| **178** | KAPPA#25 | **221** | VH_115 |
| **179** | KAPPA#12 | **222** | VH_85 |
| **180** | KAPPA#34 | **223** | VH_114 |
| **181** | KAPPA#48b | **224** | VH_78 |
| **182** | KAPPA#21 (2) | **225** | 3B5 |
| **183** | KAPPA#24 (2) | **226** | 3A3 |
| **184** | KAPPA#22b | **227** | HuVl1A-BACK-ApaLI |
| **185** | KAPPA#45 | **228** | HuVl1B-BACK-ApaLI |
| **186** | KAPPA#46 | **229** | HuVl1C-BACK-ApaLI |
| **187** | VH1 (1-02) | **230** | HuVl2-BACK-ApaLI |
| **188** | 1C2 | **231** | HuVl3A-BACK-ApaLI |
| **189** | 1G5 | **232** | HuVl3B-BACK-ApaLI |
| **190** | VH3 (3-23) | **233** | HuVl4-BACK-ApaLI |
| **191** | 5B12 | **234** | HuVl5-BACK-ApaLI |
| **192** | 1G4 | **235** | HuVl6-BACK-ApaLI |
| **193** | 5G7 | **236** | HuVl7/8-BACK-ApaLI |
| **194** | VH_99 | **237** | HuVl9-BACK-ApaLI |
| **195** | VH_76 | **238** | HuVl10-BACK-ApaLI |
| **196** | VH_86 | **239** | caClambda1-FOR |
| **197** | VH_98 | **240** | caClambda2-FOR |
| **198** | VH_89 | **241** | HuVk1B-BACK-ApaLI |
| **199** | VH_82 | **242** | HuVk2-BACK-ApaLI |
| **200** | VH_68 | **243** | HuVk3B-BACK-ApaLI |
| **201** | VH_73 | **244** | HuVk2/4-BACK-ApaLI |
| **202** | VH_107 | **245** | HuVk5-BACK-ApaLI |
| **203** | VH_94 | **246** | HuVk6-BACK-ApaLI |
| **204** | VH_90 | **247** | HuVk4B-BACK-ApaLI |
| **205** | VH_77 | **248** | caCHkapFOR-AscI |
| **206** | VH_74 | **249** | caCHkap2FOR-AscI |
| **207** | VH_67 | **250** | VH-1A1, 2B8, 2B9 |
| **208** | VH_79 | **251** | VH-1C3, 1E3, 2A7 |
| **209** | VH_75 | **252** | VH-1B3, 1G2, 2D8 |
| **210** | VH_110 | **253** | VH-1G1, 2B7 |
| **211** | VH_109 | **254** | X07448, IGHV1-2 |
| **212** | VH_103 | **255** | 1F2 VH |
| **213** | VH_84 | **256** | Humanized VH 1F2 |
| **214** | VH_113 | **257** | Safe Variant VH 1F2 |
| **215** | VH_87 | **258** | X92218, IGHV3-66 |

| **SEQ ID No.** | **Sequence name** | **SEQ ID No.** | **Sequence name** |
|---|---|---|---|
| **259** | 1E2 VH | **302** | AM939733 |
| **260** | Humanized VH 1E2 | **303** | AM939717 |
| **261** | Safe Variant VH 1E2 | **304** | AM939734 |
| **262** | Z73650, IGLV8-61 | **305** | AM939735 |
| **263** | 1F2 VL | **306** | AM939736 |
| **264** | Humanized 1F2 VL | **307** | AM939737 |
| **265** | Safe Variant VL 1F2 | **308** | L33851\|IGHV3-74*01 |
| **266** | 1E2 VL | **309** | AM939749 |
| **267** | Humanized VL 1E2 | **310** | AM939724 |
| **268** | Safe Variant VL 1E2 | **311** | AM939725 |
| **269** | X92343\|IGHV1-46*01 | **312** | AM939745 |
| **270** | LpVH1-s6 (AM939701) | **313** | AM939723 |
| **271** | LpVH1-s2 (AM939697) | **314** | X92229\|IGHV4-30-2*03 |
| **272** | LpVH1-s3 (AM939698) | **315** | LpVH2-s7 (AM939704) |
| **273** | LpVH1-s4 (AM939699) | **316** | Z14238\|IGHV4-30-4*01 |
| **274** | LpVH1-s5 Ps (AM939700) | **317** | LpVH2-s2 (AM939769) |
| **275** | M99660\|1GHV3-23*01 | **318** | LpVH2-s3 (AM939770) |
| **276** | AM939712 | **319** | LpVH2-s4 (AM939771) |
| **277** | AM939713 | **320** | LpVH2-s5 (AM939772) |
| **278** | AM939730 | **321** | LpVH2-s6 (AM939773) |
| **279** | AM939731 | **322** | LpVH2-s11 Ps (AM939703) |
| **280** | AM939744 | **323** | LpVH2-s8 (AM939705) |
| **281** | AM939726 | **324** | LpVH2-s9 Ps (AM939706) |
| **282** | AM939727 | **325** | LpVH2-s10 (AM939702) |
| **283** | AM939739 | **326** | AJ879486\|IGHV3-23*04 |
| **284** | AM939740 | **327** | S-VH1 |
| **285** | AM939741 | **328** | S-VH3 |
| **286** | AM939742 | **329** | S-VH4 |
| **287** | AM939743 | **330** | S-VH2 |
| **288** | U29481\|IGHV3-23*03 | **331** | S-VH6 |
| **289** | AM939716 | **332** | S-VH5 |
| **290** | AM939728 | **333** | D86994\|IGLV3-25*02 |
| **291** | AM939738 | **334** | VL25-28 |
| **292** | AM939710 | **335** | Z73672\|IGLV5-37*01 |
| **293** | AM939748 | **336** | VL2,12,15 |
| **294** | AM939750 | **337** | Z73650\|IGLV8-61*01 |
| **295** | AM939751 | **338** | VL3,5 |
| **296** | AM939767 | **339** | VL17-24, 29-32 |
| **297** | AM939768 | **340** | VL10 |
| **298** | AM939707 | **341** | VL4, 6, 7, 8, 9, 13, 14 |
| **299** | AM939708 | **342** | U41644\|IGKV2D-29*02 |
| **300** | AM939709 | **343** | KAPPA 33-36, 38, 39, 42, 4 |
| **301** | AM939732 | **344** | KAPPA 41, 43, 44 |

| **SEQ ID No.** | **Sequence name** | | |
|---|---|---|---|
| **345** | KAPPA 40,44 | | |
| **346** | KAPPA 37,46,48 | | |
| **347** | VH1a-BACK | | |
| **348** | VH5a-BACK | | |
| **349** | VH4a-BACK | | |
| **350** | VH4b-BACK | | |
| **351** | VH2b-BACK | | |
| **352** | VH1a-BACK-SfiI | | |
| **353** | VH5a-BACK-SfiI | | |
| **354** | VH4a-BACK-SfiI | | |
| **355** | VH4b-BACK-SfiI | | |
| **356** | VH2b-BACK-SfiI | | |

## Claims

1. A monoclonal antigen binding polypeptide comprising a VH domain and a VL domain, wherein each of the hypervariable loops in both the VH domain and the VL domain is obtained from a VH or VL domain of a species in the family *Camelidae,* wherein hypervariable loop H1 and hypervariable loop H2 in the VH domain each exhibit a predicted or actual canonical fold structure which is identical to a canonical fold structure which occurs in human antibodies, and wherein hypervariable loop H1 and hypervariable loop H2 in the VH domain form a combination of predicted or actual canonical fold structures which is identical to a combination of canonical fold structures known to occur in a human germline VH domain.

2. An antigen binding polypeptide as claimed in claim 1 comprising a VH domain, wherein the VH domain exhibits a sequence identity of 80% or greater, 85% or greater, 90% or greater, 95% or greater or 97% or greater with one or more human VH domains across the framework regions FR1, FR2, FR3 and FR4, and wherein the VL domain exhibits a sequence identity of 80% or greater, 85% or greater, 90% or greater, 95% or greater or 97% or greater with one or more human VL domains across the framework regions FR1, FR2, FR3 and FR4.

3. An antigen binding polypeptide as claimed in claim 1 which exhibits a sequence identity of 80% or greater with a human VH domain across the framework regions FR1, FR2 , FR3 and FR4, and wherein hypervariable loop H1 and hypervariable loop H2 form a combination of predicted or actual canonical fold structures which is the same as a canonical fold combination known to occur naturally in the same human VH domain.

4. An antigen binding polypeptide as claimed in any one of claims 1 to 3 wherein hypervariable loop L1 and hypervariable loop L2 in the VL domain are each obtained from a VH or VL domain of a species in the family *Camelidae* and each exhibit a predicted or actual canonical fold structure which is identical to a canonical fold structure which occurs in human antibodies , and wherein hypervariable loop L1 and hypervariable loop L2 in the VL domain form a combination of predicted or actual canonical fold structures which is identical to a combination of canonical fold structures known to occur in a human germline VL domain.

5. An antigen binding polypeptide according to any one of the preceding claims wherein the species in the family *Camelidae* is selected from the group consisting of camel, Ilama, dromedary, vicunia, guanaco and alpaca.

6. An antigen binding polypeptide according to any one of the preceding claims which is immunoreactive with or specifically binds to a target antigen, said target antigen being a non-camelid antigen, or a human antigen, or a viral antigen or a bacterial antigen, or a target of therapeutic or diagnostic importance.

7. An antigen binding polypeptide according to any one of the preceding claims which is a chimeric polypeptide, which comprises at least one constant domain having an amino acid sequence which is encoded by a human immunoglobulin gene, or an amino acid sequence at least 90% identical thereto, or which comprises the complete constant region of a human antibody.

8. An antigen binding polypeptide according to any one of the preceding claims which is an antibody, Fab, Fab', F(ab')2, bi-specific Fab', Fv fragments, diabody, linear antibody, a single chain variable fragment (scFv) or multispecific antibody formed from antibody fragments.

9. A polynucleotide molecule encoding an antigen binding polypeptide according to any of claims 1 to 8.

10. An expression vector comprising the polynucleotide molecule of claim 9 operably linked to regulatory sequences which permit expression of the antigen binding polypeptide in a host cell or cell-free expression system.

11. A host cell or cell-free expression system containing the expression vector of claim 10.

12. A method of producing a recombinant antigen binding polypeptide which comprises culturing the host cell or cell free expression system of claim 11 under conditions which permit expression of the antigen binding polypeptide and recovering the expressed antigen binding polypeptide.

## Patentansprüche

1. Monoklonales Antigen-bindendes Polypeptid, welches eine VH-Domäne und eine VL-Domäne aufweist, wobei jede der hypervariablen Schlingen sowohl in der VH-Domäne als auch in der VL-Domäne von einer VH- oder VL-Domäne einer Spezies aus der Familie der Kameliden gewonnen wird, wobei die hypervariable Schlinge H1 und die hypervariable Schlinge H2 in der VH-Domäne jeweils eine vorhergesagte oder tatsächliche kanonische Faltstruktur aufweisen, welche einer kanonischen Faltstruktur entspricht, die bei menschlichen Antikörpern auftritt und wobei die hypervariable Schlinge H1 und die hypervariable Schlinge H2 in der VH-Domäne eine Kombination vorhergesagter oder tatsächlicher kanonischer Faltstrukturen bilden, welche einer Kombination kanonischer Faltstrukturen entspricht, von der bekannt ist, dass sie in einer VH-Domäne der menschlichen Keimbahn vorkommt.

2. Antigen-bindendes Polypeptid nach Anspruch 1, welches eine VH-Domäne umfasst, wobei die VH-Domäne eine Sequenzidentität von 80% oder mehr, 85% oder mehr, 90% oder mehr, 95% oder mehr, 97% oder mehr mit einer oder mehreren menschlichen VH-Domänen in den Strukturbereichen FR1, FR2, FR3 und FR4 aufweist, und wobei die VL-Domäne eine Sequenzidentität von 80% oder mehr, 85% oder mehr, 90% oder mehr, 95% oder mehr, 97% oder mehr mit einer oder mehreren menschlichen VL-Domänen in den Strukturbereichen FR1, FR2, FR3 und FR4 aufweist.

3. Antigen-bindendes Polypeptid nach Anspruch 1, welches eine Sequenzidentität von 80% oder mehr mit einer menschlichen VH-Domäne in den Strukturbereichen FR1, FR2, FR3 und FR4 aufweist, und wobei die hypervariable Schlinge H1 und die hypervariable Schlinge H2 eine Kombination vorhergesagter oder tatsächlicher kanonischer Faltstrukturen bilden, welche einer Kombination kanonischer Faltstrukturen entspricht, von der bekannt ist, dass sie auch natürlich in derselben menschlichen VH-Domäne vorkommt.

4. Antigen-bindendes Polypeptid nach einem der Ansprüche 1 bis 3, wobei sowohl die hypervariable Schlinge L1 als auch die hypervariable Schlinge L2 in der VL-Domäne von einer VH- oder VL-Domäne einer Spezies aus der Familie der Kameliden gewonnen wird und beide eine vorhergesagte oder tatsächliche kanonische Faltstruktur aufweisen, welche einer kanonischen Faltstruktur entspricht, die bei menschlichen Antikörpern auftritt, und wobei die hypervariable Schlinge L1 und die hypervariable Schlinge L2 in der VL-Domäne eine Kombination vorhergesagter oder tatsächlicher kanonischer Faltstrukturen bilden, welche einer Kombination kanonischer Faltstrukturen entspricht, von der bekannt ist, dass sie in einer VL-Domäne der menschlichen Keimbahn vorkommt.

5. Antigen-bindendes Polypeptid nach einem der vorhergehenden Ansprüche, wobei die Spezies aus der Familie der Kameliden gewählt ist aus der Gruppe, umfassend: Kamel, Lama, Dromedar, Vikunja, Guanako und Alpaka.

6. Antigen-bindendes Polypeptid nach einem der vorhergehenden Ansprüche, welches mit einem Zielantigen immunreaktiv ist oder ein Zielantigen spezifisch bindet, wobei das Zielantigen ein Nicht- Kamelid-Antigen oder ein menschliches Antigen oder ein virales Antigen oder ein bakterielles Antigen oder ein Ziel von therapeutischer oder diagnostischer Bedeutung ist.

7. Antigen-bindendes Polypeptid nach einem der vorhergehenden Ansprüche, welches ein chimäres Polypeptid ist, das wenigstens eine konstante Domäne mit einer Aminosäuresequenz aufweist, die durch ein menschliches Immunglobulin-Gen kodiert wird oder eine dazu zu wenigstens 90% identische Aminosäuresequenz, oder die den vollständigen konstanten Bereich eines menschlichen Antikörpers aufweist.

8. Antigen-bindendes Polypeptid nach einem der vorhergehenden Ansprüche, welches ist: ein Antikörper, Fab, Fab', F(ab')2, bi-spezifisches Fab, Fv-Fragmente, Diabody, linearer Antikörper, ein variables Einzelkettenfragment (scFv) oder ein aus Antikörperfragmenten gebildeter multispezifischer Antikörper.

9. Polynukleotid-Molekül, welches ein Antigen-bindendes Polypeptid nach einem der Ansprüche 1 bis 8 kodiert.

10. Expressionsvektor, welcher das Polynukleotid-Molekül nach Anspruch 9 umfasst und operativ mit regulatorischen Sequenzen verbunden ist, welche die Expression des Antigen-bindenden Polypeptids in einer Wirtszelle oder einem zellfreien Expressionssystem ermöglichen.

11. Wirtszelle oder zellfreies Expressionssystem, welches den Expressionsvektor nach Anspruch 10 umfasst.

12. Verfahren zur Herstellung eines rekombinanten Antigen-bindenden Polypeptids, welches die Kultivierung der Wirtszelle oder des zellfreien Expressionssystems nach Anspruch 11 unter Bedingungen umfasst, welche die Expression des Antigen-bindenden Polypeptids und Gewinnung des exprimierten Antigen-bindenden Polypeptids ermöglichen.

## Revendications

1. Polypeptide monoclonal de liaison à un antigène comprenant un domaine VH et un domaine VL, où chacune des boucles hypervariables à la fois dans le domaine VH et le domaine VL est obtenue à partir d'un domaine VH ou VL d'une espèce dans la famille *Camelidae,*
où la boucle hypervariable H1 et la boucle hypervariable H2 dans le domaine VH présentent chacune une structure de pli canonique prédite ou réelle qui est identique à une structure de pli canonique qui se produit dans des anticorps humains, et où la boucle hypervariable H1 et la boucle hypervariable H2 dans le domaine VH forment une combinaison de structures de plis canoniques prédites ou réelles qui est identique à une combinaison de structures de plis canoniques se produisant dans un domaine VH de lignée germinale humaine.

2. Polypeptide de liaison à un antigène tel que revendiqué dans la revendication 1, comprenant un domaine VH, où le domaine VH présente une identité de séquence de 80 % ou plus, de 85 % ou plus, de 90 % ou plus, de 95 % ou plus ou de 97 % ou plus avec un ou plusieurs domaine(s) VH humain (s) à travers les régions de charpente FR1, FR2, FR3 et FR4, et où le domaine VL présente une identité de séquence de 80 % ou plus, de 85 % ou plus, de 90 % ou plus, de 95 % ou plus, ou de 97 % ou plus avec un ou plusieurs domaine(s) VL humain(s) à travers les régions de charpente FR1, FR2, FR3 et FR4.

3. Polypeptide de liaison à un antigène tel que revendiqué dans la revendication 1, qui présente une identité de séquence de 80 % ou plus avec un domaine VH humain à travers les régions de charpente FR1, FR2, FR3 et FR4, et où la boucle hypervariable H1 et la boucle hypervariable H2 forment une combinaison de structures de plis canoniques prédites ou réelles qui est la même qu'une combinaison de plis canoniques se produisant naturellement dans le même domaine VH humain.

4. Polypeptide de liaison à un antigène tel que revendiqué dans l'une quelconque des revendications 1 à 3, dans lequel la boucle hypervariable L1 et la boucle hypervariable L2 dans le domaine VL sont obtenues chacune à partir d'un domaine VH ou VL d'une espèce dans la famille *Camelidae* et présentent chacune une structure de pli canonique prédite ou réelle qui est identique à une structure de pli canonique qui se produit dans des anticorps humains, et où la boucle hypervariable L1 et la boucle hypervariable L2 dans le domaine VL forment une combinaison de structures de plis canoniques prédites ou réelles qui est identique à une combinaison de structures de plis canoniques se produisant dans un domaine VL de lignée germinale humaine.

5. Polypeptide de liaison à un antigène selon l'une quelconque des revendications précédentes, dans lequel l'espèce dans la famille *Camelidae* est choisie dans le groupe consistant en le chameau, le lama, le dromadaire, la vigogne, le guanaco et l'alpaga.

6. Polypeptide de liaison à un antigène selon l'une quelconque des revendications précédentes, qui est immunoréactif avec un antigène cible ou se lie spécifiquement à celui-ci, ledit antigène cible étant un antigène non camélidé, ou un antigène humain, ou un antigène viral ou un antigène bactérien, ou une cible d'importance thérapeutique ou diagnostique.

7. Polypeptide de liaison à un antigène selon l'une quelconque des revendications précédentes, qui est un polypeptide chimère, qui comprend au moins un domaine constant ayant une séquence d'acides aminés qui est codée par une gène d'immunoglobuline humaine, ou une séquence d'acides aminés identique à au moins 90 % à celui-ci, ou qui comprend la région constante complète d'un anticorps humain.

8. Polypeptide de liaison à un antigène selon l'une quelconque des revendications précédentes qui est un anticorps, des fragments Fab, Fab', F(ab')2, Fab' bispécifique, Fv, un dianticorps, un anticorps linéaire, un fragment variable à chaîne unique (scFv) ou un anticorps multispécifique formé à partir de fragments d'anticorps.

9. Molécule de polynucléotide codant pour un polypeptide de liaison à un antigène selon l'une des revendications 1 à 8.

10. Vecteur d'expression comprenant la molécule de polynucléotide de la revendication 9 liée de manière fonctionnelle à des séquences régulatrices qui permettent l'expression du polypeptide de liaison à un antigène dans une cellule hôte ou un système d'expression acellulaire.

11. Cellule hôte ou système d'expression acellulaire contenant le vecteur d'expression de la revendication 10.

12. Procédé de production d'un polypeptide recombinant de liaison à un antigène qui comprend le fait de mettre en culture la cellule hôte ou le système d'expression acellulaire de la revendication 11 dans des conditions qui permettent l'expression du polypeptide de liaison à un antigène et de récupérer le polypeptide de liaison à un antigène exprimé.
